# EUROPEAN PATENT SPECIFICATION

(11) **EP 3 694 871 B1**
(45) Date of publication and mention of the grant of the patent: **25.11.2026**
(21) Application number: 18866074.0
(22) Date of filing: 12.10.2018
(51) Int. Cl.: A61K 39/395, C07K 14/705, C07K 16/28, C07K 16/18, A61P 35/00

(54) **B CELL MATURATION ANTIGEN BINDING PROTEINS**
B-ZELLREIFUNGS-ANTIGENBINDENDE PROTEINE
PROTÉINES DE LIAISON À L'ANTIGÈNE DE MATURATION DE CELLULES B

(30) Priority: 13.10.2017 US 201762572375 P
(43) Date of publication of application: 19.08.2020
(73) Proprietor: Harpoon Therapeutics, Inc., South San Francisco, CA 94080 (US)
(72) Inventor: WESCHE, Holger, South San Francisco California 94080 (US); LEMON, Bryan D., South San Francisco California 94080 (US); AUSTIN, Richard J., South San Francisco California 94080 (US)
(74) Representative: Merck Sharp & Dohme LLC
(86) International application number: PCT/US2018/055682
(87) International publication number: WO 2019/075378

(56) References cited:
- WO-A1-2013/110531
- WO-A1-2016/187594
- WO-A1-2017/025038
- WO-A1-2018/026953
- WO-A1-2019/222278
- US-A1- 2013 273 055

## Description

### CROSS-REFERENCE

This application claims the benefit of U.S. Provisional Application No. 62/572,375 filed October 13, 2017.

### SEQUENCE LISTING

The instant application contains a Sequence Listing which has been submitted electronically in ASCII format Said ASCII copy, created on October 11, 2018, is named 47517-722_601_SL.txt and is 232,688 bytes in size.

### BACKGROUND OF THE INVENTION

Cancer is the second leading cause of human death next to coronary disease. Worldwide, millions of people die from cancer every year. In the United States alone, cancer causes the death of well over a half-million people each year, with some 1.4 million new cases diagnosed per year. While deaths from heart disease have been declining significantly, those resulting from cancer generally are on the rise. In the early part of the next century, cancer is predicted to become the leading cause of death.

Moreover, even for those cancer patients that initially survive their primary cancers, common experience has shown that their lives are dramatically altered. Many cancer patients experience strong anxieties driven by the awareness of the potential for recurrence or treatment failure. Many cancer patients experience significant physical debilitations following treatment.

Generally speaking, the fundamental problem in the management of the deadliest cancers is the lack of effective and non-toxic systemic therapies. Cancer is a complex disease characterized by genetic mutations that lead to uncontrolled cell growth. Cancerous cells are present in all organisms and, under normal circumstances, their excessive growth is tightly regulated by various physiological factors.
WO2017/025038 describes single-domain antibodies, and chimeric antigen receptors comprising one or more antigen binding domains each comprising a single-domain antibody. This document further describes engineered immune effector cells (such as T cells) comprising the chimeric antigen receptors.

### SUMMARY

### Summary of general teachings

At a general level, the present disclosure provides single domain B cell maturation antigen (BCMA) binding proteins which can be used for diagnosing and treating indications correlated to the expression of BCMA.

A domain BCMA binding protein can have an elimination half-time of at least 12 hours, at least 20 hours, at least 25 hours, at least 30 hours, at least 35 hours, at least 40 hours, at least 45 hours, at least 50 hours, at least 100 hours or more. In some embodiments, the single domain BCMA binding protein further comprises a Fc domain. In some embodiments, the single domain BCMA binding protein further comprises an anti-cancer agent.

Such a single domain BCMA binding protein can be human, humanized, affinity matured, or a combination thereof.

Provided herein are single domain BCMA binding proteins for use in a method for the treatment or amelioration of a B cell lineage cancer in a subject in need thereof.

Provided herein are multispecific binding proteins for use in a method for the treatment or amelioration of a B cell lineage cancer in a subject in need thereof, comprising administering to the subject the multispecific binding protein.

The B cell lineage cancer can be a primary cancer or a metastatic cancer.

A B cell lineage cancer to be treated with the methods described herein can be a multiple myeloma, a leukemia, a lymphoma.

### Summary of the invention

The claimed invention provides single domain B cell maturation (BCMA) binding proteins, that comprise an amino acid sequence selected from SEQ ID Nos: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419, and 421. The claimed invention also provides multispecific binding proteins comprising a single domain BCMA binding protein of the claimed invention, a human serum albumin binding domain and a CD3 binding domain. The invention further provides the said multispecific binding proteins for use in a method of treating B cell lineage cancers, such as multiple myeloma, leukemia or lymphoma.

It is immediately clear to the skilled person which of the sequences disclosed herein fall within the scope of the claims, by reference to the sequence table at the end of the Examples. Any sequences not falling within the scope of the claims are disclosed as additional information.

### BRIEF DESCRIPTION OF THE DRAWINGS

**Fig. 1** illustrates the effect of exemplary BCMA targeting molecules (01H08, 01F07, 02F02, and BH253), containing an anti-BCMA binding protein according to the present disclosure in killing of EJM cells that express BCMA compared to a negative control.
**Fig. 2** is an image of an SDS-PAGE of representative purified BCMA trispecific molecules. Lane 1: 01F07-M34Y TriTAC non-reduced; Lane 2:01F07-M34G-TriTAC non-reduced; Lane 3: 02B05 TriTAC non-reduced; Lane 4: 02G02-M34Y TriTAC non-reduced; Lane 5: 02G02 M34G TriTAC non-reduced; Lane 6: Broad Range SDS-PAGE Standard (Bio-Rad #1610317); Lane 7: 01F07-M34Y TriTAC non-reduced; Lane 8:01F07-M34G-TriTAC non-reduced; Lane 9: 02B05 TriTAC non-reduced; Lane 10: 02G02-M34Y TriTAC non-reduced; Lane 11: 02G02 M34G TriTAC non-reduced; and Lane 12: Broad Range SDS-PAGE Standard (Bio-Rad #1610317).
**Figs. 3A-3I** illustrate the effect of exemplary BCMA trispecific targeting molecules containing an anti-BCMA binding protein according to the present disclosure in killing of Jeko1 **(****Figs. 3A-3C****),** MOLP-8 **(****Figs. 3D-3F****)** or OPM-2 **(****Figs. 3G-3I****)** cells that express BCMA compared to a negative control.
**Figs. 4A-4D** illustrate binding of an exemplary BCMA trispecific targeting protein containing a BCMA binding protein of this disclosure (02B05) to purified T Cells from four different human donors, donor 02 **(****Fig. 4A****),** donor 35 **(****Fig. 4B****),** donor 81 **(****Fig. 4C****),** donor 86 **(****Fig. 4D****).**
**Figs. 5A-5F** illustrate binding of an exemplary BCMA trispecific targeting protein (02B05) to cells expressing BCMA, NCI-H929 **(****Fig. 5A****),** EJM **(****Fig. 5B****),** OPM2 **(****Fig. 5D****),** RPMI8226 **(****Fig. 5E****);** or cell lines lacking expression of BCMA, NCI-H510A **(****Fig. 5C****),** and DMS-153 **(****Fig. 5F****).**
**Fig. 6** illustrates the results of a TDCC assay using an exemplary BCMA trispecific targeting protein (02B05) and BCMA expressing EJM cells, in presence or absence of human serum albumin (HSA).
**Fig. 7** illustrates the results of a TDCC assay using an exemplary BCMA trispecific targeting protein (02B05) and BCMA expressing EJM cells, using varying effector cells to target cells ratio.
**Fig. 8** illustrates the results of a TDCC assay using an exemplary BCMA trispecific targeting protein (02B05) and BCMA expressing OPM2 cells, using varying effector cells to target cells ratio.
**Fig. 9** illustrates the results of a TDCC assay using an exemplary BCMA trispecific targeting protein (02B05) and BCMA expressing NCI-H929 cells, using varying time-points and a 1:1 effector cells to target cells ratio.
**Fig. 10** illustrates the results of a TDCC assay using an exemplary BCMA trispecific targeting protein (02B05), BCMA expressing EJM cells, and T cells from four different donors, in presence of human serum albumin (HSA).
**Fig. 11** illustrates the results of a TDCC assay using an exemplary BCMA trispecific targeting protein (02B05), BCMA expressing NCI-H929 cells, and T cells from four different donors, in presence of human serum albumin (HSA).
**Fig. 12** illustrates the results of a TDCC assay using an exemplary BCMA trispecific targeting protein (02B05), BCMA expressing OPM2 cells, and T cells from four different donors, in presence of human serum albumin (HSA).
**Fig. 13** illustrates the results of a TDCC assay using an exemplary BCMA trispecific targeting protein (02B05), BCMA expressing RPMI8226 cells, and T cells from four different donors, in presence of human serum albumin (HSA).
**Fig. 14** illustrates the results of a TDCC assay using an exemplary BCMA trispecific targeting protein (02B05), BCMA non-expressing OVCAR8 cells, and T cells from four different donors, in presence of human serum albumin (HSA).
**Fig. 15** illustrates the results of a TDCC assay using an exemplary BCMA trispecific targeting protein (02B05), BCMA non-expressing NCI-H510A cells, and T cells from four different donors, in presence of human serum albumin (HSA).
**Fig. 16** illustrates the results of a TDCC assay using an exemplary BCMA trispecific targeting protein (02B05), BCMA expressing NCI-H929 cells, and peripheral blood mononuclear cells (PBMC) from two different cynomolgus, in presence of human serum albumin (HSA).
**Fig. 17** illustrates the results of a TDCC assay using an exemplary BCMA trispecific targeting protein containing a BCMA binding protein of this disclosure (02B05), BCMA expressing RPMI8226 cells, and peripheral blood mononuclear cells (PBMC) from two different cynomolgus donors, in presence of human serum albumin (HSA).
**Fig. 18** illustrates the expression level of T cell activation biomarker CD69, following a TDCC assay using an exemplary BCMA targeting trispecific protein containing a BCMA binding protein of this disclosure (02B05) and BCMA expressing cells EJM.
**Fig. 19** illustrates the expression level of T cell activation biomarker CD25, following a TDCC assay using an exemplary BCMA targeting trispecific protein containing a BCMA binding protein of this disclosure (02B05) and BCMA expressing cells EJM.
**Fig. 20** illustrates the expression level of T cell activation biomarker CD69, following a TDCC assay using an exemplary BCMA targeting trispecific protein containing a BCMA binding protein of this disclosure (02B05) and BCMA expressing cells OPM2.
**Fig. 21** illustrates the expression level of T cell activation biomarker CD25, following a TDCC assay using an exemplary BCMA targeting trispecific protein containing a BCMA binding protein of this disclosure (02B05) and BCMA expressing cells OPM2.
**Fig. 22** illustrates the expression level of T cell activation biomarker CD69, following a TDCC assay using an exemplary BCMA targeting trispecific protein containing a BCMA binding protein of this disclosure (02B05) and BCMA expressing cells RPMI8226.
**Fig. 23** illustrates the expression level of T cell activation biomarker CD25, following a TDCC assay using an exemplary BCMA targeting trispecific protein containing a BCMA binding protein of this disclosure (02B05) and BCMA expressing cells RPMI8226.
**Fig. 24** illustrates the expression level of T cell activation biomarker CD69, following a TDCC assay using an exemplary BCMA targeting trispecific protein containing a BCMA binding protein of this disclosure (02B05) and BCMA non-expressing cells OVCAR8.
Fig. 25 illustrates the expression level of T cell activation biomarker CD25, following a TDCC assay using an exemplary BCMA targeting trispecific protein containing a BCMA binding protein of this disclosure (02B05) and BCMA non-expressing cells OVCAR8.
Fig. 26 illustrates the expression level of T cell activation biomarker CD69, following a TDCC assay using an exemplary BCMA targeting trispecific protein containing a BCMA binding protein of this disclosure (02B05) and BCMA non-expressing cells NCI-H510A.
Fig. 27 illustrates the expression level of T cell activation biomarker CD25, following a TDCC assay using an exemplary BCMA targeting trispecific protein containing a BCMA binding protein of this disclosure (02B05) and BCMA non-expressing cells NCI-H510A.
Fig. 28 illustrates the expression level a cytokine, TNF-α, in co-cultures of T cells and BCMA expressing target cells (EJM cells) treated with increasing concentrations of an exemplary BCMA targeting trispecific protein containing a BCMA binding protein of this disclosure (02B05) or with a negative control GFP trispecific protein.
**Fig. 29** illustrates tumor growth reduction in RPMI8226 xenograft model, treated with an exemplary BCMA targeting trispecific protein containing a BCMA binding protein of this disclosure (02B05), at varying concentrations, or with a control vehicle.
**Fig. 30** illustrates tumor growth reduction in Jeko1 xenograft model, treated with an exemplary BCMA targeting trispecific protein containing a BCMA binding protein of this disclosure (02B05), at varying concentrations, or with a control vehicle.
**Fig. 31** illustrates concentration of BCMA targeting trispecific protein in serum samples from cynomolgus monkeys dosed with varying concentrations of an exemplary BCMA targeting trispecific protein containing a BCMA binding protein of this disclosure (02B05).
**Fig. 32** the results of a TDCC assay using BCMA trispecific targeting protein obtained from serum samples of cynomolgus monkeys dosed with varying concentrations of an exemplary BCMA targeting trispecific protein containing a BCMA binding protein of this disclosure (02B05), BCMA expressing EJM cells, and purified human T cells, in presence of serum from cynomolgus monkeys that were not exposed to a BCMA targeting trispecific protein.

### DETAILED DESCRIPTION OF THE INVENTION

The technical disclosure set out below may in some respects go beyond the scope of the claims. Elements of the disclosure which do not fall within the scope of the claims are provided for information. It is immediately clear to the skilled person which of the sequences disclosed herein fall within the scope of the claims, by reference to the sequence table at the end of the Examples. Any sequences not falling within the scope of the claims are disclosed for information.

### Certain definitions

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the given value. Where particular values are described in the application and claims, unless otherwise stated the term "about" should be assumed to mean an acceptable error range for the particular value.

The terms "individual," "patient," or "subject" are used interchangeably. None of the terms require or are limited to situation characterized by the supervision (e.g. constant or intermittent) of a health care worker (e.g. a doctor, a registered nurse, a nurse practitioner, a physician's assistant, an orderly, or a hospice worker).

An "antibody" typically refers to a Y-shaped tetrameric protein comprising two heavy (H) and two light (L) polypeptide chains held together by covalent disulfide bonds and noncovalent interactions. Human light chains comprise a variable domain (VL) and a constant domain (CL) wherein the constant domain may be readily classified as kappa or lambda based on amino acid sequence and gene loci. Each heavy chain comprises one variable domain (VH) and a constant region, which in the case of IgG, IgA, and IgD, comprises three domains termed CH1, CH2, and CH3 (IgM and IgE have a fourth domain, CH4). In IgG, IgA, and IgD classes the CH1 and CH2 domains are separated by a flexible hinge region, which is a proline and cysteine rich segment of variable length (generally from about 10 to about 60 amino acids in IgG). The variable domains in both the light and heavy chains are joined to the constant domains by a "J" region of about 12 or more amino acids and the heavy chain also has a "D" region of about 10 additional amino acids. Each class of antibody further comprises inter-chain and intra-chain disulfide bonds formed by paired cysteine residues. There are two types of native disulfide bridges or bonds in immunoglobulin molecules: inter-chain and intra-chain disulfide bonds. The location and number of inter-chain disulfide bonds vary according to the immunoglobulin class and species. Inter-chain disulfide bonds are located on the surface of the immunoglobulin, are accessible to solvent and are usually relatively easily reduced. In the human IgG1 isotype there are four inter-chain disulfide bonds, one from each heavy chain to the light chain and two between the heavy chains. The inter-chain disulfide bonds are not required for chain association. As is well known the cysteine rich IgG1 hinge region of the heavy chain has generally been held to consist of three parts: an upper hinge, a core hinge, and a lower hinge. Those skilled in the art will appreciate that the IgG1 hinge region contain the cysteines in the heavy chain that comprise the inter-chain disulfide bonds (two heavy/heavy, two heavy/light), which provide structural flexibility that facilitates Fab movements. The inter-chain disulfide bond between the light and heavy chain of IgG1 are formed between C214 of the kappa or lambda light chain and C220 in the upper hinge region of the heavy chain. The inter-chain disulfide bonds between the heavy chains are at positions C226 and C229 (all numbered per the EU index according to Kabat, *et al.,* infra.)

As used herein the term "antibody" includes polyclonal antibodies, multiclonal antibodies, monoclonal antibodies, chimeric antibodies, humanized and primatized antibodies, CDR grafted antibodies, human antibodies, recombinantly produced antibodies, intrabodies, multispecific antibodies, bispecific antibodies, monovalent antibodies, multivalent antibodies, anti-idiotypic antibodies, synthetic antibodies, including muteins and variants thereof, immunospecific antibody fragments such as Fd, Fab, F(ab')2, F(ab') fragments, single-chain fragments (e.g., ScFv and ScFvFc), disulfide-linked Fvs (sdFv), a Fd fragment consisting of the VH and CH1 domains, linear antibodies, single domain antibodies such as sdAb (VH, VL, or VHH domains (such as a heavy chain only antibody devoid of a light chain)); and derivatives thereof including Fc fusions and other modifications, and any other immunoreactive molecule so long as it comprises a domain having a binding site for preferential association or binding with a BCMA protein. Moreover, unless dictated otherwise by contextual constraints the term further comprises all classes of antibodies (*i.e.* IgA, IgD, IgE, IgG, and IgM) and all subclasses *(i.e.,* IgG1, IgG2, IgG3, IgG4, IgA1, and IgA2). Heavy-chain constant domains that correspond to the different classes of antibodies are typically denoted by the corresponding lower case Greek letter alpha, delta, epsilon, gamma, and mu, respectively. Light chains of the antibodies from any vertebrate species can be assigned to one of two clearly distinct types, called kappa (kappa) and lambda (lambda), based on the amino acid sequences of their constant domains.

The term "Framework" or "FR" residues (or regions) refer to variable domain residues other than the CDR or hypervariable region residues as herein defined. A "human consensus framework" is a framework which represents the most commonly occurring amino acid residue in a selection of human immunoglobulin VL or VH framework sequences.

As used herein, "Variable region" or "variable domain" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the βsheet structure. The CDRs in each chain are held together in close proximity by the FR regions and, with the CDRs from the other chain, contribute to the formation of the antigen-binding site of antibodies (*see,* Kabat *et al.,* Sequences of Proteins of Immunological Interest, Fifth Edition, National Institute of Health, Bethesda, Md. (1991)). The constant domains are not involved directly in binding an antibody to an antigen, but exhibit various effector functions, such as participation of the antibody in antibody-dependent cellular toxicity. "Variable domain residue numbering as in Kabat" or "amino acid position numbering as in Kabat," and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or CDR of the variable domain. For example, a heavy chain variable domain may include a single amino acid insert (residue 52a according to Kabat) after residue 52 of H2 and inserted residues (*e.g.*, residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence. It is not intended that CDRs of the present disclosure necessarily correspond to the Kabat numbering convention.

In some cases, the BCMA binding proteins comprise a heavy chain only antibody, such as a VH or a VHH domain. In some cases, the BCMA binding proteins comprise a heavy chain only antibody that is an engineered human VH domain. In some cases, the engineered human VH domain is produced by panning of phage display libraries. In some cases, the BCMA binding proteins comprise a VHH. The term "VHH," as used herein, refers to single chain antibody binding domain devoid of light chain. In some cases, a VHH is derived from an antibody of the type that can be found in Camelidae or cartilaginous fish which are naturally devoid of light chains or to a synthetic and non-immunized VHH which can be constructed accordingly. Each heavy chain comprises a variable region encoded by V-, D- and J exons. A VHH, in some cases, is a natural VHH, such as a Camelid-derived VHH, or a recombinant protein comprising a heavy chain variable domain. In some embodiments, the VHH is derived from a species selected from the group consisting of camels, llamas, vicugnas, guanacos, and cartilaginous fish (such as, but not limited to, sharks). In another embodiment, the VHH is derived from an alpaca (such as, but not limited to, a Huacaya Alpaca or a Suri alpaca).

As used herein, the term "Percent (%) amino acid sequence identity" with respect to a sequence is defined as the percentage of amino acid residues in a candidate sequence that are identical with the amino acid residues in the specific sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software programs such as EMBOSS MATCHER, EMBOSS WATER, EMBOSS STRETCHER, EMBOSS NEEDLE, EMBOSS LALIGN, BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

As used herein, "elimination half-time" is used in its ordinary sense, as is described in Goodman and Gillman's The Pharmaceutical Basis of Therapeutics 21-25 (Alfred Goodman Gilman, Louis S. Goodman, and Alfred Gilman, eds., 6th ed. 1980). Briefly, the term is meant to encompass a quantitative measure of the time course of drug elimination. The elimination of most drugs is exponential *(i.e.,* follows first-order kinetics), since drug concentrations usually do not approach those required for saturation of the elimination process. The rate of an exponential process may be expressed by its rate constant, k, which expresses the fractional change per unit of time, or by its half-time, t_{1/2} the time required for 50% completion of the process. The units of these two constants are time⁻¹ and time, respectively. A first-order rate constant and the half-time of the reaction are simply related (k×t_{1/2}=0.693) and may be interchanged accordingly. Since first-order elimination kinetics dictates that a constant fraction of drug is lost per unit time, a plot of the log of drug concentration versus time is linear at all times following the initial distribution phase (i.e. after drug absorption and distribution are complete). The half-time for drug elimination can be accurately determined from such a graph.

As used herein, the term "binding affinity" refers to the affinity of the proteins described in the disclosure to their binding targets, and is expressed numerically using "Kd" values. If two or more proteins are indicated to have comparable binding affinities towards their binding targets, then the Kd values for binding of the respective proteins towards their binding targets, are within ±2-fold of each other. If two or more proteins are indicated to have comparable binding affinities towards single binding target, then the Kd values for binding of the respective proteins towards said single binding target, are within ±2-fold of each other. If a protein is indicated to bind two or more targets with comparable binding affinities, then the Kd values for binding of said protein to the two or more targets are within ±2-fold of each other. In general, a higher Kd value corresponds to a weaker binding. In some embodiments, the "Kd" is measured by a radiolabeled antigen binding assay (RIA) or surface plasmon resonance assays using a BIACORE^{®}-2000 or a BIACORE^{®}-3000 (BIAcore, Inc., Piscataway, N.J.). In certain embodiments, an "on-rate" or "rate of association" or "association rate" or "kon" and an "off-rate" or "rate of dissociation" or "dissociation rate" or "koff" are also determined with the surface plasmon resonance technique using a BIACORE^{®}-2000 or a BIACORE^{®}-3000 (BIAcore, Inc., Piscataway, N.J.). In additional embodiments, the "Kd", "kon", and "koff" are measured using the OCTET^{®} Systems (Pall Life Sciences). In an exemplary method for measuring binding affinity using the OCTET^{®} Systems, the ligand, e.g., biotinylated human BCMA, is immobilized on the OCTET^{®} streptavidin capillary sensor tip surface which streptavidin tips are then activated according to manufacturer's instructions using about 20-50 µg/ml human BCMA protein. A solution of PBS/Casein is also introduced as a blocking agent. For association kinetic measurements, BCMA binding protein variants are introduced at a concentration ranging from about 10 ng/mL to about 100 µg/mL, about 50 ng/mL to about 5 µg/mL, or about 2 ng/mL to about 20 µg/mL. In some embodiments, the BCMA binding single domain proteins are used at a concentration ranging from about 2 ng/mL to about 20 µg/mL. Complete dissociation is observed in case of the negative control, assay buffer without the binding proteins. The kinetic parameters of the binding reactions are then determined using an appropriate tool, e.g., ForteBio software.

Disclosed herein are pharmaceutical compositions as well as nucleic acids, recombinant expression vectors, and host cells for making BCMA binding proteins. Also disclosed herein are methods of using the disclosed BCMA binding proteins in the prevention, and/or treatment of diseases, conditions and disorders. The BCMA binding proteins are capable of specifically binding to BCMA. In some embodiments, the BCMA binding proteins include additional domains, such as a CD3 binding domain and an albumin binding domain.

### B cell maturation antigen (BCMA)

B cell maturation antigen (BCMA, TNFRSF17, CD269) is a transmembrane protein belonging to the tumor necrosis family receptor (TNFR) super family that is primarily expressed on terminally differentiated B cells. BCMA expression is restricted to the B cell lineage and mainly present on plasma cells and plasmablasts and to some extent on memory B cells, but virtually absent on peripheral and naive B cells. BCMA is also expressed on multiple myeloma (MM) cells, on leukemia cells and lymphoma cells.

BCMA was identified through molecular analysis of a t(4;16)(q26;p13) translocation found in a human intestinal T cell lymphoma and an in-frame sequence was mapped to the 16p13.1 chromosome band.

Human BCMA cDNA has an open reading frame of 552 bp that encodes a 184 amino acid polypeptide. The BCMA gene is organized into three exons that are separated by two introns, each flanked by GT donor and AG acceptor consensus splicing sites, and codes for a transcript of 1.2 kb. The structure of BCMA protein includes an integral transmembrane protein based on a central 24 amino acid hydrophobic region in an alpha-helix structure.

The murine BCMA gene is located on chromosome 16 syntenic to the human 16p13 region, and also includes three exons that are separated by two introns. The gene encodes a 185 amino acid protein. Murine BCMA mRNA is expressed as a 404 bp transcript at the highest levels in plasmacytoma cells (J558) and at modest levels in the A20 B cell lymphoma line. Murine BCMA mRNA transcripts have also been detected at low levels in T cell lymphoma (EL4, BW5147) and dendritic cell (CB1D6, D2SC1) lines in contrast to human cell lines of T cell and dendritic cell origin. The murine BCMA cDNA sequence has 69.3% nucleotide identity with the human BCMA cDNA sequence and slightly higher identity (73.7%) when comparing the coding regions between these two cDNA sequences. Mouse BCMA protein is 62% identical to human BCMA protein and, like human BCMA, contains a single hydrophobic region, which may be an internal transmembrane segment. The N-terminal 40 amino acid domain of both murine and human BCMA protein have six conserved cysteine residues, consistent with the formation of a cysteine repeat motif found in the extracellular domain of TNFRs. Similar to members of the TNFR superfamily, BCMA protein contains a conserved aromatic residue four to six residues C-terminal from the first cysteine.

BCMA is not expressed at the cell surface, but rather, is located on the Golgi apparatus. The amount of BCMA expression is proportional to the stage of cellular differentiation (highest in plasma cells).

BCMA is involved in B cell development and homeostasis due to its interaction with its ligands BAFF (B cell activating factor, also designated as TALL-1 or TNFSF13B) and APRIL (A proliferation inducing ligand).

BCMA regulates different aspects of humoral immunity, B cell development and homeostasis along with its family members TACI (transmembrane activator and cyclophylin ligand interactor) and BAFF-R (B cell activation factor receptor, also known as tumor necrosis factor receptor superfamily member 13C). Expression of BCMA appears rather late in B cell differentiation and contributes to the long term survival of plasmablasts and plasma cells in the bone marrow. BCMA also supports growth and survival of multiple myeloma (MM) cells.

BCMA is mostly known for its functional activity in mediating the survival of plasma cells that maintain long-term humoral immunity.

There is a need for having treatment options for solid tumor diseases related to the overexpression of BCMA, such as cancer multiple myeloma, leukemias and lymphomas. The present disclosure provides, in certain embodiments, single domain proteins which specifically bind to BCMA on the surface of tumor target cells.

### BCMA binding proteins

Disclosed herein are BCMA binding proteins, in particular anti-BCMA single domain antibodies or antibody variants, which bind to an epitope in the BCMA protein. In some embodiments, the BCMA binding protein binds to a human BCMA protein comprising the sequence of SEQ ID NO: 468. In some embodiments, the BCMA binding protein binds to a human BCMA protein comprising a truncated sequence compared to SEQ ID NO: 468. In one non-limiting example, the BCMA binding protein binds to a human BCMA protein comprising amino acid residues 5-51 of SEQ ID NO: 468. The claimed invention provides single domain BCMA binding proteins that comprise the sequences set out in the claims.

The BCMA binding proteins of the present disclosure can be expressed within a multidomain protein that includes additional immunoglobulin domains. Such multidomain proteins can act via immunotoxin-based inhibition of tumor growth and induction of antibody-dependent cellular cytotoxicity (ADCC). In some embodiments, the multidomain proteins containing the BCMA binding proteins of the present disclosure exhibit complement-dependent cytotoxicity (CDC) activity. In some embodiments, the multidomain proteins containing the BCMA binding proteins of the present disclosure exhibit both ADCC and CDC activity, against cancer cells expressing BCMA. An amino acid sequence of a Fc domain can be added on to the BCMA binding proteins described herein to induce ACDD or CDC. Amino acid sequences of Fc domains are known in the art and are contemplated herein.

A BCMA binding protein described herein binds to the extracellular domain of BCMA. In one instance, a BCMA binding protein described herein binds to amino acid residues 5-51 of human BCMA.

In some embodiments, the BCMA binding protein is an anti-BCMA antibody or an antibody variant. As used herein, the term "antibody variant" refers to variants and derivatives of an antibody described herein. In certain embodiments, amino acid sequence variants of the anti-BCMA antibodies described herein are contemplated. For example, in certain embodiments amino acid sequence variants of anti-BCMA antibodies described herein are contemplated to improve the binding affinity and/or other biological properties of the antibodies. Exemplary methods for preparing amino acid variants include, but are not limited to, introducing appropriate modifications into the nucleotide sequence encoding the antibody, or by peptide synthesis. Such modifications include, for example, deletions from, and/or insertions into and/or substitutions of residues within the amino acid sequences of the antibody.

Any combination of deletion, insertion, and substitution can be made to arrive at the final construct, provided that the final construct possesses the desired characteristics, e.g., antigen- binding. In certain embodiments, antibody variants having one or more amino acid substitutions are provided. Sites of interest for substitution mutagenesis include the CDRs and framework regions. Examples of such substitutions are described below. Amino acid substitutions may be introduced into an antibody of interest and the products screened for a desired activity, e.g., retained/improved antigen binding, decreased immunogenicity, or improved antibody-dependent cell mediated cytotoxicity (ADCC) or complement dependent cytotoxicity (CDC). Both conservative and non-conservative amino acid substitutions are contemplated for preparing the antibody variants.

In another example of a substitution to create a variant anti-BCMA antibody, one or more hypervariable region residues of a parent antibody are substituted. In general, variants are then selected based on improvements in desired properties compared to a parent antibody, for example, increased affinity, reduced affinity, reduced immunogenicity, increased pH dependence of binding. For example, an affinity matured variant antibody can be generated, *e.g.,* using phage display-based affinity maturation techniques such as those described herein and known in the field.

In some embodiments, the BCMA binding protein described herein is a single domain antibody such as a heavy chain variable domain (VH), a variable domain (VHH) of llama derived sdAb, peptide, ligand or a small molecule entity specific for BCMA. In some embodiments, the BCMA binding domain of the BCMA binding protein described herein is any domain that binds to BCMA including, but not limited to, domains from a monoclonal antibody, a polyclonal antibody, a recombinant antibody, a human antibody, a humanized antibody. In certain embodiments, the BCMA binding protein is a single-domain antibody. In other embodiments, the BCMA binding protein is a peptide. In further embodiments, the BCMA binding protein is a small molecule.

Generally, it should be noted that the term single domain antibody as used herein in its broadest sense is not limited to a specific biological source or to a specific method of preparation. Single domain antibodies are antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, and bovine. For example, in some embodiments, the single domain antibodies of the disclosure are obtained: (1) by isolating the VHH domain of a naturally occurring heavy chain antibody; (2) by expression of a nucleotide sequence encoding a naturally occurring VHH domain; (3) by "humanization" of a naturally occurring VHH domain or by expression of a nucleic acid encoding a such humanized VHH domain; (4) by "camelization" of a naturally occurring VH domain from any animal species, and in particular from a species of mammal, such as from a human being, or by expression of a nucleic acid encoding such a camelized VH domain; (5) by "camelisation" of a "domain antibody" or "Dab", or by expression of a nucleic acid encoding such a camelized VH domain; (6) by using synthetic or semi-synthetic techniques for preparing proteins, polypeptides or other amino acid sequences; (7) by preparing a nucleic acid encoding a single domain antibody using techniques for nucleic acid synthesis known in the field, followed by expression of the nucleic acid thus obtained; and/or (8) by any combination of one or more of the foregoing.

In one embodiment, a single domain antibody corresponds to the VHH domains of naturally occurring heavy chain antibodies directed against BCMA. As further described herein, such VHH sequences can generally be generated or obtained by suitably immunizing a species of Llama with BCMA, *(i.e.,* so as to raise an immune response and/or heavy chain antibodies directed against BCMA), by obtaining a suitable biological sample from said Llama (such as a blood sample, serum sample or sample of B-cells), and by generating VHH sequences directed against BCMA, starting from said sample, using any suitable technique known in the field.

In another embodiment, such naturally occurring VHH domains against BCMA, are obtained from naive libraries of Camelid VHH sequences, for example by screening such a library using BCMA, or at least one part, fragment, antigenic determinant or epitope thereof using one or more screening techniques known in the field. Such libraries and techniques are, for example, described in WO 99/37681, WO 01/90190, WO 03/025020 and WO 03/035694. Alternatively, improved synthetic or semi-synthetic libraries derived from naive VHH libraries are used, such as VHH libraries obtained from naive VHH libraries by techniques such as random mutagenesis and/or CDR shuffling, as for example, described in WO 00/43507.

In a further embodiment, yet another technique for obtaining VHH sequences directed against BCMA, involves suitably immunizing a transgenic mammal that is capable of expressing heavy chain antibodies *(i.e.,* so as to raise an immune response and/or heavy chain antibodies directed against BCMA), obtaining a suitable biological sample from said transgenic mammal (such as a blood sample, serum sample or sample of B-cells), and then generating VHH sequences directed against BCMA, starting from said sample, using any suitable technique known in the field. For example, for this purpose, the heavy chain antibody-expressing rats or mice and the further methods and techniques described in WO 02/085945 and in WO 04/049794 can be used.

In some embodiments, an anti-BCMA antibody, as described herein comprises single domain antibody with an amino acid sequence that corresponds to the amino acid sequence of a naturally occurring VHH domain, but that has been "humanized", *i.e.,* by replacing one or more amino acid residues in the amino acid sequence of said naturally occurring VHH sequence (and in particular in the framework sequences) by one or more of the amino acid residues that occur at the corresponding position(s) in a VH domain from a conventional 4-chain antibody from a human being (*e.g.,* as indicated above). This can be performed in a manner known in the field, which will be clear to the skilled person, for example on the basis of the further description herein. Again, it should be noted that such humanized anti-BCMA single domain antibodies of the disclosure are obtained in any suitable manner known per se (*i.e.,* as indicated under points (1)-(8) above) and thus are not strictly limited to polypeptides that have been obtained using a polypeptide that comprises a naturally occurring VHH domain as a starting material. In some additional embodiments, a single domain BCMA antibody, as described herein, comprises a single domain antibody with an amino acid sequence that corresponds to the amino acid sequence of a naturally occurring VH domain, but that has been "camelized", *i.e.,* by replacing one or more amino acid residues in the amino acid sequence of a naturally occurring VH domain from a conventional 4-chain antibody by one or more of the amino acid residues that occur at the corresponding position(s) in a VHH domain of a heavy chain antibody. Such "camelizing" substitutions are preferably inserted at amino acid positions that form and/or are present at the VH-VL interface, and/or at the so-called Camelidae hallmark residues (see for example WO 94/04678 and Davies and Riechmann (1994 and 1996)). Preferably, the VH sequence that is used as a starting material or starting point for generating or designing the camelized single domain is preferably a VH sequence from a mammal, more preferably the VH sequence of a human being, such as a VH3 sequence. However, it should be noted that such camelized anti-BCMA single domain antibodies of the disclosure, in certain embodiments, are obtained in any suitable manner known in the field (*i.e.,* as indicated under points (1)-(8) above) and thus are not strictly limited to polypeptides that have been obtained using a polypeptide that comprises a naturally occurring VH domain as a starting material. For example, as further described herein, both "humanization" and "camelization" are performed by providing a nucleotide sequence that encodes a naturally occurring VHH domain or VH domain, respectively, and then changing, one or more codons in said nucleotide sequence in such a way that the new nucleotide sequence encodes a "humanized" or "camelized" single domain antibody, respectively. This nucleic acid can then be expressed, so as to provide the desired anti-BCMA single domain antibody of the disclosure. Alternatively, in other embodiments, based on the amino acid sequence of a naturally occurring VHH domain or VH domain, respectively, the amino acid sequence of the desired humanized or camelized anti-BCMA single domain antibody of the disclosure, respectively, are designed and then synthesized de novo using known techniques for peptide synthesis. In some embodiments, based on the amino acid sequence or nucleotide sequence of a naturally occurring VHH domain or VH domain, respectively, a nucleotide sequence encoding the desired humanized or camelized anti-BCMA single domain antibody of the disclosure, respectively, is designed and then synthesized de novo using known techniques for nucleic acid synthesis, after which the nucleic acid thus obtained is expressed in using known expression techniques, so as to provide the desired anti-BCMA single domain antibody of the disclosure.

Other suitable methods and techniques for obtaining the anti-BCMA single domain antibody of the disclosure and/or nucleic acids encoding the same, starting from naturally occurring VH sequences or VHH sequences for example comprises combining one or more parts of one or more naturally occurring VH sequences (such as one or more framework (FR) sequences and/or complementarity determining region (CDR) sequences), one or more parts of one or more naturally occurring VHH sequences (such as one or more FR sequences or CDR sequences), and/or one or more synthetic or semi-synthetic sequences, in a suitable manner, so as to provide an anti-BCMA single domain antibody of the disclosure or a nucleotide sequence or nucleic acid encoding the same.

It is contemplated that in some embodiments the BCMA binding protein is fairly small and no more than 25 kD, no more than 20 kD, no more than 15 kD, or no more than 10 kD in some embodiments. In certain instances, the BCMA binding protein is 5 kD or less if it is a peptide or small molecule entity.

In some embodiments, the BCMA binding protein is an anti-BCMA specific antibody comprising a heavy chain variable complementarity determining region CDR1, a heavy chain variable CDR2, a heavy chain variable CDR3, a light chain variable CDR1, a light chain variable CDR2, and a light chain variable CDR3. In some embodiments, the BCMA binding protein comprises any domain that binds to BCMA including but not limited to domains from a monoclonal antibody, a polyclonal antibody, a recombinant antibody, a human antibody, a humanized antibody, or antigen binding fragments such as single domain antibodies (sdAb), Fab, Fab', F(ab)2, and Fv fragments, fragments comprised of one or more CDRs, single-chain antibodies (*e.g*., single chain Fv fragments (scFv)), disulfide stabilized (dsFv) Fv fragments, heteroconjugate antibodies (*e.g*., bispecific antibodies), pFv fragments, heavy chain monomers or dimers, light chain monomers or dimers, and dimers consisting of one heavy chain and one light chain. In some embodiments, the BCMA binding protein is a single domain antibody. In some embodiments, the anti-BCMA single domain antibody comprises heavy chain variable complementarity determining regions (CDR), CDR1, CDR2, and CDR3.

In some embodiments, the BCMA binding protein is a single domain antibody comprising the sequence of SEQ ID NO: 365. In some embodiments, the BCMA binding protein is a single domain antibody comprising the sequence of SEQ ID NO: 368.

In some embodiments, the BCMA binding protein is a single domain antibody comprising the sequence of SEQ ID NO: 374. In some embodiments, the BCMA binding protein is a single domain antibody comprising the sequence of SEQ ID NO: 376.

In some embodiments, the BCMA binding protein is a single domain antibody comprising the sequence of SEQ ID NO: 381. In some embodiments, the BCMA binding protein is a single domain antibody comprising the sequence of SEQ ID NO: 382. In some embodiments, the BCMA binding protein is a single domain antibody comprising the sequence of SEQ ID NO: 383. In some embodiments, the BCMA binding protein is a single domain antibody comprising the sequence of SEQ ID NO: 384. In some embodiments, the BCMA binding protein is a single domain antibody comprising the sequence of SEQ ID NO: 385. In some embodiments, the BCMA binding protein is a single domain antibody comprising the sequence of SEQ ID NO: 386. In some embodiments, the BCMA binding protein is a single domain antibody comprising the sequence of SEQ ID NO: 387. In some embodiments, the BCMA binding protein is a single domain antibody comprising the sequence of SEQ ID NO: 388. In some embodiments, the BCMA binding protein is a single domain antibody comprising the sequence of SEQ ID NO: 389.

In some embodiments, the BCMA binding protein is a single domain antibody comprising the sequence of SEQ ID NO: 390. In some embodiments, the BCMA binding protein is a single domain antibody comprising the sequence of SEQ ID NO: 391. In some embodiments, the BCMA binding protein is a single domain antibody comprising the sequence of SEQ ID NO: 393. In some embodiments, the BCMA binding protein is a single domain antibody comprising the sequence of SEQ ID NO: 394. In some embodiments, the BCMA binding protein is a single domain antibody comprising the sequence of SEQ ID NO: 395. In some embodiments, the BCMA binding protein is a single domain antibody comprising the sequence of SEQ ID NO: 396. In some embodiments, the BCMA binding protein is a single domain antibody comprising the sequence of SEQ ID NO: 397. In some embodiments, the BCMA binding protein is a single domain antibody comprising the sequence of SEQ ID NO: 398. In some embodiments, the BCMA binding protein is a single domain antibody comprising the sequence of SEQ ID NO: 399.

In some embodiments, the BCMA binding protein is a single domain antibody comprising the sequence of SEQ ID NO: 400. In some embodiments, the BCMA binding protein is a single domain antibody comprising the sequence of SEQ ID NO: 401. In some embodiments, the BCMA binding protein is a single domain antibody comprising the sequence of SEQ ID NO: 402. In some embodiments, the BCMA binding protein is a single domain antibody comprising the sequence of SEQ ID NO: 403. In some embodiments, the BCMA binding protein is a single domain antibody comprising the sequence of SEQ ID NO: 404. In some embodiments, the BCMA binding protein is a single domain antibody comprising the sequence of SEQ ID NO: 406. In some embodiments, the BCMA binding protein is a single domain antibody comprising the sequence of SEQ ID NO: 407. In some embodiments, the BCMA binding protein is a single domain antibody comprising the sequence of SEQ ID NO: 408.

In some embodiments, the BCMA binding protein is a single domain antibody comprising the sequence of SEQ ID NO: 411. In some embodiments, the BCMA binding protein is a single domain antibody comprising the sequence of SEQ ID NO: 412. In some embodiments, the BCMA binding protein is a single domain antibody comprising the sequence of SEQ ID NO: 413. In some embodiments, the BCMA binding protein is a single domain antibody comprising the sequence of SEQ ID NO: 414. In some embodiments, the BCMA binding protein is a single domain antibody comprising the sequence of SEQ ID NO: 416. In some embodiments, the BCMA binding protein is a single domain antibody comprising the sequence of SEQ ID NO: 419.

In some embodiments, the BCMA binding protein is a humanized single domain antibody comprising the sequence of SEQ ID NO: 421.

A BCMA binding protein described herein can bind to human BCMA with a Kd ranging from about 0.1 nM to about 500 nM. In some embodiments, the hKd ranges from about 0.1 nM to about 450 nM. In some embodiments, the hKd ranges from about 0.1 nM to about 400 nM. In some embodiments, the hKd ranges from about 0.1 nM to about 350 nM. In some embodiments, the hKd ranges from about 0.1 nM to about 300 nM. In some embodiments, the hKd ranges from about 0.1 nM to about 250 nM. In some embodiments, the hKd ranges from about 0.1 nM to about 200 nM. In some embodiments, the hKd ranges from about 0.1 nM to about 150 nM. In some embodiments, the hKd ranges from about 0.1 nM to about 100 nM. In some embodiments, the hKd ranges from about 0.1 nM to about 90 nM. In some embodiments, the hKd ranges from about 0.2 nM to about 80 nM. In some embodiments, the hKd ranges from about 0.3 nM to about 70 nM. In some embodiments, the hKd ranges from about 0.4 nM to about 50 nM. In some embodiments, the hKd ranges from about 0.5 nM to about 30 nM. In some embodiments, the hKd ranges from about 0.6 nM to about 10 nM. In some embodiments, the hKd ranges from about 0.7 nM to about 8 nM. In some embodiments, the hKd ranges from about 0.8 nM to about 6 nM. In some embodiments, the hKd ranges from about 0.9 nM to about 4 nM. In some embodiments, the hKd ranges from about 1 nM to about 2 nM.

In some embodiments, any of the foregoing BCMA binding proteins are affinity peptide tagged for ease of purification. In some embodiments, the affinity peptide tag is six consecutive histidine residues, also referred to as a His tag or a 6X-His tag (*e.g.,* SEQ ID NO: 471).

In certain embodiments, the BCMA binding proteins according to the present disclosure may be incorporated into BCMA targeting trispecific proteins. In some examples, the trispecific binding protein comprises a CD3 binding domain, a human serum albumin (HSA) binding domain and an anti-BCMA binding domain according to the present disclosure. In some instances, the trispecific binding protein comprises the domains described above in the following orientation: BCMA-HSA-CD3.

In certain embodiments, the BCMA binding proteins of the present disclosure preferentially bind membrane bound BCMA over soluble BCMA. Membrane bound BCMA refers to the presence of BCMA in or on the cell membrane surface of a cell that expresses BCMA. Soluble BCMA refers to BCMA that is no longer on in or on the cell membrane surface of a cell that expresses or expressed BCMA. In certain instances, the soluble BCMA is present in the blood and/or lymphatic circulation in a subject. In one embodiment, the BCMA binding proteins bind membrane-bound BCMA at least 5 fold, 10 fold, 15 fold, 20 fold, 25 fold, 30 fold, 40 fold, 50 fold, 100 fold, 500 fold, or 1000 fold greater than soluble BCMA. In one embodiment, the antigen binding proteins of the present disclosure preferentially bind membrane-bound BCMA 30 fold greater than soluble BCMA. Determining the preferential binding of an antigen binding protein to membrane bound BCMA over soluble BCMA can be readily determined using assays well known in the art.

### Integration into chimeric antigen receptors (CAR)

The BCMA binding proteins of the present disclosure, *e.g*., an anti-BCMA single domain antibody, can, in certain examples, be incorporated into a chimeric antigen receptor (CAR). An engineered immune effector cell, *e.g.,* a T cell or NK cell, can be used to express a CAR that includes an anti-BCMA single domain antibody as described herein. In one embodiment, the CAR including an anti-BCMA single domain antibody as described herein is connected to a transmembrane domain via a hinge region, and further a costimulatory domain, *e.g.,* a functional signaling domain obtained from OX40, CD27, CD28, CD5, ICAM-1, LFA-1 (CD11a/CD18), ICOS (CD278), or 4-1BB. In some embodiments, the CAR further comprises a sequence encoding an intracellular signaling domain, such as 4-1BB and/or CD3 zeta.

### Multispecific protein targeting BCMA

One embodiment provides a multispecific protein comprising a BCMA binding domain, wherein the BCMA binding domain is according to any one of above embodiments. In some embodiments, the multispecific protein comprises the BCMA binding domain according to any one of above embodiments (anti-BCMA domain), a CD3 binding domain (anti-CD3 domain), and an albumin binding domain (anti-ALB domain). In some embodiments, the BCMA targeting multispecific protein is a trispecific protein, wherein the trispecific protein has a domain order of H₂N-(C)-(A)-(B)-COOH. In some embodiments, the anti-BCMA domain (the anti-target domain, T), the anti-CD3 domain (C), and the anti-ALB domain (A) are in an anti-CD3: anti-ALB: anti-BCMA (CAT) orientation. In some embodiments, the anti-BCMA domain (the anti-target domain, T) the anti-CD3 domain (C), and the anti-ALB domain (A) are in an anti-BCMA: anti-ALB: anti-CD3 (TAC) orientation.

### Tumor growth reduction properties

In certain embodiments, the BCMA binding proteins of the disclosure reduces the growth of tumor cells *in vivo* when administered to a subject who has tumor cells that express BCMA. Measurement of the reduction of the growth of tumor cells can be determined by multiple different methodologies well known in the art. Non-limiting examples include direct measurement of tumor dimension, measurement of excised tumor mass and comparison to control subjects, measurement via imaging techniques (*e.g*., CT or MRI) that may or may not use isotopes or luminescent molecules (*e.g*., luciferase) for enhanced analysis, and the like. In specific embodiments, administration of the antigen binding agents of the disclosure results in a reduction of *in vivo* growth of tumor cells as compared to a control antigen binding agent by at least about 10%, 20%, 30%, 40%, 50%, 60%, 70%, 80%, 90% or 100%, with an about 100% reduction in tumor growth indicating a complete response and disappearance of the tumor. In further embodiments, administration of the antigen binding agents of the disclosure results in a reduction of *in vivo* growth of tumor cells as compared to a control antigen binding agent by about 50-100%, about 75-100% or about 90-100%. In further embodiments, administration of the antigen binding agents of the disclosure results in a reduction of *in vivo* growth of tumor cells as compared to a control antigen binding agent by about 50-60%, about 60-70%, about 70-80%, about 80-90%, or about 90-100%.

### BCMA binding protein modifications

The BCMA binding proteins described herein encompass derivatives or analogs in which (i) an amino acid is substituted with an amino acid residue that is not one encoded by the genetic code, (ii) the mature polypeptide is fused with another compound such as polyethylene glycol, or (iii) additional amino acids are fused to the protein, such as a leader or secretory sequence or a sequence to block an immunogenic domain and/or for purification of the protein.

Typical modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristylation, oxidation, proteolytic processing, phosphorylation, prenylation, racemization, selenoylation, sulfation, transfer-RNA mediated addition of amino acids to proteins such as arginylation, and ubiquitination.

Modifications are made anywhere in the BCMA binding proteins described herein, including the peptide backbone, the amino acid side-chains, and the amino or carboxyl termini. Certain common peptide modifications that are useful for modification of BCMA binding proteins include glycosylation, lipid attachment, sulfation, gamma-carboxylation of glutamic acid residues, hydroxylation, blockage of the amino or carboxyl group in a polypeptide, or both, by a covalent modification, and ADP-ribosylation.

### Polynucleotides encoding BCMA binding proteins

Disclosed herein are polynucleotide molecules encoding a BCMA binding protein as described herein. In some cases, the polynucleotide molecules are provided as DNA constructs. In other cases, the polynucleotide molecules are provided as messenger RNA transcripts.

The polynucleotide molecules are constructed by known methods such as by combining the genes encoding the anti-BCMA binding protein, operably linked to a suitable promoter, and optionally a suitable transcription terminator, and expressing it in bacteria or other appropriate expression system such as, for example CHO cells.

In some cases, the polynucleotide is inserted into a vector, preferably an expression vector. This recombinant vector can be constructed according to known methods. Vectors of particular interest include plasmids, phagemids, phage derivatives, virii (*e.g.,* retroviruses, adenoviruses, adeno-associated viruses, herpes viruses, lentiviruses, and the like), and cosmids.

A variety of expression vector/host systems may be utilized to contain and express the polynucleotide encoding the polypeptide of the described BCMA binding protein. Examples of expression vectors for expression in *E. coli* are pSKK (Le Gall et al., J Immunol Methods. (2004) 285(1):111-27), pcDNA5 (Invitrogen) for expression in mammalian cells, PICHIAPINK^{™} Yeast Expression Systems (Invitrogen), BACUVANCE^{™} Baculovirus Expression System (GenScript).

Thus, the BCMA binding proteins as described herein, in some embodiments, are produced by introducing a vector encoding the protein as described above into a host cell and culturing said host cell under conditions whereby the protein domains are expressed, may be isolated and, optionally, further purified.

### Pharmaceutical compositions

Disclosed herein are pharmaceutical compositions comprising a BCMA binding protein described herein, a vector comprising the polynucleotide encoding the polypeptide of the BCMA binding proteins or a host cell transformed by this vector and at least one pharmaceutically acceptable carrier. The term "pharmaceutically acceptable carrier" includes, but is not limited to, any carrier that does not interfere with the effectiveness of the biological activity of the ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Preferably, the compositions are sterile. These compositions may also contain adjuvants such as preservative, emulsifying agents and dispersing agents. Prevention of the action of microorganisms may be ensured by the inclusion of various antibacterial and antifungal agents. A further case provides one or more of the above described binding proteins, such as anti-BCMA single domain antibodies or antigen-binding fragments thereof packaged in lyophilized form, or packaged in an aqueous medium.

In some cases of the pharmaceutical compositions, the BCMA binding protein described herein is encapsulated in nanoparticles. In some cases, the nanoparticles are fullerenes, liquid crystals, liposome, quantum dots, superparamagnetic nanoparticles, dendrimers, or nanorods. In other cases of the pharmaceutical compositions, the BCMA binding protein is attached to liposomes. In some instances, the BCMA binding protein is conjugated to the surface of liposomes. In some instances, the BCMA binding protein is encapsulated within the shell of a liposome. In some instances, the liposome is a cationic liposome.

The BCMA binding proteins described herein are contemplated for use as a medicament. Administration is effected by different ways, *e.g.,* by intravenous, intraperitoneal, subcutaneous, intramuscular, topical or intradermal administration. In some embodiments, the route of administration depends on the kind of therapy and the kind of compound contained in the pharmaceutical composition. The dosage regimen will be determined by the attending physician and other clinical factors. Dosages for any one patient depends on many factors, including the patient's size, body surface area, age, sex, the particular compound to be administered, time and route of administration, the kind of therapy, general health and other drugs being administered concurrently. An "effective dose" refers to amounts of the active ingredient that are sufficient to affect the course and the severity of the disease, leading to the reduction or remission of such pathology and may be determined using known methods.

In some embodiments, the BCMA binding proteins of this disclosure are administered at a dosage of up to 10 mg/kg at a frequency of once a week. In some cases, the dosage ranges from about 1 ng/kg to about 10 mg/kg. In some embodiments, the dose is from about 1 ng/kg to about 10 ng/kg, about 5 ng/kg to about 15 ng/kg, about 12 ng/kg to about 20 ng/kg, about 18 ng/kg to about 30 ng/kg, about 25 ng/kg to about 50 ng/kg, about 35 ng/kg to about 60 ng/kg, about 45 ng/kg to about 70 ng/kg, about 65 ng/kg to about 85 ng/kg, about 80 ng/kg to about 1 µg/kg, about 0.5 µg/kg to about 5 µg/kg, about 2 µg/kg to about 10 µg/kg, about 7 µg/kg to about 15 µg/kg, about 12 µg/kg to about 25 µg/kg, about 20 µg/kg to about 50 µg/kg, about 35 µg/kg to about 70 µg/kg, about 45 µg/kg to about 80 µg/kg, about 65 µg/kg to about 90 µg/kg, about 85 µg/kg to about 0.1 mg/kg, about 0.095 mg/kg to about 10 mg/kg. In some cases, the dosage is about 0.1 mg/kg to about 0.2 mg/kg; about 0.25 mg/kg to about 0.5 mg/kg, about 0.45 mg/kg to about 1 mg/kg, about 0.75 mg/kg to about 3 mg/kg, about 2.5 mg/kg to about 4 mg/kg, about 3.5 mg/kg to about 5 mg/kg, about 4.5 mg/kg to about 6 mg/kg, about 5.5 mg/kg to about 7 mg/kg, about 6.5 mg/kg to about 8 mg/kg, about 7.5 mg/kg to about 9 mg/kg, or about 8.5 mg/kg to about 10 mg/kg. The frequency of administration, in some embodiments, is about less than daily, every other day, less than once a day, twice a week, weekly, once in 7 days, once in two weeks, once in two weeks, once in three weeks, once in four weeks, or once a month. In some cases, the frequency of administration is weekly. In some cases, the frequency of administration is weekly and the dosage is up to 10 mg/kg. In some cases, duration of administration is from about 1 day to about 4 weeks or longer.

### Methods of treatment

The references to the methods of treatment disclosed herein are to be interpreted as references to compounds of the invention for use in those methods. Also provided herein, in some embodiments, are methods and uses for stimulating the immune system of an individual in need thereof comprising administration of a BCMA binding protein as described herein. In some instances, the administration of a BCMA binding protein described herein induces and/or sustains cytotoxicity towards a cell expressing a target antigen. In some instances, the cell expressing a target antigen is a terminally differentiated B cell that is a cancer or tumor cell, or a metastatic cancer or tumor cell.

Also provided herein are methods and uses for a treatment of a disease, disorder or condition associated with BCMA comprising administering to an individual in need thereof a BCMA binding protein or a multispecific binding protein comprising the BCMA binding protein described herein.

Diseases, disorders or conditions associated with BCMA include, but are not limited to, a cancer or a metastasis that is of a B cell lineage.

Cancers that can be treated, prevented, or managed by the BCMA binding proteins of the present disclosure, and methods of using them, include but are not limited to a primary cancer or a metastatic cancer.

Examples of such leukemias include, but are not limited to,: acute lymphoblastic leukemia (ALL), acute myeloid leukemia (AML), chronic lymphocytic leukemia (CLL) and chronic myeloid leukemia (CML), as well as a number of less common types such as, for example, Hairy cell leukemia (HCL), T-cell prolymphocytic leukemia (T-PLL), Large granular lymphocytic leukemia and Adult T-cell leukemia, *etc.* Acute lymphoblastic leukemia (ALL) subtypes to be treated include, but are not limited to, precursor B acute lymphoblastic leukemia, precursor T acute lymphoblastic leukemia, Burkitt's leukemia, and acute biphenotypic leukemia. Chronic lymphocytic leukemia (CLL) subtypes to be treated include, but are not limited to, B-cell prolymphocytic leukemia. Acute myelogenous leukemia (AML) subtypes to be treated include, but are not limited to, acute promyelocytic leukemia, acute myeloblastic leukemia, and acute megakaryoblastic leukemia. Chronic myelogenous leukemia (CML) subtypes to be treated include, but are not limited to, chronic myelomonocytic leukemia.

Examples of a lymphoma to be treated with the subject methods include, but not limited to Hodgkin's disease, non-Hodgkin's disease, or any subtype of lymphoma.

Examples of such multiple myelomas include, but are not limited to, a multiple myeloma of the bone or other tissues including, for example, a smoldering multiple myeloma, a non-secretory myeloma, a osteosclerotic myeloma, *etc.*

For a review of such disorders, *see* Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

As used herein, in some embodiments, "treatment" or "treating" or "treated" refers to therapeutic treatment wherein the object is to slow (lessen) an undesired physiological condition, disorder or disease, or to obtain beneficial or desired clinical results. For the purposes described herein, beneficial or desired clinical results include, but are not limited to, alleviation of symptoms; diminishment of the extent of the condition, disorder or disease; stabilization (*i.e.,* not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment. In other embodiments, "treatment" or "treating" or "treated" refers to prophylactic measures, wherein the object is to delay onset of or reduce severity of an undesired physiological condition, disorder or disease, such as, for example is a person who is predisposed to a disease (*e.g.*, an individual who carries a genetic marker for a disease such as breast cancer).

In some embodiments of the methods described herein, the BCMA binding proteins as described herein are administered in combination with an agent for treatment of the particular disease, disorder or condition. Agents include, but are not limited to, therapies involving antibodies, small molecules (*e.g*., chemotherapeutics), hormones (steroidal, peptide, and the like), radiotherapies (γ-rays, X-rays, and/or the directed delivery of radioisotopes, microwaves, UV radiation and the like), gene therapies (*e.g.*, antisense, retroviral therapy and the like) and other immunotherapies. In some embodiments, a BCMA binding protein as described herein is administered in combination with anti-diarrheal agents, anti-emetic agents, analgesics, opioids and/or non-steroidal anti-inflammatory agents. In some embodiments, a BCMA binding protein as described herein is administered in combination with anti-cancer agents. Non-limiting examples of anti-cancer agents that can be used in the various embodiments of the disclosure, including pharmaceutical compositions and dosage forms and kits of the disclosure, include: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alpha-2a; interferon alpha-2b; interferon alpha-n1 interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinzolidine sulfate; vinzolidine sulfate; vorozole; zeniplatin; zinostatin; zorubicin hydrochloride. Other examples of anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-I receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; HMG-CoA reductase inhibitor (such as but not limited to, Lovastatin, Pravastatin, Fluvastatin, Statin, Simvastatin, and Atorvastatin); loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; VITAXIN^{®}; vorozole; zanoterone; zeniplatin; zilascorb; and zinostatin stimalamer. Additional anti-cancer drugs are 5-fluorouracil and leucovorin. These two agents are particularly useful when used in methods employing thalidomide and a topoisomerase inhibitor. In some embodiments, the anti-BCMA single domain binding protein of the present disclosure is used in combination with gemcitabine.

In some embodiments, a BCMA binding proteins as described herein is administered before, during, or after surgery.

In some embodiments, the anti-cancer agent is conjugated via any suitable means to the trispecific protein.

### Methods of detection of BCMA expression and diagnosis of BCMA associated cancer

Disclosed herein are kits for detecting expression of BCMA *in vitro* or *in vivo.* The kits include the foregoing BCMA binding proteins (*e.g.,* a labeled anti-BCMA single domain antibody or antigen binding fragments thereof), and one or more compounds for detecting the label. In some cases, the label is selected from the group consisting of a fluorescent label, an enzyme label, a radioactive label, a nuclear magnetic resonance active label, a luminescent label, and a chromophore label.

In some cases, BCMA expression is detected in a biological sample. The sample can be any sample, including, but not limited to, tissue from biopsies, autopsies and pathology specimens. Biological samples also include sections of tissues, for example, frozen sections taken for histological purposes. Biological samples further include body fluids, such as blood, serum, plasma, sputum, spinal fluid or urine. A biological sample is typically obtained from a mammal, such as a human or non-human primate.

In one case, provided is a method of determining if a subject has cancer by contacting a sample from the subject with an anti-BCMA single domain antibody as disclosed herein; and detecting binding of the single domain antibody to the sample. An increase in binding of the antibody to the sample as compared to binding of the antibody to a control sample identifies the subject as having cancer.

In another case, provided is a method of confirming a diagnosis of cancer in a subject by contacting a sample from a subject diagnosed with cancer with an anti-BCMA single domain antibody as disclosed herein; and detecting binding of the antibody to the sample. An increase in binding of the antibody to the sample as compared to binding of the antibody to a control sample confirms the diagnosis of cancer in the subject.

In some examples of the disclosed methods, the single domain antibody is directly labeled.

In some examples, the methods further include contacting a second antibody that specifically binds the single domain antibody with the sample; and detecting the binding of the second antibody. An increase in binding of the second antibody to the sample as compared to binding of the second antibody to a control sample detects cancer in the subject or confirms the diagnosis of cancer in the subject.

In some cases, the cancer is a lymphoma, a leukemia or a multiple myeloma.

In some examples, the control sample is a sample from a subject without cancer. In particular examples, the sample is a blood or tissue sample.

In some cases, the antibody that binds (for example specifically binds) BCMA is directly labeled with a detectable label. In another case, the antibody that binds (for example, specifically binds) BCMA (the first antibody) is unlabeled and a second antibody or other molecule that can bind the antibody that specifically binds BCMA is labeled. A second antibody is chosen such that it is able to specifically bind the specific species and class of the first antibody. For example, if the first antibody is a llama IgG, then the secondary antibody may be an anti-llama-IgG. Other molecules that can bind to antibodies include, without limitation, Protein A and Protein G, both of which are available commercially. Suitable labels for the antibody or secondary antibody are described above, and include various enzymes, prosthetic groups, fluorescent materials, luminescent materials, magnetic agents and radioactive materials. Non-limiting examples of suitable enzymes include horseradish peroxidase, alkaline phosphatase, beta-galactosidase, or acetylcholinesterase. Non-limiting examples of suitable prosthetic group complexes include streptavidin/biotin and avidin/biotin. Non- limiting examples of suitable fluorescent materials include umbelliferone, fluorescein, fluorescein isothiocyanate, rhodamine, dichlorotriazinylamine fluorescein, dansyl chloride or phycoerythrin. A non-limiting exemplary luminescent material is luminol; a non-limiting exemplary a magnetic agent is gadolinium, and non-limiting exemplary radioactive labels include 125I, 131I, 35S or 3H.

In an alternative case, BCMA can be assayed in a biological sample by a competition immunoassay utilizing BCMA standards labeled with a detectable substance and an unlabeled antibody that specifically binds BCMA. In this assay, the biological sample, the labeled BCMA standards and the antibody that specifically bind BCMA are combined and the amount of labeled BCMA standard bound to the unlabeled antibody is determined. The amount of BCMA in the biological sample is inversely proportional to the amount of labeled BCMA standard bound to the antibody that specifically binds BCMA.

The immunoassays and method disclosed herein can be used for a number of purposes. In one case, the antibody that specifically binds BCMA may be used to detect the production of BCMA in cells in cell culture. In another case, the antibody can be used to detect the amount of BCMA in a biological sample, such as a tissue sample, or a blood or serum sample. In some examples, the BCMA is cell-surface BCMA. In other examples, the BCMA is soluble BCMA (*e.g.,* BCMA in a cell culture supernatant or soluble BCMA in a body fluid sample, such as a blood or serum sample).

In one case, a kit is provided for detecting BCMA in a biological sample, such as a blood sample or tissue sample. For example, to confirm a cancer diagnosis in a subject, a biopsy can be performed to obtain a tissue sample for histological examination. Alternatively, a blood sample can be obtained to detect the presence of soluble BCMA protein or fragment. Kits for detecting a polypeptide will typically comprise a single domain antibody, according to the present disclosure, that specifically binds BCMA. In some cases, an antibody fragment, such as a scFv fragment, a VH domain, or a Fab is included in the kit. In a further case, the antibody is labeled (for example, with a fluorescent, radioactive, or an enzymatic label).

In one case, a kit includes instructional materials disclosing means of use of an antibody that binds BCMA. The instructional materials may be written, in an electronic form (such as a computer diskette or compact disk) or may be visual (such as video files), or provided through an electronic network, for example, over the internet, World Wide Web, an intranet, or other network. The kits may also include additional components to facilitate the particular application for which the kit is designed. Thus, for example, the kit may additionally contain means of detecting a label (such as enzyme substrates for enzymatic labels, filter sets to detect fluorescent labels, appropriate secondary labels such as a secondary antibody, or the like). The kits may additionally include buffers and other reagents routinely used for the practice of a particular method. Such kits and appropriate contents are well known to those of skill in the art.

In one case, the diagnostic kit comprises an immunoassay. Although the details of the immunoassays may vary with the particular format employed, the method of detecting BCMA in a biological sample generally includes the steps of contacting the biological sample with an antibody which specifically reacts, under immunologically reactive conditions, to a BCMA polypeptide. The antibody is allowed to specifically bind under immunologically reactive conditions to form an immune complex, and the presence of the immune complex (bound antibody) is detected directly or indirectly.

Methods of determining the presence or absence of a cell surface marker are well known in the art. For example, the antibodies can be conjugated to other compounds including, but not limited to, enzymes, magnetic beads, colloidal magnetic beads, haptens, fluorochromes, metal compounds, radioactive compounds or drugs. The antibodies can also be utilized in immunoassays such as but not limited to radioimmunoassays (RIAs), ELISA, or immunohistochemical assays. The antibodies can also be used for fluorescence activated cell sorting (FACS). FACS employs a plurality of color channels, low angle and obtuse light-scattering detection channels, and impedance channels, among other more sophisticated levels of detection, to separate or sort cells (see U.S. Patent No. 5, 061,620). Any of the single domain antibodies that bind BCMA, as disclosed herein, can be used in these assays. Thus, the antibodies can be used in a conventional immunoassay, including, without limitation, an ELISA, an RIA, FACS, tissue immunohistochemistry, Western blot or immunoprecipitation.

### EXAMPLES

The following examples illustrate the invention and its broader context. Elements not falling within the scope of the claims are provided to illustrate the said broader context. It is immediately clear to the skilled person which of the exemplified sequences fall within the scope of the claims, by reference to the sequence table at the end of the Examples. Any sequences not falling within the scope of the claims are disclosed for information.

### Example 1

### Ability of an exemplar anti-BCMA trispecific domain antibody containing a BCMA binding protein of the present disclosure to mediate T cell killing of cancer cells expressing BCMA, in TDCC (T cell dependent cell cytotoxic) assays

### Protein Production

Sequences of BCMA targeting trispecific molecules, containing a BCMA binding protein according to the present disclosure, were cloned into mammalian expression vector pcDNA 3.4 (Invitrogen) preceded by a leader sequence and followed by a 6x Histidine Tag (SEQ ID NO: 471). Expi293 cells (Life Technologies A14527) were maintained in suspension in Optimum Growth Flasks (Thomson) between 0.2 to 8 x 1e6 cells/mL in Expi293 media. Purified plasmid DNA was transfected into Expi293 cells in accordance with Expi293 Expression System Kit (Life Technologies, A14635) protocols, and maintained for 4-6 days post transfection. The amount of the exemplary trispecific proteins being tested, in the conditioned media, from the transfected Expi293 cells was quantitated using an Octet instrument with Protein A tips and using a control trispecific protein for a standard curve.

### T cell dependent cellular cytotoxicity assays

Titrations of conditioned media was added to TDCC assays (T cell Dependent Cell Cytotoxicity assays) to assess whether the anti-BCMA single domain antibody is capable of forming a synapse between T cells and a BCMA-expressing cell line and direct the T cells to kill the BCMA-expressing cell line. In this assay (Nazarian et al., 2015. J. Biomol. Screen., 20:519-27), T cells and target cancer cell line cells were mixed together at a 10:1 ratio in a 384-well plate, and varying amounts of the trispecific proteins being tested were added. The tumor cell lines were engineered to express luciferase protein. After 48 hours, to quantitate the remaining viable tumor cells, STEADY-GLO^{®} Luminescent Assay (Promega) was used.

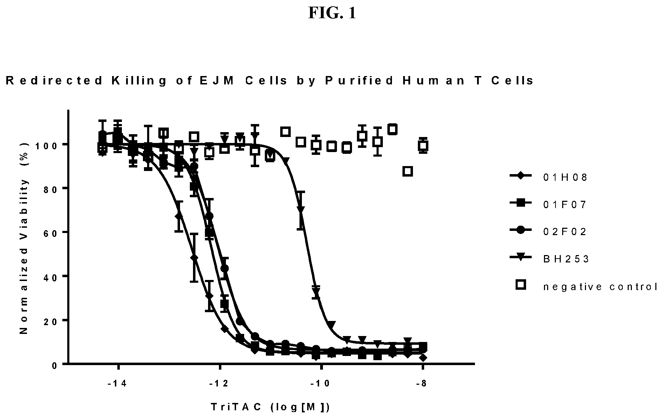

In this example EJM cells were used, which is a cell line that serves as an *in vitro* model for multiple myeloma and plasma cell leukemia. Viability of the EJM cells is measured after 48 hours. It was seen that the trispecific proteins mediated T cell killing. **Fig. 1** shows an example cell viability assay with test proteins 01H08, 01F07, 02F02 and BH253 compared to a negative control. The EC₅₀ for the TDCC activity of several other test trispecific proteins are listed below in **Table 1.**

### Binding affinity

In the instant study, the binding affinity to human BCMA protein of the BCMA targeting trispecific proteins containing a BCMA binding protein according to the present disclosure was determined. The affinity measurements are listed in **Table 1**.

**Table 1: Binding affinity and TDCC Activity of several BCMA targeting trispecific proteins.**

| **Construct Name** | **Human BCMA K_{D} (M)** | **TDCC EC50 (M)** |
|---|---|---|
| 253BH10 | 2.77E-08 | 5.29E-11 |
| 01H08 | 2.86E-09 | 3.41E-13 |
| 01F07 | 4.18E-09 | 7.02E-13 |
| 01H06 | ND | 1.00E-12 |
| 02G02 | 5.26E-09 | 1.08E-12 |
| 02B05 | 5.39E-09 | 1.22E-12 |
| 01C01 | 6.52E-09 | 1.33E-12 |
| 02F02 | 6.73E-09 | 1.36E-12 |
| 02E05 | 6.53E-09 | 1.37E-12 |
| 01E08 | 5.56E-09 | 1.50E-12 |
| 02C01 | 5.31E-09 | 1.55E-12 |
| 02E06 | 6.31E-09 | 1.57E-12 |
| 02B06 | 6.77E-09 | 1.65E-12 |
| 02F04 | 6.75E-09 | 1.72E-12 |
| 01G08 | 6.27E-09 | 1.91E-12 |
| 02C06 | 6.90E-09 | 1.95E-12 |
| 01H09 | 5.44E-09 | 2.21E-12 |
| 01F04 | 6.55E-09 | 2.21E-12 |
| 01D02 | 7.35E-09 | 2.25E-12 |
| 02D11 | 6.71E-09 | 2.35E-12 |
| 01A07 | 6.95E-09 | 2.49E-12 |
| 02C03 | 7.09E-09 | 2.52E-12 |
| 02F07 | 7.06E-09 | 2.59E-12 |
| 01E04 | 7.29E-09 | 2.67E-12 |
| 02H09 | 6.83E-09 | 2.88E-12 |
| 01E03 | 6.36E-09 | 2.98E-12 |
| 02F05 | 7.15E-09 | 3.00E-12 |
| 01B05 | 6.52E-09 | 3.01E-12 |
| 01C05 | 6.09E-09 | 3.07E-12 |
| 02F12 | 7.76E-09 | 3.14E-12 |
| 01H11 | 7.06E-09 | 3.17E-12 |
| 02G06 | 7.50E-09 | 3.39E-12 |
| 01E06 | 8.91E-09 | 3.77E-12 |
| 01G11 | 9.70E-09 | 3.98E-12 |
| 02A05 | 7.06E-09 | 4.21E-12 |
| 01A08 | 1.17E-08 | 4.25E-12 |
| 02G05 | 7.12E-09 | 4.33E-12 |
| 01B09 | 1.12E-08 | 5.27E-12 |
| 01G01 | 1.46E-08 | 5.83E-12 |
| 01B06 | 9.10E-09 | 6.97E-12 |
| 01F10 | 1.44E-08 | 7.44E-12 |
| 01E05 | 1.17E-08 | 1.08E-11 |
| 02G01 | 1.63E-08 | 1.08E-11 |
| 01A06 | 1.58E-08 | 1.10E-11 |
| 02B04 | 1.52E-08 | 1.13E-11 |
| 01D06 | 1.49E-08 | 1.35E-11 |
| 02B07 | 1.58E-08 | 1.42E-11 |
| 02B11 | 1.33E-08 | 1.44E-11 |
| 01H04 | 1.74E-08 | 1.47E-11 |
| 01D03 | 2.09E-08 | 1.49E-11 |
| 01A05 | 1.70E-08 | 1.51E-11 |
| 02F11 | 2.00E-08 | 1.52E-11 |
| 01D04 | 1.89E-08 | 1.60E-11 |
| 01B04 | 1.86E-08 | 1.61E-11 |
| 02C05 | 1.56E-08 | 1.62E-11 |
| 02E03 | 1.68E-08 | 1.65E-11 |
| 01D05 | 1.78E-08 | 1.66E-11 |
| 01C04 | 2.16E-08 | 1.75E-11 |
| 01E07 | 1.99E-08 | 1.92E-11 |
| 01G06 | 1.70E-08 | 1.92E-11 |
| 02F06 | 2.19E-08 | 1.93E-11 |
| 01B01 | 1.99E-08 | 1.95E-11 |
| 01D07 | 1.93E-08 | 1.96E-11 |
| 02A08 | 9.51E-09 | 2.01E-11 |
| 01A02 | 2.15E-08 | 2.18E-11 |
| 02G11 | 2.05E-08 | 2.38E-11 |
| 01G04 | 1.17E-08 | 2.41E-11 |
| 02F03 | 2.57E-08 | 2.45E-11 |
| 01C06 | 1.88E-08 | 2.51E-11 |
| 01A01 | 2.13E-08 | 2.64E-11 |
| 01B12 | 2.07E-08 | 2.73E-11 |
| 02A07 | 1.84E-08 | 2.79E-11 |
| 02G08 | 1.80E-08 | 2.86E-11 |
| 02E09 | 2.09E-08 | 3.11E-11 |
| 02H06 | 2.33E-08 | 3.19E-11 |
| 01H10 | 2.48E-08 | 3.52E-11 |
| 01F05 | 1.67E-08 | 3.72E-11 |
| 01C02 | 2.00E-08 | 3.73E-11 |
| 02A04 | 1.76E-08 | 3.82E-11 |
| 02H05 | 1.96E-08 | 3.89E-11 |
| 02G09 | 3.44E-08 | 3.96E-11 |
| 02D06 | 2.33E-08 | 4.28E-11 |
| 02G07 | 1.93E-08 | 4.46E-11 |
| 01H05 | 2.74E-08 | 4.54E-11 |
| 01C08 | 2.83E-08 | 4.57E-11 |
| 01A03 | 3.08E-08 | 4.61E-11 |
| 01A09 | 2.39E-08 | 4.84E-11 |
| 02B01 | 2.14E-08 | 5.18E-11 |
| 02H01 | 3.56E-08 | 5.42E-11 |
| 02H04 | 3.11E-08 | 5.99E-11 |
| 02A11 | 2.52E-08 | 6.06E-11 |
| 01E10 | 1.85E-08 | 6.23E-11 |
| 02D09 | 2.89E-08 | 6.73E-11 |
| 01F08 | 2.14E-08 | 7.12E-11 |
| 01F03 | 1.50E-08 | 7.64E-11 |
| 02H11 | 2.75E-08 | 7.75E-11 |
| 01C07 | 1.98E-08 | 8.33E-11 |
| 01B08 | 2.56E-08 | 8.76E-11 |
| 01B03 | 2.62E-08 | 9.64E-11 |
| 01H01 | 3.59E-08 | 1.18E-10 |
| 02B12 | 2.52E-08 | 1.24E-10 |
| 01G10 | 4.19E-08 | 1.43E-10 |
| 01A04 | 3.75E-08 | 1.59E-10 |
| 01B07 | 4.39E-08 | 1.74E-10 |
| 01C10 | 4.64E-08 | 2.08E-10 |
| 01F02 | 4.13E-08 | 2.25E-10 |
| 01B02 | 1.88E-08 | 3.59E-10 |
| 01F12 | 4.05E-08 | 3.92E-10 |
| 01G09 | 8.78E-08 | 4.41E-10 |
| 01D10 | 5.39E-08 | 4.53E-10 |
| 01F09 | 5.28E-08 | 9.45E-10 |

### ND: Not determined.

Molecules 01H08, 01F07, 01H06, 02G02, 02B05, 01C01, 02F02, 02E05, 01E08, 02C01, 02E06, 02B06, 02F04, 01G08, 02C06, 01H09, 01F04, 01D02, 02D11, 01A07, 02C03, 02F07, 01E04, 02H09, 01E03, 02F05, 01B05, 01C05, 02F12, 01H11, 02G06, 01E06, 01G11, 02A05, 01A08, 02G05, 01B09, 01G01, 01B06, 01F10, 01E05, 02G01, 01A06, 02B04, 01D06, 02B07, 02B11, 01H04, 01D03, 01A05, 02F11, 01D04, 01B04, 02C05, 02E03, 01D05, 01C04, 01E07, 01G06, 02F06, 01B01, 01D07, 02A08, 01A02, 02G11, 01G04, 02F03, 01C06, 01A01 have at least two fold increase TDCC potency and also show increase affinity compared to a molecule with the parental CDRs, 253BH10.

Molecules 01H08, 01F07, 01H06, 02G02, 02B05, 01C01, 02F02, 02E05, 01E08, 02C01, 02E06, 02B06, 02F04, 01G08, 02C06, 01H09, 01F04, 01D02, 02D11, 01A07, 02C03, 02F07, 01E04, 02H09, 01E03, 02F05, 01B05, 01C05, 02F12, 01H11, 02G06, 01E06, 01G11, 02A05, 01A08, 02G05, 01B09 have at least ten-fold increase TDCC potency and also show increase affinity compared to a molecule with the parental CDRs, 253BH10.

An anti-GFP trispecific molecule, included in these assays as a negative control, had no detectable BCMA binding and no effect on cell viability in the TDCC assay (data not shown).

### Example 2

### Methods to assess binding and cytotoxic activities of exemplary purified trispecific antigen binding proteins containing a BCMA binding domain according to the present disclosure against Jekol, MOLP8 and OPM2 cells

### Protein Production

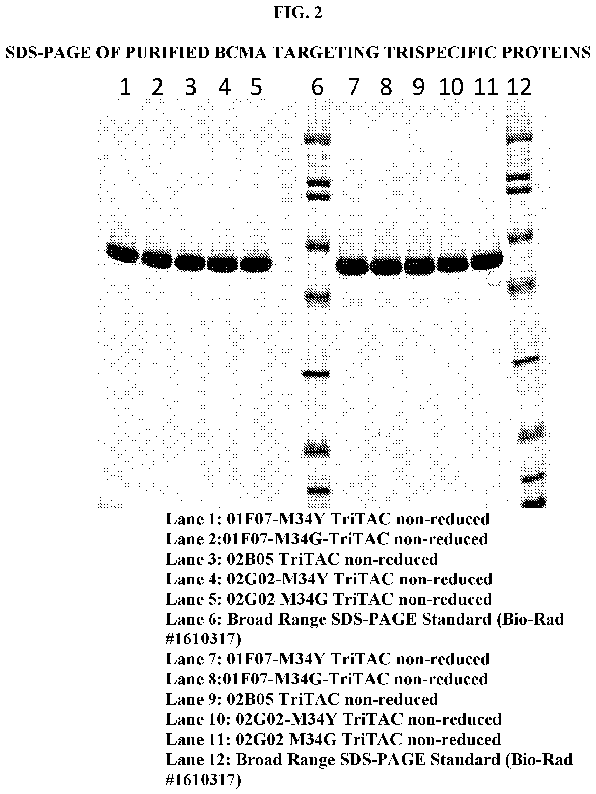

Sequences of BCMA targeting trispecific molecules, containing a BCMA binding protein according to the present disclosure, preceded by a leader sequence and followed by a 6x Histidine Tag (SEQ ID NO: 471), were expressed using the vectors and methods previously described (Running Deer and Allison, 2004. Biotechnol Prog. 20:880-9) except lipid based reagents and non-linearized plasmid DNA were used for cell transfection. Recombinant trispecific proteins were purified using affinity chromatography, ion exchange, and/or size exclusion chromatography. Purified protein was quantitated using theoretical extinction coefficients and absorption spectroscopy. An image of a Coomassie stained SDS-PAGE demonstrates the purity of the proteins **(****Fig. 2****).**

### Cytotoxicity assays

A human T-cell dependent cellular cytotoxicity (TDCC) assay was used to measure the ability of T cell engagers, including trispecific molecules, to direct T cells to kill tumor cells (Nazarian et al., 2015. J. Biomol. Screen., 20:519-27). In this assay, T cells and target cancer cell line cells are mixed together at a 10:1 ratio in a 384-well plate, and varying amounts of the trispecific proteins being tested are added. The tumor cell lines are engineered to express luciferase protein. After 48 hours, to quantitate the remaining viable tumor cells, STEADY-GLO^{®} Luminescent Assay (Promega) was used.

In the instant study, titrations of purified protein were added to TDCC assays (T cell Dependent Cell Cytotoxicity assays) to assess whether the anti-BCMA single domain antibody was capable of forming a synapse between T cells and BCMA-expressing Jeko1, MOLP8 and OPM2 cancer cell lines. Jeko1 is a B cell lymphoma cell line. MOLP-8 is a myeloma cell line. OPM-2 is a human myeloma cell line.

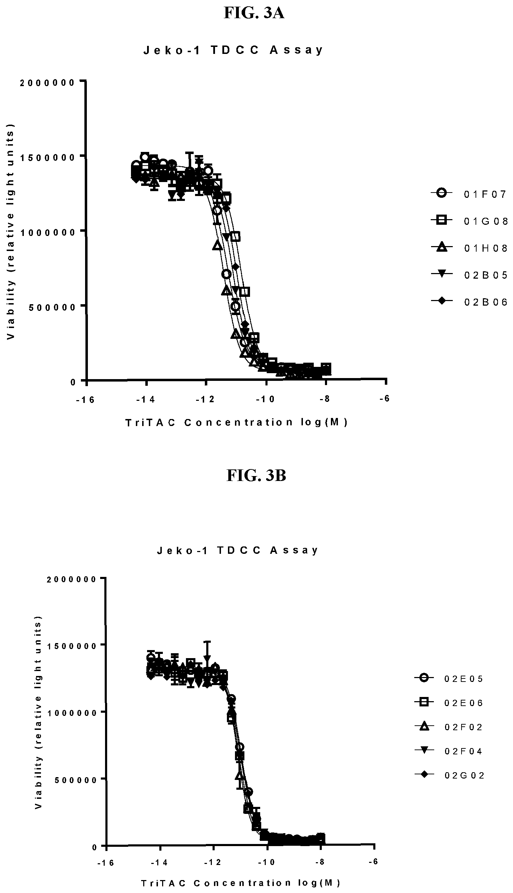

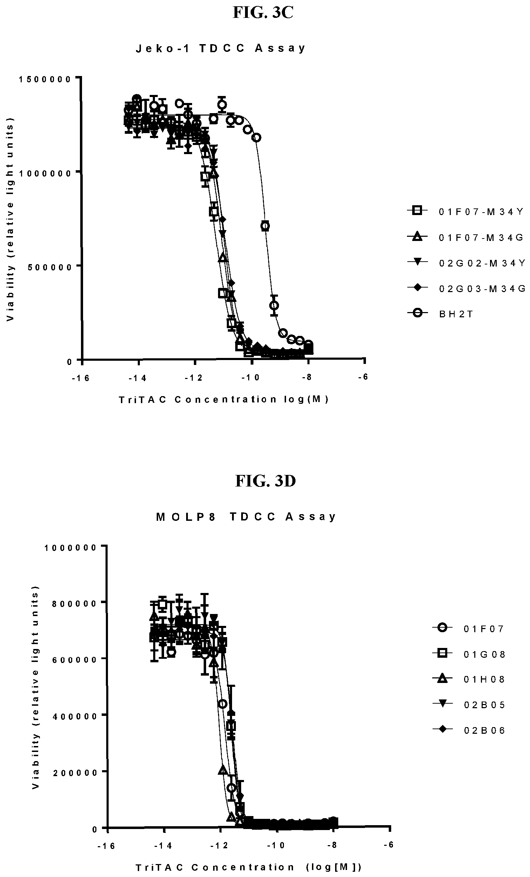

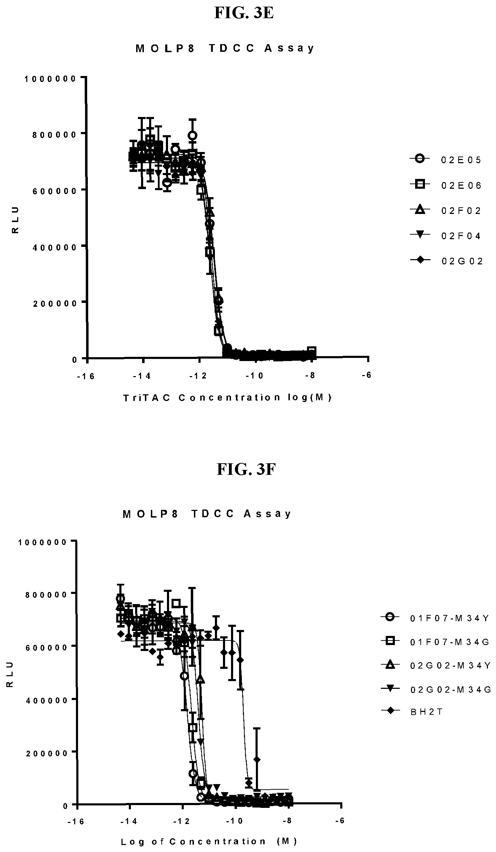

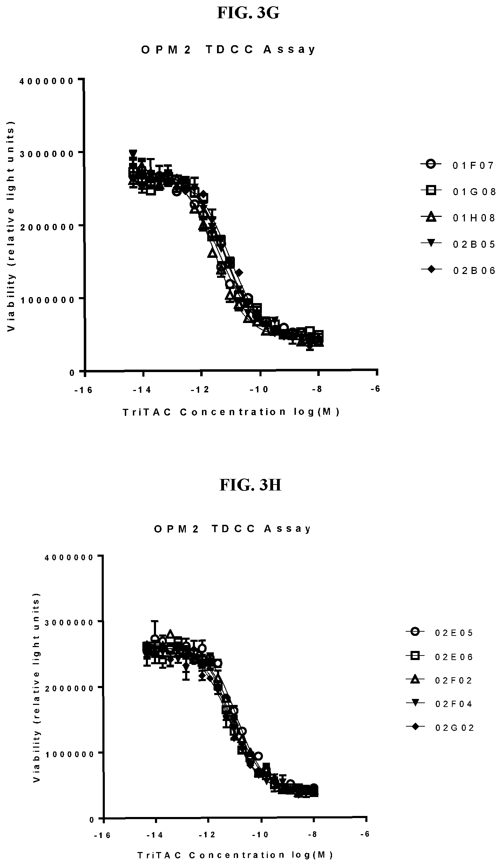

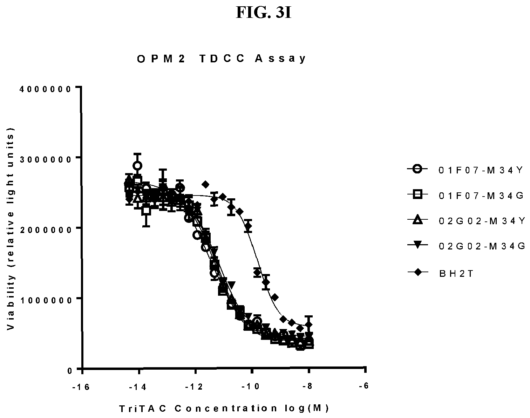

Viability of the cells was measured after 48 hours. It was seen that the trispecific proteins mediated T cell killing. **Fig. 3** shows an example cell viability assay with test proteins compared to a negative control. The EC₅₀ for the TDCC activity of several other test trispecific proteins are listed below in **Table 2**. An anti-GFP trispecific molecule, included in these assays as a negative control, had no effect on cell viability (data not shown).

**Table 2: TDCC EC₅₀ Values for 3 Cell Lines for Select Binder Sequences in TriTAC format**

| **Construct name** | **Jeko1 EC50 (M)** | **MOLP-8 EC50 (M)** | **OPM-2 EC50 (M)** |
|---|---|---|---|
| BH2T TriTAC | 3.2E-10 | 2.0E-10 | 1.6E-10 |
| 01F07 TriTAC | 5.3E-12 | 1.5E-12 | 4.4E-12 |
| 01F07-M34Y TriTAC | 5.6E-12 | 1.5E-12 | 3.6E-12 |
| 01F07-M34G TriTAC | 9.0E-12 | 2.2E-12 | 5.6E-12 |
| 01G08 TriTAC | 1.5E-11 | 2.5E-12 | 6.9E-12 |
| 01H08 TriTAC | 4.0E-12 | 9.4E-13 | 3.1E-12 |
| 02B05 TriTAC | 8.3E-12 | 2.5E-12 | 6.5E-12 |
| 02B06 TriTAC | 1.1E-11 | 2.8E-12 | 9.7E-12 |
| 02E05 TriTAC | 1.1E-11 | 3.3E-12 | 1.2E-11 |
| 02E06 TriTAC | 9.1E-12 | 2.4E-12 | 7.4E-12 |
| 02F02 TriTAC | 8.2E-12 | 3.5E-12 | 1.0E-11 |
| 02F04 TriTAC | 1.0E-11 | 2.5E-12 | 7.3E-12 |
| 02G02 TriTAC | 1.1E-11 | 2.8E-12 | 6.6E-12 |
| 02G02-M34Y TriTAC | 1.1E-11 | 5.6E-12 | 6.2E-12 |
| 02G02-M34G TriTAC | 1.2E-11 | 4.0E-12 | 7.1E-12 |

### Binding affinity

In the instant study, the binding affinity to human BCMA protein of the BCMA targeting trispecific proteins containing a BCMA binding protein according to the present disclosure was determined.

**Table 3: Binding affinity of purified targeting trispecific proteins containing a BCMA binding protein according to the present disclosure.**

| **Construct name** | **Human BCMA KD (M)** |
|---|---|
| 01F07-M34Y TriTAC | 3.0E-09 |
| 01F07-M34G TriTAC | 6.0E-09 |
| 02B05 TriTAC | 6.0E-09 |
| 02G02-M34Y TriTAC | 5.0E-09 |
| 02G02-M34G TriTAC | 7.0E-09 |

### Example 3

### ADCC activity of an exemplar anti-BCMA multidomain antibody of the present disclosure

This study is directed to determining the ability of an exemplary anti-BCMA multidomain antibody of the present disclosure to mediate ADCC as compared to a parental llama anti-BCMA antibody which does not have sequence modifications or substitutions as the exemplary antibody of the disclosure. Both antibodies are expressed as multidomain proteins which include an additional immunoglobulin Fc domain.

### Materials

Donors are leukophoresed, and NK cells are isolated from the leukopack by the Cell Purification Group using the Milteni AUTOMACS^{®} Pro negative selection system. NK cells are held overnight at 4° C on a rocker, then washed, counted and resuspended at 4×10⁶ cells/mL in complete RPMI for use in the ADCC assay.

Targets: Tumor cell targets are selected based on BCMA expression. Targets are washed and counted. 6×10⁶ targets are resuspended in complete RPMI and labeled in a final concentration of 10 µM calcein (Sigma #C1359-00UL calcein am 4 mm in anhydrous DMSO) for 40 minutes at 37 °C, 5% CO₂. Cells are washed twice in PBS, resuspended in complete RPMI and incubated at 37 °C, 5% CO₂ for 2 hrs. After labeling, target cells are washed, recounted and resuspended at 0.2×10⁶ cells/mL in complete RPMI for use in the ADCC assay.

### Methods

The ADCC assay is performed in a 96-well round bottom tissue culture plate (Corning 3799). The test proteins are titrated from 20 µg/mL to 0.0002 µg/mL by carrying 10 µL in 1000 µL of complete RPMI containing 10% FCS (a 1:10 dilution). Calcein labeled targets are added, 50 µL to contain 10,000 cells. Target cells and various concentrations of the multidomain proteins containing either the exemplar anti-BCMA single domain antibody or the comparator antibody are incubated for 40 minutes at 4 °C, then NK cell effectors added, 50 µL to contain 100,000 cells (10:1 E:T ratio). Cultures are incubated for 4 hrs at 37 °C then supernatants pulled and assayed for calcein release by measuring fluorescence at 485-535 nm on a Wallac Victor II 1420 Multilable HTS counter. 100% lysis values are determined by lysing six wells of labeled targets with IGEPAL^{®} 630 detergent (3 µL per well) and spontaneous lysis values determined by measuring the fluorescence in supernatants from targets alone.

### Statistical Analysis

Percent (%) specific lysis is defined as (sample fluorescence) - (spontaneous lysis fluorescence) / (100% lysis-spontaneous lysis fluorescence). Spontaneous lysis is determined by wells containing only targets and 100% lysis is determined by wells where targets are lysed with IGEPAL CA 630 detergent. Raw data is entered in an Excel spreadsheet with embedded formulae to calculate % specific lysis and resultant values transferred to graphic program (GraphPad Prism) where the data is transformed in a curve fit graph. Subsequent analyses (linear regression calculations) are done in GraphPad to generate EC₅₀ values.

### Example 4

### CDC activity of an exemplar anti-BCMA single domain antibody of the present disclosure

To evaluate the anti-tumor activity of exemplar anti-BCMA single domain antibody, according to the present disclosure, against cancer cells, the cytotoxic activity in A431/H9 and NCI-H226 cell models in the presence of human serum as a source of complement is tested. The exemplar anti-BCMA single domain antibody is expressed as a multidomain protein containing a Fc domain.

A multidomain protein containing the exemplar anti-BCMA single domain antibody of the present disclosure exerts potent CDC activity by killing cancer cell lines, and shows no activity on a BCMA-negative cell line.

### Example 5

### Xenograft tumor model

An exemplary BCMA targeting trispecific protein containing an exemplary BCMA binding protein of this disclosure, 02B05 (SEQ ID NO: 383), was evaluated in a xenograft model.

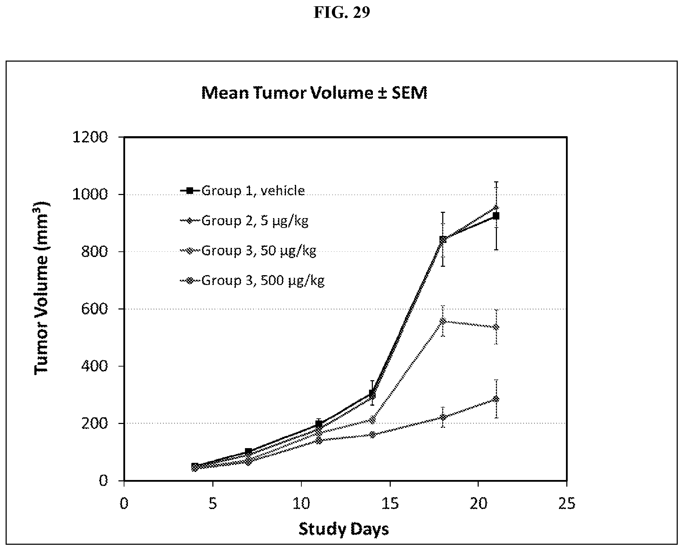

On day 0, NCG mice were subcutaneously inoculated with RPMI-8226 cells, and also intraperitoneally implanted with normal human peripheral blood mononuclear cells (PBMCs). Treatment with the exemplary BCMA targeting trispecific protein was also started on day 0 (qdx10) (once daily for 10 days). The dosage of administration was 5 µg/kg, 50 µg/kg, or 500 µg/kg of the BCMA targeting trispecific protein 02B05, or a vehicle as control. Tumor volumes were determined for 25 days. As shown in **Fig. 29****,** the mean tumor volumes were significantly lower in mice treated with the exemplary BCMA targeting trispecific protein (02B05) (at 50 µg/kg, or 500 µg/kg), as compared to the mice treated with the vehicle or the lower dose of BCMA targeting trispecific protein (02B05) (at 5 µg/kg).

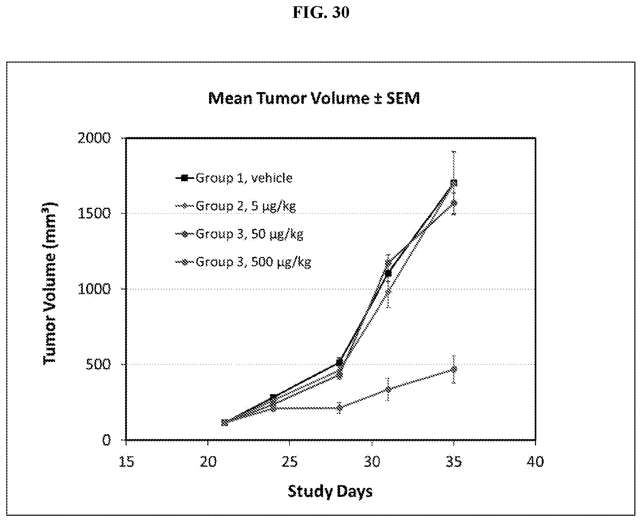

On day 0, NCG mice were subcutaneously inoculated with Jeko 1 cells, and also intraperitoneally implanted with normal human peripheral blood mononuclear cells (PBMCs). Treatment with the exemplary BCMA targeting trispecific protein was started on day 3 (qdx10) (once daily for 10 days). The dosage of administration was 5 µg/kg, 50 µg/kg, or 500 µg/kg of the BCMA targeting trispecific protein 02B05, or a vehicle as control. Tumor volumes were determined for 25 days. As shown in **Fig. 30****,** the mean tumor volumes were significantly lower in mice treated with the exemplary BCMA targeting trispecific protein (02B05) (at 500 µg/kg), as compared to the mice treated with the vehicle or the lower doses of BCMA targeting trispecific protein (02B05) (at 5 µg/kg or 50 µg/kg).

### Example 6

### Affinity Measurements for human and cynomolgus BCMA, CD3ε, and albumin, using an exemplary BCMA targeting trispecific containing a BCMA binding protein of this disclosure protein of this disclosure

The aim of this study was to assess the affinity of an exemplary BCMA targeting trispecific protein containing a BCMA binding protein of this disclosure (02B05) (SEQ ID NO: 383), toward human BCMA, cynomolgus BCMA, human CD3ε, cynomolgus CD3ε, human albumin, cynomolgus albumin, and mouse albumin. The affinities were measured using an Octet instrument. For these measurements, streptavidin tips were first loaded with 2.5 nM human BCMA-Fc, 2.5 nM cynomologus BCMA-Fc, 2.5 nM human CD3ε-Fc, 2.5 nM cynomolgus CD3ε-Fc, 50 nM human serum albumin (HSA), 50 nM cynomolgus serum albumin, or 50 nM mouse serum albumin. Subsequently, the exemplary BCMA targeting trispecific protein containing a BCMA binding protein of this disclosure 02B05 was incubated with the tips, and following an association period, the tips were moved to a buffer solution to allow the exemplary BCMA targeting trispecific protein containing a BCMA binding protein of this disclosure (02B05) to disassociate. The affinities for binding to human and cynomolgus BCMA and CD3ε were measured in the presence of 15 mg/ml human serum albumin. Average calculated K_{D} values from these studies are provided in **Table 4** (n indicates the number of independent measurements, n/d indicates no binding detected under the conditions tested). Binding was detected to human BCMA, human CD3ε, cynomolgus CD3ε, human serum albumin, cynomolgus serum albumin, and mouse serum albumin. Under the conditions tested, no binding was detected to cynomolgus BCMA.

**Table 4. Measured K_{D} values for exemplary BCMA targeting trispecific protein containing a BCMA binding protein of this disclosure, 02B05, to protein ligands.**

| **Protein ligand** | **Species** | **K_{D} (nM)** | **n** |
|---|---|---|---|
| **BCMA** | human | 2.4 ± 0.2 | 2 |
| | cynomolgus | n/d | 2 |
| CD3ε | human | 8 ± 1 | 2 |
| | cynomolgus | 7.8 ± 0.4 | 2 |
| Albumin | human | 6 ± 1 | 3 |
| | cynomolgus | 7.5 | 1 |
| | mouse | 76 | 1 |

### Example 7

### Human T cell binding ability of an exemplary BCMA targeting trispecific protein containing a BCMA binding protein of this disclosure of this disclosure

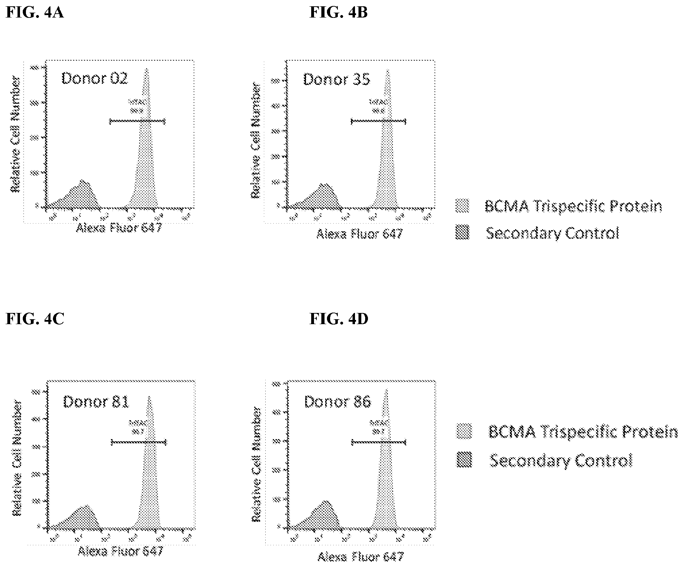

Exemplary BCMA targeting trispecific protein containing a BCMA binding protein of this disclosure, 02B05 (SEQ ID NO: 383) was tested for its ability to bind to purified T cells. Briefly, the BCMA trispecific protein or phosphate buffered saline (PBS) were incubated with purified T cells from 4 different anonymous human donors. After washing unbound protein, the T cells were then incubated with an Alexa Fluor 647 conjugated antibody that recognizes the anti-albumin domain in the 02B05 BCMA trispecific antigen-binding protein. The T cells were then analyzed by flow cytometry. It was observed that human T cells incubated with the 02B05 BCMA trispecific antigen-binding protein had notable shifts associated with Alexa Fluor 647 staining compared to cells that were incubated with PBS. The results are shown in Figs. **4A, 4B, 4C,** and **4D****.** In conclusion, this study indicated that the exemplary BCMA targeting trispecific protein containing a BCMA binding protein of this disclosure was able to bind human T cells.

### Example 8

### Ability of an exemplary BCMA targeting trispecific protein of this disclosure to bind BCMA expressing cells

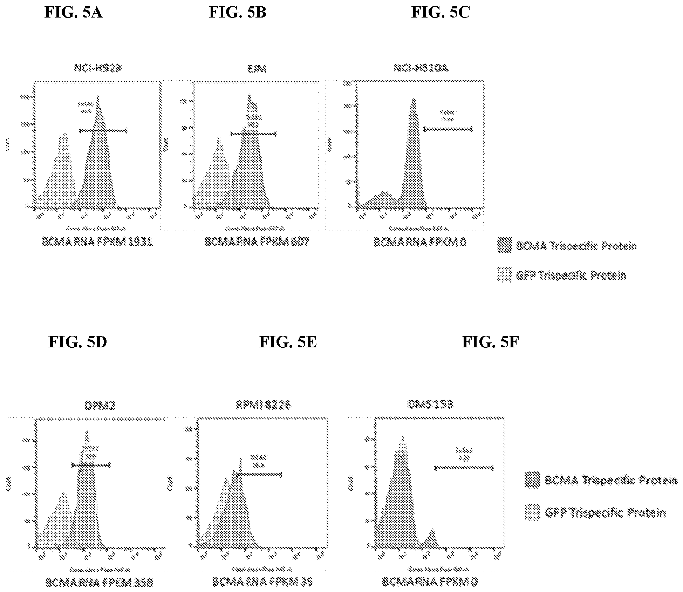

Exemplary BCMA targeting trispecific protein containing a BCMA binding protein of this disclosure 02B05 (SEQ ID NO: 383) was tested for its ability to bind to BCMA expressing cells. Briefly, the 02B05 BCMA trispecific antigen-binding protein was incubated with cell lines expressing BCMA (NCI-H929; EJM; RPMI-8226; OPM2) or lacking BCMA (NCI-H510A; DMS-153). Expression of BCMA RNA in these cells is indicated by the FPKM (fragments per kilobase million) values listed in **Figs. 5A****-F:** the RNA FPKM values are from the Cancer Cell Line Encyclopedia (Broad Institute, Cambridge, MA USA). After washing unbound protein, the cells were then incubated with an Alexa Fluor 647 conjugated antibody that recognizes the anti-albumin domain in the 02B05 BCMA trispecific antigen-binding protein. The cells were then analyzed by flow cytometry. As a negative control, cells were incubated with a trispecific protein targeting GFP. Cells expressing BCMA RNA and incubated with the BCMA trispecific protein had notable shifts associated with Alexa Fluor 647 staining compared to cells that were incubated with GFP trispecific protein (as in **Figs. 5A, 5B, 5D, and 5E****).** Whereas, cells lacking BCMA RNA produced equivalent Alexa Fluor 647 staining with the BCMA trispecific protein and the GFP trispecific protein (as seen in **Figs. 5C,** and **5F****).** Thus, this study indicated that the exemplary BCMA trispecific antigen-binding was able to selectively bind to cells expressing BCMA.

### Example 9

### Ability of an exemplary BCMA targeting trispecific protein containing a BCMA binding protein of this disclosure to mediate T cell killing of cancer cells expressing BCMA

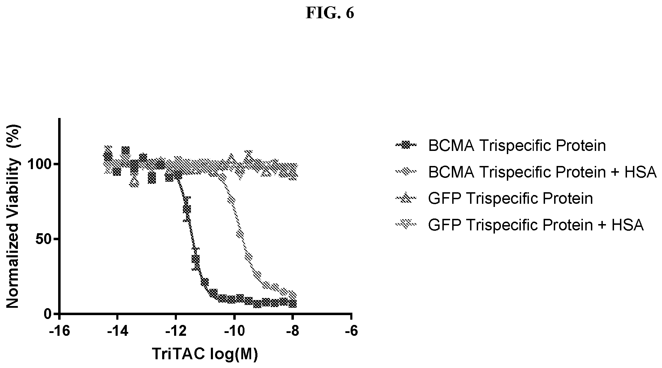

Exemplary BCMA trispecific protein containing a BCMA binding protein of this disclosure 02B05 (SEQ ID NO: 383) was tested for its ability to direct T cells to kill BCMA expressing cells in the presence and absence of human serum albumin (HSA) using a standard TDCC assay as described in **Example 1.** Because the exemplary BCMA trispecific protein contains an anti-albumin domain, this experiment was performed to confirm that binding to albumin would not prevent the BCMA trispecific antigen-binding protein from directing T cells to kill BCMA expressing cells. Five BCMA expressing cell lines were tested: EJM, Jeko, OPM2, MOLP8, and NCI-H929. Representative data for an experiment with the EJM cells are shown in **Fig. 6****.** It was observed that viability of the EJM cells decreased with increasing amount of the exemplary 02B05 BCMA trispecific antigen-binding protein in the presence or absence of human serum albumin (HSA), whereas a control GFP targeting trispecific protein did not affect cell viability. In the presence of albumin, higher concentrations of BCMA trispecific protein were needed to reduce viability of the EJM cells. The EC₅₀ values for cell killing by BCMA trispecific protein for the EJM cells as well as the Jeko, OPM2, MOL8, and NCI-H929 cells in the absence or presence of HSA are provided in **Table 5.** With all five cell lines, the exemplary 02B05 BCMA trispecific antigen-binding protein directed T cells to kill target cells in the presence of HSA.

**Table 5 TDCC EC₅₀ Values for an exemplary BCMA targeting trispecific protein containing a BCMA binding protein of this disclosure in the presence or absence of human serum albumin with five different BCMA expressing cell lines**

| **Cell Line** | **EC₅₀ without HSA (pM)** | **EC₅₀ with HSA (pM)** |
|---|---|---|
| **EJM** | 1.0 | 53 |
| **Jeko** | 8.3 | 662 |
| **OPM2** | 6.5 | 328 |
| **MOLP8** | 2.5 | 388 |
| **NCI-H929** | 6.7 | 194 |

### Example 10

### Ability of an exemplary BCMA targeting trispecific protein containing a BCMA binding protein of this disclosure to mediate T cell killing of cancer cells expressing BCMA, using a smaller target cell to effector cell ratio

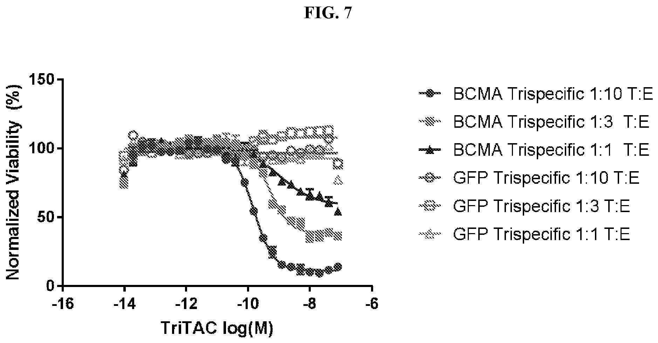

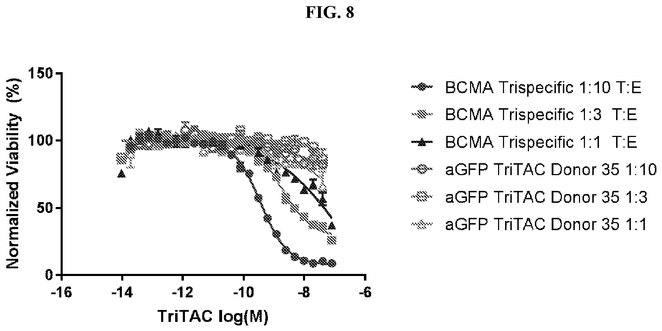

In the standard TDCC assay (as described in **Example 1),** a ratio 1 target cell (EJM cells or OPM2 cells) per 10 effector cells (T cells) is used in a 48 hour assay. In this experiment, the ability of exemplary BCMA targeting trispecific protein containing an exemplary BCMA binding protein of this disclosure 02B05 (SEQ ID NO: 383) to direct T cells to kill target cells with smaller target cell to effector ratios was tested. The expectation was that less killing would be observed when fewer effector cells were used. Two BCMA expressing cell lines were tested, EJM and OPM2, using target to effector cell ratios of 1:1, 1:3, and 1:10, and the experiment was performed in the presence of 15 mg/ml HSA. A GFP targeting trispecific protein was used as a negative control. Data from this experiment is shown in **Fig. 7** (TDCC assay with EJM cells) and **Fig. 8** (TDCC assay with OPM2 cells). As expected, near complete killing of the target cells was observed with a 1:10 target to effector cell ratio. The amount of killing was reduced with decreasing effector cells. The EC₅₀ values for cell killing with each ratio are listed in **Table 6** (n/d indicates insufficient killing was observed to calculate an EC₅₀ value). The EC₅₀ values increased when fewer effector cells were present. Thus, as expected, reducing the number of effector cells to target cells reduced TDCC activity of the BCMA trispecific protein.

**Table 6 TDCC EC₅₀ values for an exemplary BCMA targeting trispecific protein containing a BCMA binding protein of this disclosure (02B05) with varied target cell (EJM cells) to effector cell (T cells) ratios (tested in presence of 15 mg/ml HSA)**

| **Target cell: T Cell ratio** | **EJM EC₅₀ (pM)** | **OPM2 EC₅₀ (pM)** |
|---|---|---|
| **1:10** | 154 | 371 |
| **1:3** | 523 | 1896 |
| **1:1** | 1147 | n/d |

### Example 11

### Ability of an exemplary BCMA targeting trispecific protein containing a BCMA binding protein of this disclosure to mediate T cell killing of cancer cells expressing BCMA, in a time course study, using a smaller target cell to effector cell ratio

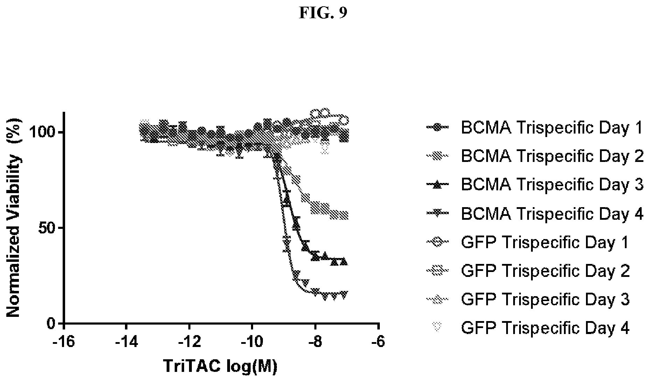

In the standard TDCC assay **(Example 1),** a ratio 1 target cell per 10 effector cells (T cells) is used in a 48 hour assay. In this experiment, a time course was performed using a 1 to 1 ratio of target cells (EJM cells) to effector cells (T cells). The expectation was that with increased time, a 1 to 1 ratio would result in target cell killing. The TDCC assay was performed using EJM and a 1:1 target to effector cell ratio, and the experiment was performed in the presence of 15 mg/ml HSA. A GFP targeting trispecific protein was used as a negative control. Target cell viability was measured on days 1, 2, 3, and 4 following incubation of the target cells and effector cells, at a 1:1 ratio, in presence of the exemplary 02B05 BCMA trispecific antigen-binding protein and 15 mg/ml HSA, or the GFP targeting trispecific protein and 15 mg/ml HSA. While no target cell killing was observed on day 1, killing was observed at all other time points with the 02B05 BCMA trispecific-antigen-binding protein, with the amount of killing increasing with time **(****Fig. 9****).** No target cell killing was observed with the GFP targeting trispecific protein. The EC₅₀ values calculated for cell killing on each day are provided in **Table 7** (n/d indicates insufficient killing to determine an EC₅₀ value). From this study it was concluded that the exemplary 02B05 BCMA trispecific protein was able to direct T cell killing with lower numbers of effector cells, but more time was needed to achieve more complete killing.

**Table 7 TDCC EC₅₀ values for an exemplary BCMA targeting trispecific protein containing a BCMA binding protein of this disclosure (02B05) with a 1 to 1 target cell (EJM cells) to effector cell (T cells) ratios (tested in presence of 15 mg/ml HSA), at varied time points**

| | **EC₅₀ (pM)** |
|---|---|
| Day 1 | n/d |
| Day 2 | 1859 |
| Day 3 | 1420 |
| Day 4 | 1012 |

### Example 12

### Ability of an exemplary BCMA targeting trispecific protein containing a BCMA binding protein of this disclosure to direct human T cells to kill BCMA expressing cells

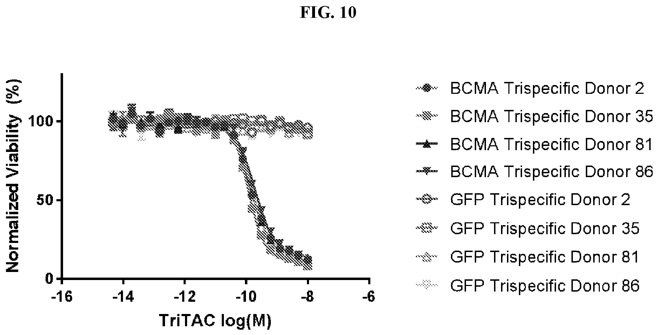

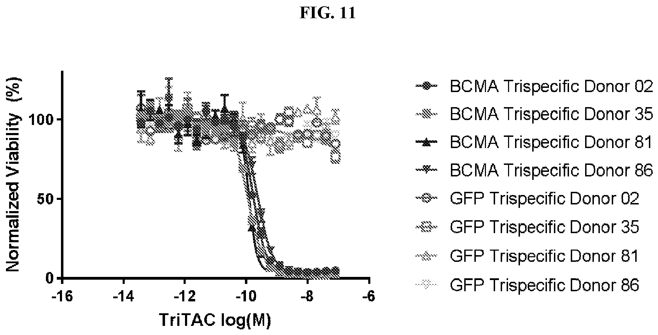

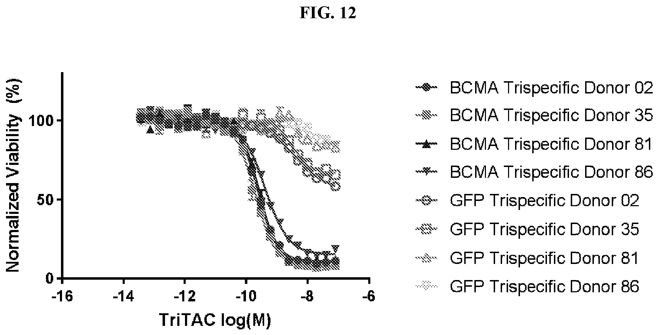

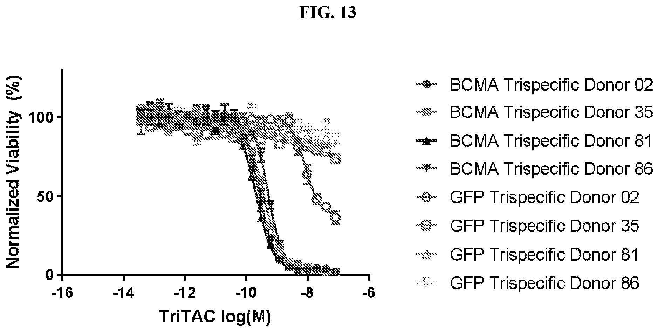

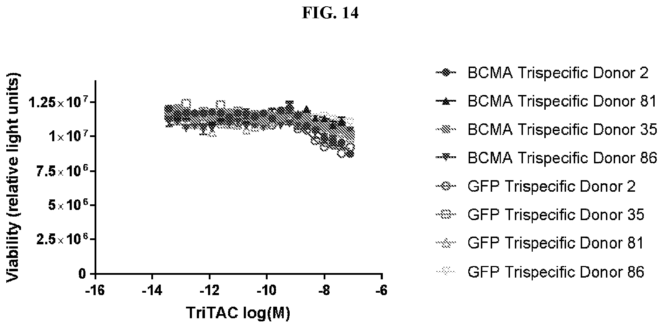

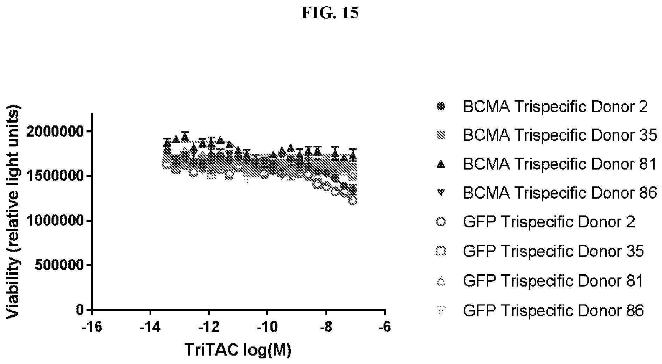

Exemplary BCMA trispecific protein containing a BCMA binding protein of this disclosure 02B05 (SEQ ID NO: 383) was tested for its ability to direct T cells from four different anonymous human donors to kill four different BCMA expressing cells in the presence of 15 mg/ml human serum albumin (HSA) using a standard TDCC assay as described in **Example 1.** The BCMA expressing cell lines were EJM, NCI-H929, OPM2, and RPMI8226. As negative controls, two cell lines that lack BCMA expression, OVCAR8 and NCI-H510A, were also tested in the TDCC assays. A control GFP targeting trispecific protein was also used as a negative control. With the four BCMA expressing cell lines and all four T cell donors, cell viability decreased with increasing amounts of the BCMA trispecific protein but not with the GFP trispecific protein **(****Figs. 10****,** **11****,** **12****, and** **13****).** The EC₅₀ values for cell killing are provided in **Table 8.** The exemplary 02B05 BCMA trispecific antigen-binding protein did not direct killing of the cell lines lacking BCMA expression **(****Figs. 14** **and** **15****).** Thus, it was inferred that the exemplary 02B05 BCMA trispecific antigen-binding protein was able to direct T cells from multiple donors to kill a spectrum of BCMA expressing cell lines.

**Table 8 Exemplary BCMA trispecific protein EC₅₀ values from TDCC assays with four BCMA expressing cell lines and four T cell donors in presence of 15 mg/ml HSA**

| | **EC50 (pM)** | | | |
|---|---|---|---|---|
| | **H929** | **OPM2** | **RPMI8226** | **EJM** |
| **Donor 02** | 169 | 250 | 275 | 151 |
| **Donor 35** | 113 | 199 | 371 | 121 |
| **Donor 81** | 124 | 265 | 211 | 143 |
| **Donor 86** | 239 | 416 | 543 | 191 |

### Example 13

### Ability of an exemplary BCMA targeting trispecific protein containing a BCMA binding protein of this disclosure to direct cynomolgus T cells to kill BCMA expressing cells

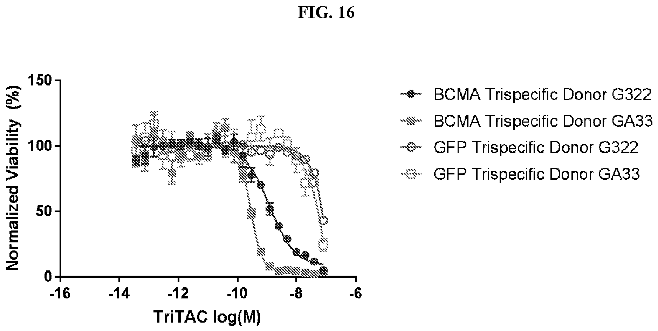

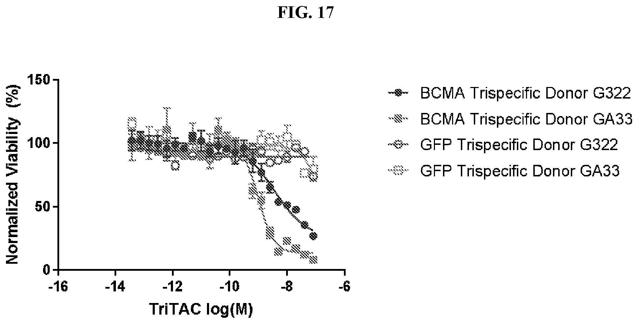

Exemplary BCMA targeting trispecific protein containing a BCMA binding protein of this disclosure 02B05 (SEQ ID NO: 383) was tested for its ability to direct T cells from cynomolgus monkeys to kill BCMA expressing cells in the presence of 15 mg/ml human serum albumin (HSA). The experimental conditions were the same as described in Example 1 except peripheral blood mononuclear cells (PBMC) from cynomolgus monkeys were used as a source of T cells. Two BCMA expressing cell lines were tested, RPMI8226 and NCI-H929. As shown in **Figs. 16** and **17****,** the BCMA trispecific protein was able to direct T cells present in the cynomolgus PBMCs to kill the two BCMA expressing cell lines. The EC₅₀ values for the cell killing are listed in **Table 9.** A GFP trispecific protein did not affect viability of the BCMA expressing cells. Thus, the BCMA expressing trispecific protein, which can bind cynomolgus CD3ε (as shown in **Example 6),** can direct cynomolgus T cells to kill cells expressing human BCMA.

**Table 9 BCMA trispecific protein EC₅₀ values from TDCC Assays with two cell lines and two cynomolgus PMBC donors in the presence of 15 mg/ml HSA**

| | **EC₅₀ (pM)** | |
|---|---|---|
| | **RPMI8226** | **NCI-H929** |
| **Donor G322** | 3654 | 1258 |
| **Donor GA33** | 1003 | 288 |

### Example 14

### Exemplary BCMA targeting trispecific protein containing a BCMA binding protein of this disclosure in mediating induction of T cell activation

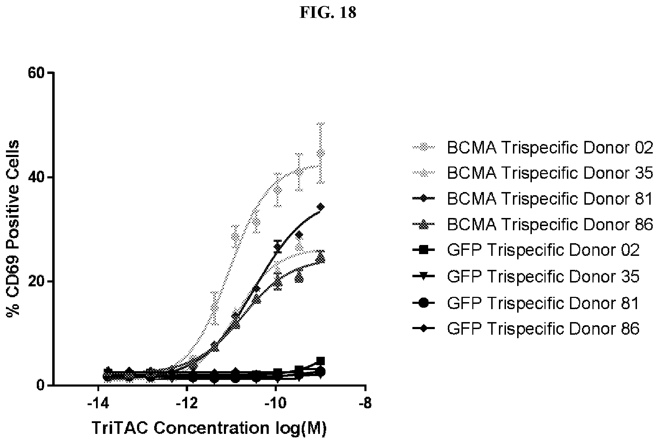

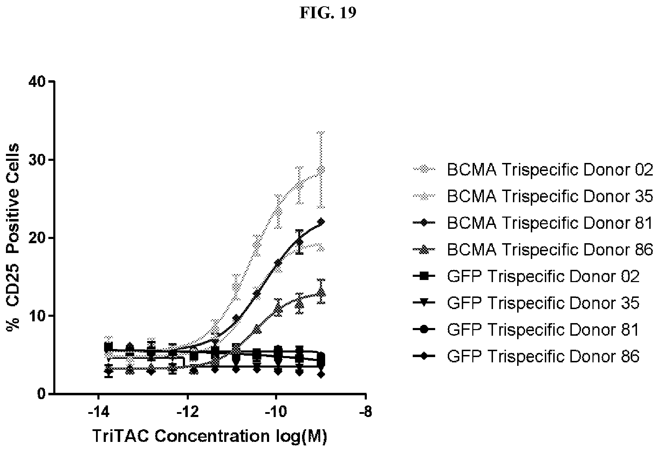

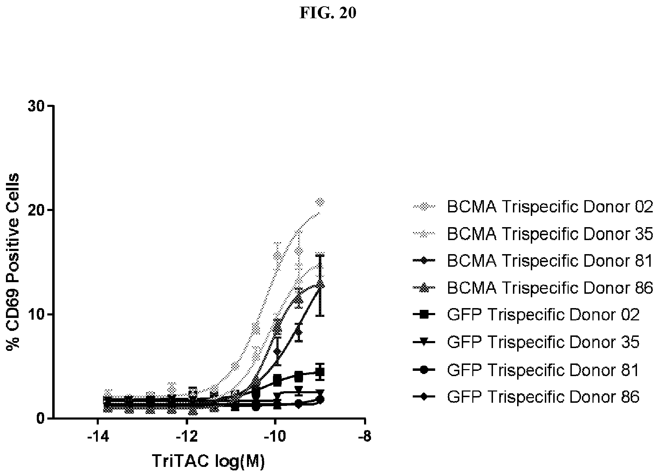

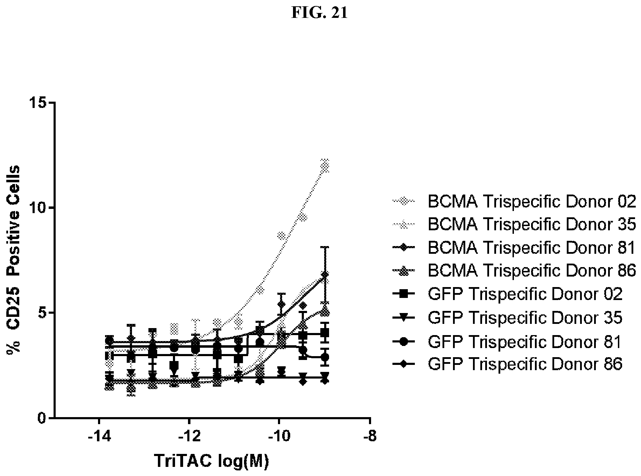

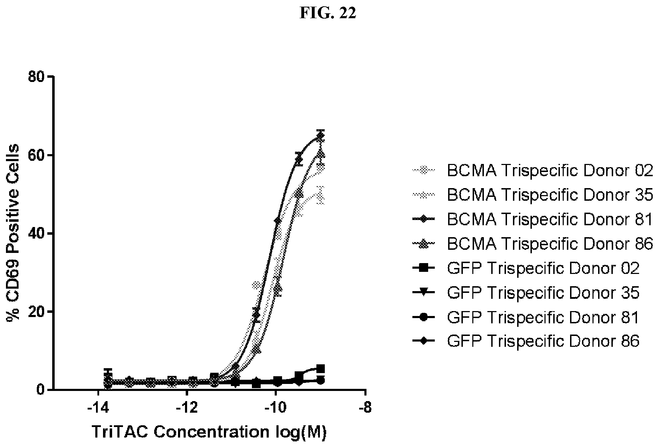

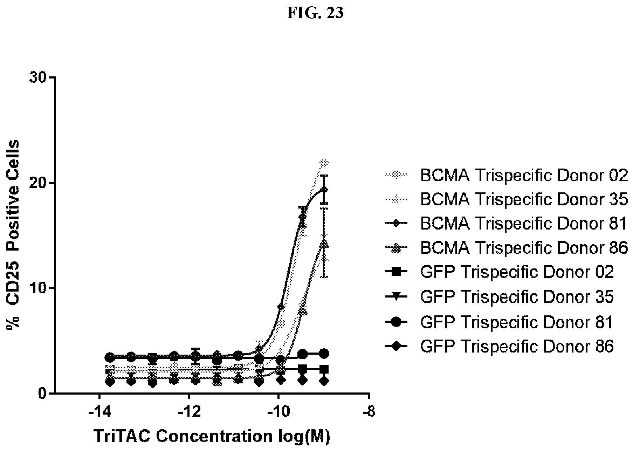

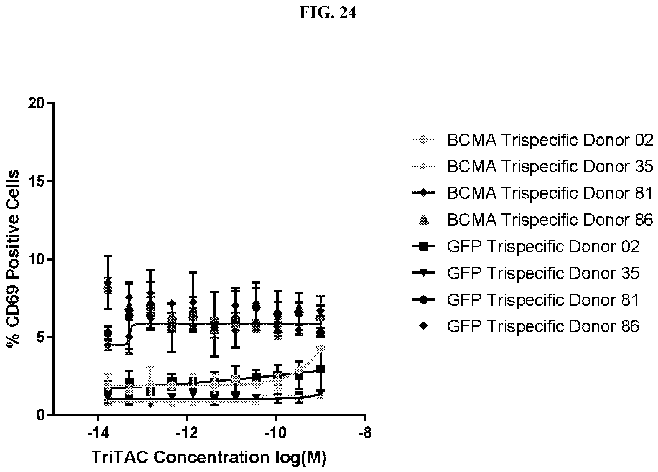

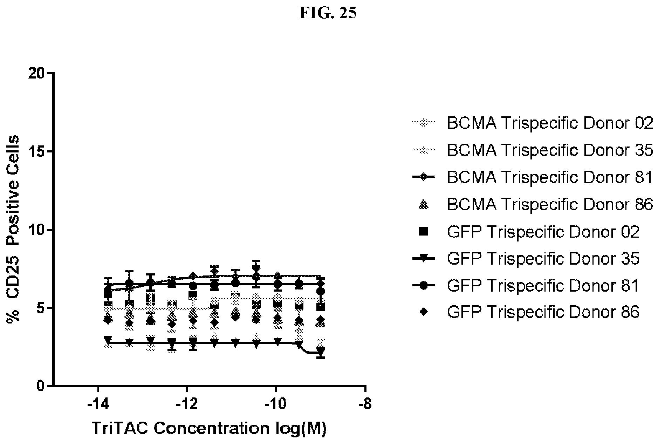

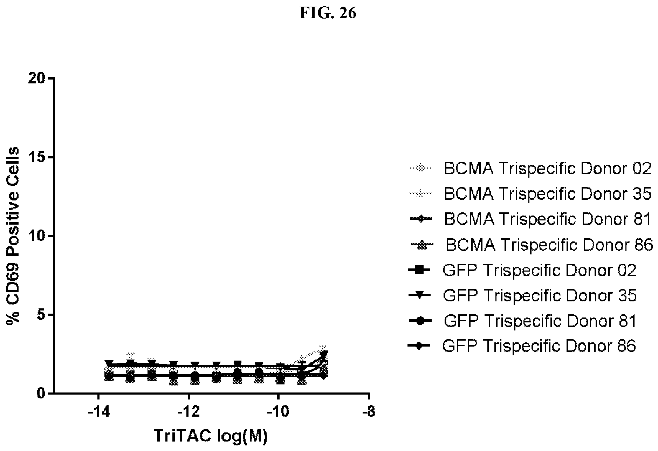

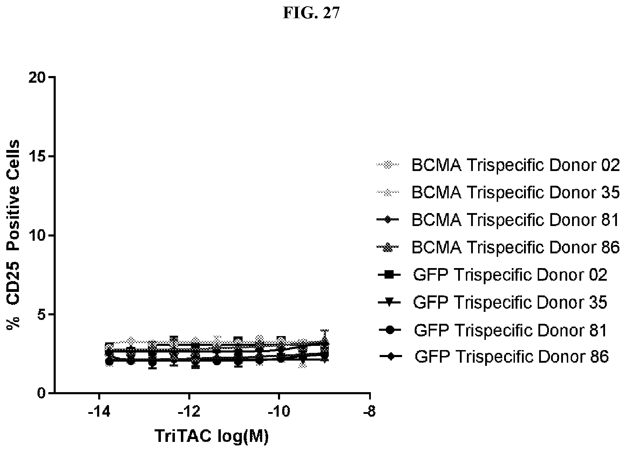

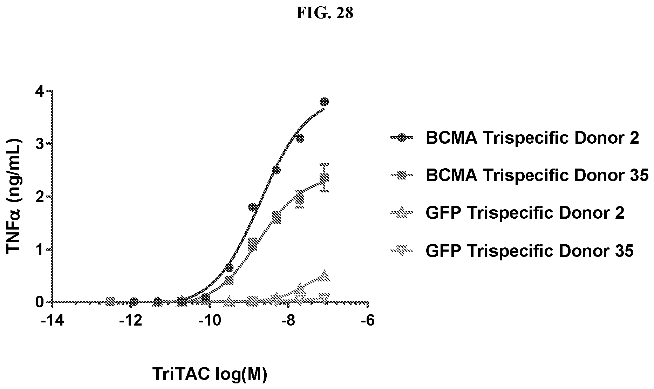

Exemplary BCMA targeting trispecific protein containing a BCMA binding protein of this disclosure 02B05 (SEQ ID NO: 383) was tested for its ability to activate T cells in the presence of BCMA expressing cells. The BCMA expressing cell lines were EJM, OPM2, and RPMI8226. As negative controls, two cells lines that lack BCMA expression were also included, OVCAR8 and NCI-H510A. T cells were obtained from four different anonymous human donors. The assays were set up using the conditions of a standard TDCC assay as described in **Example 1** except the assay was adapted to 96-well format and the assay was carried out in the presence of 15 mg/ml HSA. After the 48 hour assay, T cell activation was assessed by using flow cytometry to measure expression of T cell activation biomarkers CD25 and CD69 on the surface of the T cells. With increasing concentrations of the exemplary 02B05 BCMA trispecific antigen-binding protein increased expression of CD69 and CD25 was observed on T cells when co-cultured with the BCMA expressing cells (as shown in **Figs. 18-23****).** Thus, the observed interaction was dependent on interaction of the BCMA binding sequence within the exemplary BCMA targeting trispecific protein, as little to no activation was observed with a control GFP trispecific protein (as shown in **Figs. 18-23****)** or with target cells with no BCMA expression (as shown in **Figs. 24-27****).** Therefore the exemplary BCMA targeting trispecific protein activated T cells in co-cultures containing BCMA expressing cells. This conclusion is bolstered by additional data. For instance, expression of a cytokine, TNFα, was measured in the medium collected from a co-culture of T cells and BCMA expressing target cells treated with increasing concentrations of the exemplary BCMA targeting trispecific protein or with the negative control GFP trispecific protein. The co-cultures were set up using the conditions of a standard TDCC assay (as described in Example 1) supplemented with 15 mg/ml HSA. TNFα was measured using an electrochemiluminescent assay (Meso Scale Discovery). Robust induction of TNFα expression was observed with the exemplary BCMA targeting trispecific protein containing a BCMA binding protein of this disclosure 02B05 (SEQ ID NO: 383) and not the GFP trispecific protein **(****Fig. 28****).** This result further supports that the exemplary BCMA targeting trispecific protein activated T cells in co-cultures containing BCMA expressing cells.

### Example 15

### Pharmacokinetics of an exemplary BCMA targeting trispecific protein containing a BCMA binding protein of this disclosure

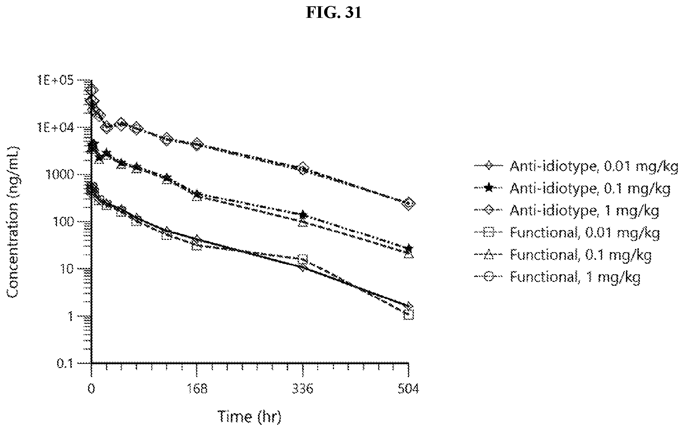

Cynomolgus monkeys were administered single intravenous doses of an exemplary BCMA targeting trispecific protein containing a BCMA binding protein of this disclosure (02B05) (SEQ ID NO: 383), at 0.01 mg/kg, 0.1 mg/kg, or 1 mg/kg. Two animals were included per dose group. Following the administration, serum samples were collected and analyzed by two different electrochemiluminescent assays. One assay used biotinylated CD3ε as a capture reagent and detected with sulfo tagged BCMA (termed the functional assay). Another assay used as a capture reagent a biotinylated antibody recognizing the anti-albumin domain in the exemplary BCMA targeting trispecific protein containing a BCMA binding protein of this disclosure 02B05 (SEQ ID NO: 383) and used as a detection reagent a sulfo tagged antibody recognizing the anti-CD3 binding domain in the exemplary BCMA targeting trispecific protein containing a BCMA binding protein of this disclosure (*i.e.,* an anti-idiotype antibody). The results from the electrochemiluminescent assays are plotted in **Fig. 31****.** As seen in **Fig. 31****,** the exemplary BCMA targeting trispecific protein was detected in the cynomolgus serum samples, even after 504 hours after the administration. The exemplary BCMA targeting trispecific protein was identified using both the sulfo-tagged BCMA (lines labeled using the term "functional" in **Fig. 31****)** and by the anti-idiotype antibody (lines labeled using the term "anti-idiotype" in **Fig. 31****).**

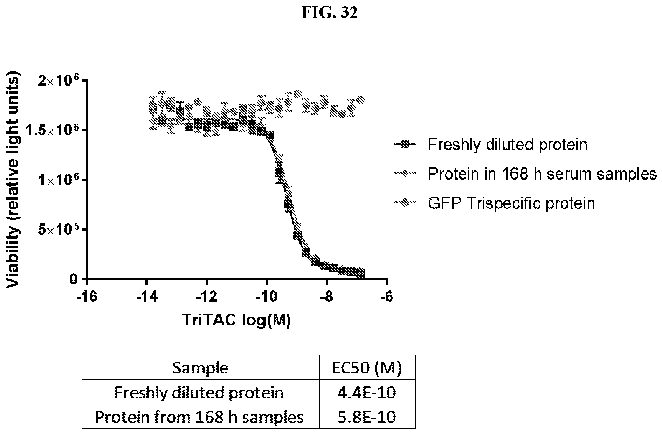

To confirm that the exemplary BCMA targeting trispecific protein retained the ability to direct T cells to kill BCMA expressing EJM cells, after in vivo administration, serum samples from the 168 hour time point were tested in a TDCC assay (as described in Example 1) in the presence of 16.7% serum from a cynomolgus monkey that has not been exposed to a BCMA targeting trispecific protein, titrating the exemplary BCMA targeting trispecific protein using the protein concentrations determined using the electrochemiluminescent assays (shown in Fig. 32). Fresh diluted exemplary BCMA targeting trispecific protein containing a BCMA binding protein of this disclosure 02B05 (SEQ ID NO: 383) was compared to the BCMA trispecific protein collected from the test cynomolgus monkeys at 168 h. A GFP trispecific protein was included as a negative control. This study demonstrated that the exemplary BCMA targeting trispecific protein collected from the test cynomolgus monkeys' serum had identical activity as freshly diluted protein, and that the protein in the serum samples retained the ability to direct T cells to kill BCMA expressing EJM cells.

### Sequence Table

| **SEQ ID NO:** | | **Description** | **Sequence** |
|---|---|---|---|
| | 1. | **Exemplary CDR1** | X₁X₂X₃X₄X₅X₆X₇PX₈G where X₁ is T or S; X₂ is N, D, or S; X₃ is I, D, Q, H, V, or E; X₄ is F, S, E, A, T, M, V, I, D, Q, P, R, or G; X₅ is S, M, R, or N; X₆ is I, K, S, T, R, E, D, N, V, H, L, A, Q, or G; X₇ is S, T, Y, R, or N; and X₈ is M, G, or Y |
| | 2. | **Exemplary CDR2** | AIX₉GX₁₀X₁₁TX₁₂YADSVK where X₉ is H, N, or S; X₁₀ is F, G, K, R, P, D, Q, H, E, N, T, S, A, I, L, or V; X₁₁ is S, Q, E, T, K, or D; and X₁₂ is L, V, I, F, Y, or W |
| | 3. | **Exemplary CDR3** | VPWGX₁₃YHPX₁₄X₁₅VX₁₆ where X₁₃ is D, I, T, K, R, A, E, S, or Y; X₁₄ is R, G, L, K, T, Q, S, or N; X₁₅ is N, K, E, V, R, M, or D; and X₁₆ is Y, A, V, K, H, L, M, T, R, Q, C, S, or N |

| **SEQ ID NO:** | | **Name** | **HCDR1** |
|---|---|---|---|
| | 4. | 01A01 | TDIFSISPMG |
| | 5. | 01A02 | TNIFSSSPMG |
| | 6. | 01A03 | TNIFSISPGG |
| | 7. | 01A04 | TNIFMISPMG |
| | 8. | 01A05 | TNIFSSSPMG |
| | 9. | 01A06 | TNIFSIRPMG |
| | 10. | 01A07 | TNISSISPMG |
| | 11. | 01A08 | TNIFSSSPMG |
| | 12. | 01A09 | TNIFSITPMG |
| | 13. | 01B01 | TNIPSISPMG |
| | 14. | 01B02 | TNITSISPMG |
| | 15. | 01B03 | TNIFSKSPMG |
| | 16. | 01B04 | TNDFSISPMG |
| | 17. | 01B05 | TNITSISPMG |
| | 18. | 01B06 | TNIFSISPMG |
| | 19. | 01B07 | TNIFSRSPMG |
| | 20. | 01B08 | TNIESISPMG |
| | 21. | 01B09 | SNIFSISPMG |
| | 22. | 01B12 | TNIFSTSPMG |
| | 23. | 01C01 | TNIVSISPMG |
| | 24. | 01C02 | TNIESISPMG |
| | 25. | 01C04 | TNIPSISPMG |
| | 26. | 01C05 | TNIFSSSPMG |
| | 27. | 01C06 | TNIFSISPMG |
| | 28. | 01C07 | TNIFSIYPMG |
| | 29. | 01C08 | TNIFSNSPMG |
| | 30. | 01C10 | TNISSISPMG |
| | 31. | 01D02 | TNIVSISPMG |
| | 32. | 01D03 | TNIFSNSPMG |
| | 33. | 01D04 | TNITSISPMG |
| | 34. | 01D05 | TNIFSDSPMG |
| | 35. | 01D06 | TNIFSRSPMG |
| | 36. | 01D07 | TNIFSASPMG |
| | 37. | 01D10 | TNIFSASPMG |
| | 38. | 01E03 | TNITSISPMG |
| | 39. | 01E04 | TNIASISPMG |
| | 40. | 01E05 | TNIFSRSPMG |
| | 41. | 01E06 | TNIFSLSPMG |
| | 42. | 01E07 | TNIPSISPMG |
| | 43. | 01E08 | TNIFSQSPMG |
| | 44. | 01E10 | TNIESISPMG |
| | 45. | 01F02 | TNIFSHSPMG |
| | 46. | 01F03 | TNIFSESPMG |
| | 47. | 01F04 | TNIDSISPMG |
| | 48. | 01F05 | TNIFSSSPMG |
| | 49. | 01F07 | TNIFSTSPMG |
| | 50. | 01F08 | TNITSVSPMG |
| | 51. | 01F09 | TNISSISPMG |
| | 52. | 01F10 | SNIFSISPMG |
| | 53. | 01F12 | TNIFRISPMG |
| | 54. | 01G01 | TNIVSISPMG |
| | 55. | 01G04 | TNIDSISPMG |
| | 56. | 01G06 | TNIFSRSPMG |
| | 57. | 01G08 | TNIQSISPMG |
| | 58. | 01G09 | TNIFNISPMG |
| | 59. | 01G10 | TNEFSISPMG |
| | 60. | 01G11 | TNIPSISPMG |
| | 61. | 01H01 | TNIGSISPMG |
| | 62. | 01H04 | TNIFSKSPMG |
| | 63. | 01H05 | TNIFSITPMG |
| | 64. | 01H06 | TSDFSISPMG |
| | 65. | 01H08 | TNIMSISPMG |
| | 66. | 01H09 | TNIMSISPMG |
| | 67. | 01H10 | TNIPSISPMG |
| | 68. | 01H11 | TNIFSTSPMG |
| | 69. | 02A04 | TNIFSQSPMG |
| | 70. | 02A05 | TNIASISPMG |
| | 71. | 02A07 | TNIFSKSPMG |
| | 72. | 02A08 | TNIFSRSPMG |
| | 73. | 02A11 | TNHFSISPMG |
| | 74. | 02B01 | TNIFSNSPMG |
| | 75. | 02B04 | TNIFSTSPMG |
| | 76. | 02B05 | TNIFSISPYG |
| | 77. | 02B06 | TNIFSNSPMG |
| | 78. | 02B07 | TNIFSSSPMG |
| | 79. | 02B11 | TNIVSISPMG |
| | 80. | 02B12 | TNISSISPMG |
| | 81. | 02C01 | TNIISISPMG |
| | 82. | 02C03 | TNIASISPMG |
| | 83. | 02C05 | TNIFSESPMG |
| | 84. | 02C06 | TNIFSTSPMG |
| | 85. | 02D06 | TNISSISPMG |
| | 86. | 02D09 | TNVVSISPMG |
| | 87. | 02D11 | TNEFSISPMG |
| | 88. | 02E03 | TNIFSNSPMG |
| | 89. | 02E05 | TNIFSRSPMG |
| | 90. | 02E06 | TNIFSDSPMG |
| | 91. | 02E09 | TNDFSISPMG |
| | 92. | 02F02 | TNIFSKSPMG |
| | 93. | 02F03 | TNIFSIYPMG |
| | 94. | 02F04 | TNIFSSSPMG |
| | 95. | 02F05 | TNIFSVSPMG |
| | 96. | 02F06 | TNIFSITPMG |
| | 97. | 02F07 | TNIESISPMG |
| | 98. | 02F11 | TNIFSTSPMG |
| | 99. | 02F12 | TNIESISPMG |
| | 100. | 02G01 | TNIFSINPMG |
| | 101. | 02G02 | TNIFSITPMG |
| | 102. | 02G05 | TNITSISPMG |
| | 103. | 02G06 | TNIFSGSPMG |
| | 104. | 02G07 | TNIFSITPMG |
| | 105. | 02G08 | TNIDSISPMG |
| | 106. | 02G09 | TNIFSDSPMG |
| | 107. | 02G11 | TNIDSISPMG |
| | 108. | 02H01 | TNIFSKSPMG |
| | 109. | 02H04 | TNIFSVSPMG |
| | 110. | 02H05 | TNQFSISPMG |
| | 111. | 02H06 | TNIRSISPMG |
| | 112. | 02H09 | TNIFSRSPMG |
| | 113. | 02H11 | TNITSISPMG |
| | 114. | 01F07-M34Y | TNIFSTSPYG |
| | 115. | 01F01-M34G | TNIFSTSPGG |
| | 116. | 02G02-M34Y | TNIFSITPYG |
| | 117. | 02G02-M34G | TNIFSITPGG |

| **SEQ ID NO:** | | **Name** | **CDR2** |
|---|---|---|---|
| | 118. | 01A01 | AIHGGSTLYADSVK |
| | 119. | 01A02 | AINGFSTLYADSVK |
| | 120. | 01A03 | AIHGSSTLYADSVK |
| | 121. | 01A04 | AIHGDSTLYADSVK |
| | 122. | 01A05 | AIHGFSTLYADSVK |
| | 123. | 01A06 | AIHGFSTVYADSVK |
| | 124. | 01A07 | AIHGTSTLYADSVK |
| | 125. | 01A08 | AIHGESTLYADSVK |
| | 126. | 01A09 | AIHGRSTLYADSVK |
| | 127. | 01B01 | AIHGESTLYADSVK |
| | 128. | 01B02 | AISGFSTLYADSVK |
| | 129. | 01B03 | AIHGKSTLYADSVK |
| | 130. | 01B04 | AIHGKSTLYADSVK |
| | 131. | 01B05 | AIHGFETLYADSVK |
| | 132. | 01B06 | AIHGDSTLYADSVK |
| | 133. | 01B07 | AIHGNSTLYADSVK |
| | 134. | 01B08 | AIHGSSTLYADSVK |
| | 135. | 01B09 | AIHGSSTLYADSVK |
| | 136. | 01B12 | AIHGFQTLYADSVK |
| | 137. | 01C01 | AIHGHSTLYADSVK |
| | 138. | 01C02 | AIHGNSTLYADSVK |
| | 139. | 01C04 | AIHGDSTLYADSVK |
| | 140. | 01C05 | AIHGFKTLYADSVK |
| | 141. | 01C06 | AIHGDSTLYADSVK |
| | 142. | 01C07 | AIHGFSTYYADSVK |
| | 143. | 01C08 | AIHGGSTLYADSVK |
| | 144. | 01C10 | AIHGFSTLYADSVK |
| | 145. | 01D02 | AIHGKSTLYADSVK |
| | 146. | 01D03 | AIHGDSTLYADSVK |
| | 147. | 01D04 | AIHGVSTLYADSVK |
| | 148. | 01D05 | AIHGTSTLYADSVK |
| | 149. | 01D06 | AIHGDSTLYADSVK |
| | 150. | 01D07 | AIHGSSTLYADSVK |
| | 151. | 01D10 | AIHGSSTLYADSVK |
| | 152. | 01E03 | AIHGDSTLYADSVK |
| | 153. | 01E04 | AIHGTSTLYADSVK |
| | 154. | 01E05 | AIHGTSTLYADSVK |
| | 155. | 01E06 | AIHGDSTLYADSVK |
| | 156. | 01E07 | AIHGQSTLYADSVK |
| | 157. | 01E08 | AIHGDSTLYADSVK |
| | 158. | 01E10 | AIHGKSTLYADSVK |
| | 159. | 01F02 | AIHGTSTLYADSVK |
| | 160. | 01F03 | AIHGNSTLYADSVK |
| | 161. | 01F04 | AIHGFQTLYADSVK |
| | 162. | 01F05 | AIHGFSTWYADSVK |
| | 163. | 01F07 | AIHGFSTIYADSVK |
| | 164. | 01F08 | AIHGPSTLYADSVK |
| | 165. | 01F09 | AIHGHSTLYADSVK |
| | 166. | 01F10 | AIHGESTLYADSVK |
| | 167. | 01F12 | AIHGDSTLYADSVK |
| | 168. | 01G01 | AIHGDSTLYADSVK |
| | 169. | 01G04 | AIHGNSTLYADSVK |
| | 170. | 01G06 | AIHGFETLYADSVK |
| | 171. | 01G08 | AIHGFETLYADSVK |
| | 172. | 01G09 | AIHGFSTYYADSVK |
| | 173. | 01G10 | AIHGLSTLYADSVK |
| | 174. | 01G11 | AIHGASTLYADSVK |
| | 175. | 01H01 | AIHGQSTLYADSVK |
| | 176. | 01H04 | AIHGQSTLYADSVK |
| | 177. | 01H05 | AIHGTSTLYADSVK |
| | 178. | 01H06 | AIHGFETLYADSVK |
| | 179. | 01H08 | AIHGFSTVYADSVK |
| | 180. | 01H09 | AIHGNSTLYADSVK |
| | 181. | 01H10 | AIHGESTLYADSVK |
| | 182. | 01H11 | AIHGFSTLYADSVK |
| | 183. | 02A04 | AIHGKSTLYADSVK |
| | 184. | 02A05 | AIHGKSTLYADSVK |
| | 185. | 02A07 | AIHGNSTLYADSVK |
| | 186. | 02A08 | AIHGESTLYADSVK |
| | 187. | 02A11 | AIHGSSTLYADSVK |
| | 188. | 02B01 | AIHGRSTLYADSVK |
| | 189. | 02B04 | AIHGFSTIYADSVK |
| | 190. | 02B05 | AIHGTSTLYADSVK |
| | 191. | 02B06 | AIHGFSTLYADSVK |
| | 192. | 02B07 | AIHGHSTLYADSVK |
| | 193. | 02B11 | AIHGDSTLYADSVK |
| | 194. | 02B12 | AIHGFDTLYADSVK |
| | 195. | 02C01 | AIHGASTLYADSVK |
| | 196. | 02C03 | AIHGSSTLYADSVK |
| | 197. | 02C05 | AIHGFTTLYADSVK |
| | 198. | 02C06 | AIHGTSTLYADSVK |
| | 199. | 02D06 | AIHGFSTVYADSVK |
| | 200. | 02D09 | AIHGKSTLYADSVK |
| | 201. | 02D11 | AIHGESTLYADSVK |
| | 202. | 02E03 | AIHGPSTLYADSVK |
| | 203. | 02E05 | AIHGISTLYADSVK |
| | 204. | 02E06 | AIHGFSTFYADSVK |
| | 205. | 02E09 | AIHGGSTLYADSVK |
| | 206. | 02F02 | AIHGSSTLYADSVK |
| | 207. | 02F03 | AIHGSSTLYADSVK |
| | 208. | 02F04 | AIHGFSTLYADSVK |
| | 209. | 02F05 | AIHGNSTLYADSVK |
| | 210. | 02F06 | AIHGESTLYADSVK |
| | 211. | 02F07 | AIHGFSTLYADSVK |
| | 212. | 02F11 | AIHGTSTLYADSVK |
| | 213. | 02F12 | AIHGTSTLYADSVK |
| | 214. | 02G01 | AIHGFDTLYADSVK |
| | 215. | 02G02 | AIHGASTLYADSVK |
| | 216. | 02G05 | AIHGNSTLYADSVK |
| | 217. | 02G06 | AIHGNSTLYADSVK |
| | 218. | 02G07 | AIHGESTLYADSVK |
| | 219. | 02G08 | AIHGESTLYADSVK |
| | 220. | 02G09 | AIHGFSTLYADSVK |
| | 221. | 02G11 | AIHGSSTLYADSVK |
| | 222. | 02H01 | AIHGSSTLYADSVK |
| | 223. | 02H04 | AIHGNSTLYADSVK |
| | 224. | 02H05 | AIHGKSTLYADSVK |
| | 225. | 02H06 | AIHGSSTLYADSVK |
| | 226. | 02H09 | AIHGSSTLYADSVK |
| | 227. | 02H11 | AIHGESTLYADSVK |
| | 228. | 01F07-M34Y | AIHGFSTIYADSVK |
| | 229. | 01F01-M34G | AIHGFSTIYADSVK |
| | 230. | 02G02-M34Y | AIHGASTLYADSVK |
| | 231. | 02G02-M34G | AIHGASTLYADSVK |

| | **SEQ ID NO:** | **Name** | **CDR3** |
|---|---|---|---|
| | 232. | 01A01 | VPWGDYHPRNVA |
| | 233. | 01A02 | VPWGDYHPRNVH |
| | 234. | 01A03 | VPWGDYHPRNVY |
| | 235. | 01A04 | VPWGRYHPRNVY |
| | 236. | 01A05 | VPWGDYHPRNVY |
| | 237. | 01A06 | VPWGDYHPRNVY |
| | 238. | 01A07 | VPWGDYHPGNVY |
| | 239. | 01A08 | VPWGDYHPRKVY |
| | 240. | 01A09 | VPWGSYHPRNVY |
| | 241. | 01B01 | VPWGDYHPRNVA |
| | 242. | 01B02 | VPWGDYHPRNVY |
| | 243. | 01B03 | VPWGDYHPRNVV |
| | 244. | 01B04 | VPWGDYHPRNVK |
| | 245. | 01B05 | VPWGDYHPGNVY |
| | 246. | 01B06 | VPWGEYHPRNVY |
| | 247. | 01B07 | VPWGIYHPRNVY |
| | 248. | 01B08 | VPWGRYHPRNVY |
| | 249. | 01B09 | VPWGDYHPGNVY |
| | 250. | 01B12 | VPWGDYHPRNVV |
| | 251. | 01C01 | VPWGDYHPGNVY |
| | 252. | 01C02 | VPWGRYHPRNVY |
| | 253. | 01C04 | VPWGDYHPRNVY |
| | 254. | 01C05 | VPWGDYHPGNVY |
| | 255. | 01C06 | VPWGKYHPRNVY |
| | 256. | 01C07 | VPWGSYHPRNVY |
| | 257. | 01C08 | VPWGDYHPRNVH |
| | 258. | 01C10 | VPWGYYHPRNVY |
| | 259. | 01D02 | VPWGDYHPGNVY |
| | 260. | 01D03 | VPWGDYHPRNVR |
| | 261. | 01D04 | VPWGDYHPRNVQ |
| | 262. | 01D05 | VPWGDYHPRNVY |
| | 263. | 01D06 | VPWGDYHPRNVT |
| | 264. | 01D07 | VPWGDYHPRNVN |
| | 265. | 01D10 | VPWGRYHPRNVY |
| | 266. | 01E03 | VPWGDYHPGNVY |
| | 267. | 01E04 | VPWGDYHPGNVY |
| | 268. | 01E05 | VPWGKYHPRNVY |
| | 269. | 01E06 | VPWGDYHPRNVY |
| | 270. | 01E07 | VPWGDYHPRNVQ |
| | 271. | 01E08 | VPWGDYHPGNVC |
| | 272. | 01E10 | VPWGDYHPRRVY |
| | 273. | 01F02 | VPWGRYHPRNVY |
| | 274. | 01F03 | VPWGTYHPRNVY |
| | 275. | 01F04 | VPWGDYHPGNVY |
| | 276. | 01F05 | VPWGRYHPRNVY |
| | 277. | 01F07 | VPWGDYHPGNVY |
| | 278. | 01F08 | VPWGDYHPTNVY |
| | 279. | 01F09 | VPWGRYHPRNVY |
| | 280. | 01F10 | VPWGDYHPRNVT |
| | 281. | 01F12 | VPWGRYHPRNVY |
| | 282. | 01G01 | VPWGDYHPRRVY |
| | 283. | 01G04 | VPWGDYHPRMVY |
| | 284. | 01G06 | VPWGDYHPRNVL |
| | 285. | 01G08 | VPWGDYHPGNVY |
| | 286. | 01G09 | VPWGRYHPRNVY |
| | 287. | 01G10 | VPWGAYHPRNVY |
| | 288. | 01G11 | VPWGDYHPRNVA |
| | 289. | 01H01 | VPWGDYHPQNVY |
| | 290. | 01H04 | VPWGDYHPRNVT |
| | 291. | 01H05 | VPWGRYHPRNVY |
| | 292. | 01H06 | VPWGDYHPGNVY |
| | 293. | 01H08 | VPWGDYHPGNVY |
| | 294. | 01H09 | VPWGDYHPGNVY |
| | 295. | 01H10 | VPWGDYHPRNVY |
| | 296. | 01H11 | VPWGDYHPGNVY |
| | 297. | 02A04 | VPWGDYHPSNVY |
| | 298. | 02A05 | VPWGDYHPGNVY |
| | 299. | 02A07 | VPWGDYHPREVY |
| | 300. | 02A08 | VPWGRYHPGNVY |
| | 301. | 02A11 | VPWGDYHPRVVY |
| | 302. | 02B01 | VPWGDYHPRNVM |
| | 303. | 02B04 | VPWGDYHPLNVY |
| | 304. | 02B05 | VPWGDYHPGNVY |
| | 305. | 02B06 | VPWGDYHPGNVY |
| | 306. | 02B07 | VPWGDYHPRNVT |
| | 307. | 02B11 | VPWGDYHPRNVS |
| | 308. | 02B12 | VPWGDYHPRNVY |
| | 309. | 02C01 | VPWGDYHPGNVY |
| | 310. | 02C03 | VPWGDYHPGNVY |
| | 311. | 02C05 | VPWGDYHPRNVT |
| | 312. | 02C06 | VPWGDYHPGNVY |
| | 313. | 02D06 | VPWGRYHPRNVY |
| | 314. | 02D09 | VPWGDYHPNNVY |
| | 315. | 02D11 | VPWGDYHPGNVY |
| | 316. | 02E03 | VPWGDYHPRNVT |
| | 317. | 02E05 | VPWGDYHPGNVY |
| | 318. | 02E06 | VPWGDYHPGNVY |
| | 319. | 02E09 | VPWGDYHPRNVA |
| | 320. | 02F02 | VPWGDYHPGNVY |
| | 321. | 02F03 | VPWGDYHPKNVY |
| | 322. | 02F04 | VPWGDYHPGNVY |
| | 323. | 02F05 | VPWGKYHPRNVY |
| | 324. | 02F06 | VPWGRYHPRNVY |
| | 325. | 02F07 | VPWGDYHPGNVY |
| | 326. | 02F11 | VPWGDYHPRNVQ |
| | 327. | 02F12 | VPWGDYHPGNVY |
| | 328. | 02G01 | VPWGDYHPRNVS |
| | 329. | 02G02 | VPWGDYHPGNVY |
| | 330. | 02G05 | VPWGDYHPGNVY |
| | 331. | 02G06 | VPWGDYHPGNVY |
| | 332. | 02G07 | VPWGDYHPRDVY |
| | 333. | 02G08 | VPWGDYHPRNVT |
| | 334. | 02G09 | VPWGDYHPRNVA |
| | 335. | 02G11 | VPWGDYHPRNVT |
| | 336. | 02H01 | VPWGDYHPRNVY |
| | 337. | 02H04 | VPWGDYHPRNVY |
| | 338. | 02H05 | VPWGDYHPRNVV |
| | 339. | 02H06 | VPWGDYHPRNVV |
| | 340. | 02H09 | VPWGDYHPGNVY |
| | 341. | 02H11 | VPWGDYHPRNVY |
| | 342. | 01F07-M34Y | VPWGDYHPGNVY |
| | 343. | 01F01-M34G | VPWGDYHPGNVY |
| | 344. | 02G02-M34Y | VPWGDYHPGNVY |
| | 345. | 02G02-M34G | VPWGDYHPGNVY |

| **SEQ ID NO** | **Construct Name** | **VHH Sequences** |
|---|---|---|
| 346. | BH2T | EVQLVESGGGLVQPGRSLTLSCAASTNIFSISPMGWYRQAPGKQRELVAAIHGFS TLYADSVKGRFTISRDNAKNSIYLQMNSLRPEDTALYYCNKVPWGDYHPRNVYWG QGTQVTVSS |
| 347. | 01A01 | EVQLVESGGGLVQPGRSLTLSCAASTDIFSISPMGWYRQAPGKQRELVAAIHGGS TLYADSVKGRFTISRDNAKNSIYLQMNSLRPEDTALYYCNKVPWGDYHPRNVAWG QGTQVTVSS |
| 348. | 02E09 | EVQLVESGGGLVQPGRSLTLSCAASTNDFSISPMGWYRQAPGKQRELVAAIHGGS TLYADSVKGRFTISRDNAKNSIYLQMNSLRPEDTALYYCNKVPWGDYHPRNVAWG QGTQVTVSS |
| 349. | 01B03 | EVQLVESGGGLVQPGRSLTLSCAASTNIFSKSPMGWYRQAPGKQRELVAAIHGKS TLYADSVKGRFTISRDNAKNSIYLQMNSLRPEDTALYYCNKVPWGDYHPRNVVWG QGTQVTVSS |
| 350. | 01B04 | EVQLVESGGGLVQPGRSLTLSCAASTNDFSISPMGWYRQAPGKQRELVAAIHGKS TLYADSVKGRFTISRDNAKNSIYLQMNSLRPEDTALYYCNKVPWGDYHPRNVKWG QGTQVTVSS |
| 351. | 02H05 | EVQLVESGGGLVQPGRSLTLSCAASTNQFSISPMGWYRQAPGKQRELVAAIHGKS TLYADSVKGRFTISRDNAKNSIYLQMNSLRPEDTALYYCNKVPWGDYHPRNVVWG QGTQVTVSS |
| 352. | 01A02 | EVQLVESGGGLVQPGRSLTLSCAASTNIFSSSPMGWYRQAPGKQRELVAAINGFS TLYADSVKGRFTISRDNAKNSIYLQMNSLRPEDTALYYCNKVPWGDYHPRNVHWG QGTQVTVSS |
| 353. | 01A05 | EVQLVESGGGLVQPGRSLTLSCAASTNIFSSSPMGWYRQAPGKQRELVAAIHGFS TLYADSVKGRFTISRDNAKNSIYLQMNSLRPEDTALYYCNKVPWGDYHPRNVYWG QGTQVTVSS |
| 354. | 01B12 | EVQLVESGGGLVQPGRSLTLSCAASTNIFSTSPMGWYRQAPGKQRELVAAIHGFQ TLYADSVKGRFTISRDNAKNSIYLQMNSLRPEDTALYYCNKVPWGDYHPRNVVWG QGTQVTVSS |
| 355. | 01G06 | EVQLVESGGGLVQPGRSLTLSCAASTNIFSRSPMGWYRQAPGKQRELVAAIHGFE TLYADSVKGRFTISRDNAKNSIYLQMNSLRPEDTALYYCNKVPWGDYHPRNVLWG QGTQVTVSS |
| 356. | 02C05 | EVQLVESGGGLVQPGRSLTLSCAASTNIFSESPMGWYRQAPGKQRELVAAIHGFT TLYADSVKGRFTISRDNAKNSIYLQMNSLRPEDTALYYCNKVPWGDYHPRNVTWG QGTQVTVSS |
| 357. | 02G09 | EVQLVESGGGLVQPGRSLTLSCAASTNIFSDSPMGWYRQAPGKQRELVAAIHGFS TLYADSVKGRFTISRDNAKNSIYLQMNSLRPEDTALYYCNKVPWGDYHPRNVAWG QGTQVTVSS |
| 358. | 01C08 | EVQLVESGGGLVQPGRSLTLSCAASTNIFSNSPMGWYRQAPGKQRELVAAIHGGS TLYADSVKGRFTISRDNAKNSIYLQMNSLRPEDTALYYCNKVPWGDYHPRNVHWG QGTQVTVSS |
| 359. | 02B01 | EVQLVESGGGLVQPGRSLTLSCAASTNIFSNSPMGWYRQAPGKQRELVAAIHGRS TLYADSVKGRFTISRDNAKNSIYLQMNSLRPEDTALYYCNKVPWGDYHPRNVMWG QGTQVTVSS |
| 360. | 02E03 | EVQLVESGGGLVQPGRSLTLSCAASTNIFSNSPMGWYRQAPGKQRELVAAIHGPS TLYADSVKGRFTISRDNAKNSIYLQMNSLRPEDTALYYCNKVPWGDYHPRNVTWG QGTQVTVSS |
| 361. | 01D03 | EVQLVESGGGLVQPGRSLTLSCAASTNIFSNSPMGWYRQAPGKQRELVAAIHGDS TLYADSVKGRFTISRDNAKNSIYLQMNSLRPEDTALYYCNKVPWGDYHPRNVRWG QGTQVTVSS |
| 362. | 01D06 | EVQLVESGGGLVQPGRSLTLSCAASTNIFSRSPMGWYRQAPGKQRELVAAIHGDS TLYADSVKGRFTISRDNAKNSIYLQMNSLRPEDTALYYCNKVPWGDYHPRNVTWG QGTQVTVSS |
| 363. | 01H04 | EVQLVESGGGLVQPGRSLTLSCAASTNIFSKSPMGWYRQAPGKQRELVAAIHGQS TLYADSVKGRFTISRDNAKNSIYLQMNSLRPEDTALYYCNKVPWGDYHPRNVTWG QGTQVTVSS |
| 364. | 02B07 | EVQLVESGGGLVQPGRSLTLSCAASTNIFSSSPMGWYRQAPGKQRELVAAIHGHS TLYADSVKGRFTISRDNAKNSIYLQMNSLRPEDTALYYCNKVPWGDYHPRNVTWG QGTQVTVSS |
| 365. | 01A08 | EVQLVESGGGLVQPGRSLTLSCAASTNIFSSSPMGWYRQAPGKQRELVAAIHGES TLYADSVKGRFTISRDNAKNSIYLQMNSLRPEDTALYYCNKVPWGDYHPRKVYWG QGTQVTVSS |
| 366. | 01B07 | EVQLVESGGGLVQPGRSLTLSCAASTNIFSRSPMGWYRQAPGKQRELVAAIHGNS TLYADSVKGRFTISRDNAKNSIYLQMNSLRPEDTALYYCNKVPWGIYHPRNVYWG QGTQVTVSS |
| 367. | 01F03 | EVQLVESGGGLVQPGRSLTLSCAASTNIFSESPMGWYRQAPGKQRELVAAIHGNS TLYADSVKGRFTISRDNAKNSIYLQMNSLRPEDTALYYCNKVPWGTYHPRNVYWG QGTQVTVSS |
| 368. | 02F05 | EVQLVESGGGLVQPGRSLTLSCAASTNIFSVSPMGWYRQAPGKQRELVAAIHGNS TLYADSVKGRFTISRDNAKNSIYLQMNSLRPEDTALYYCNKVPWGKYHPRNVYWG QGTQVTVSS |
| 369. | 02H04 | EVQLVESGGGLVQPGRSLTLSCAASTNIFSVSPMGWYRQAPGKQRELVAAIHGNS TLYADSVKGRFTISRDNAKNSIYLQMNSLRPEDTALYYCNKVPWGDYHPRNVYWG QGTQVTVSS |
| 370. | 02A07 | EVQLVESGGGLVQPGRSLTLSCAASTNIFSKSPMGWYRQAPGKQRELVAAIHGNS TLYADSVKGRFTISRDNAKNSIYLQMNSLRPEDTALYYCNKVPWGDYHPREVYWG QGTQVTVSS |
| 371. | 01D05 | EVQLVESGGGLVQPGRSLTLSCAASTNIFSDSPMGWYRQAPGKQRELVAAIHGTS TLYADSVKGRFTISRDNAKNSIYLQMNSLRPEDTALYYCNKVPWGDYHPRNVYWG QGTQVTVSS |
| 372. | 01E05 | EVQLVESGGGLVQPGRSLTLSCAASTNIFSRSPMGWYRQAPGKQRELVAAIHGTS TLYADSVKGRFTISRDNAKNSIYLQMNSLRPEDTALYYCNKVPWGKYHPRNVYWG QGTQVTVSS |
| 373. | 01F02 | EVQLVESGGGLVQPGRSLTLSCAASTNIFSHSPMGWYRQAPGKQRELVAAIHGTS TLYADSVKGRFTISRDNAKNSIYLQMNSLRPEDTALYYCNKVPWGRYHPRNVYWG QGTQVTVSS |
| 374. | 02C06 | EVQLVESGGGLVQPGRSLTLSCAASTNIFSTSPMGWYRQAPGKQRELVAAIHGTS TLYADSVKGRFTISRDNAKNSIYLQMNSLRPEDTALYYCNKVPWGDYHPGNVYWG QGTQVTVSS |
| 375. | 02F11 | EVQLVESGGGLVQPGRSLTLSCAASTNIFSTSPMGWYRQAPGKQRELVAAIHGTS TLYADSVKGRFTISRDNAKNSIYLQMNSLRPEDTALYYCNKVPWGDYHPRNVQWG QGTQVTVSS |
| 376. | 01E06 | EVQLVESGGGLVQPGRSLTLSCAASTNIFSLSPMGWYRQAPGKQRELVAAIHGDS TLYADSVKGRFTISRDNAKNSIYLQMNSLRPEDTALYYCNKVPWGDYHPRNVYWG QGTQVTVSS |
| 377. | 01A03 | EVQLVESGGGLVQPGRSLTLSCAASTNIFSISPGGWYRQAPGKQRELVAAIHGSS TLYADSVKGRFTISRDNAKNSIYLQMNSLRPEDTALYYCNKVPWGDYHPRNVYWG QGTQVTVSS |
| 378. | 02A11 | EVQLVESGGGLVQPGRSLTLSCAASTNHFSISPMGWYRQAPGKQRELVAAIHGSS TLYADSVKGRFTISRDNAKNSIYLQMNSLRPEDTALYYCNKVPWGDYHPRVVYWG QGTQVTVSS |
| 379. | 01D07 | EVQLVESGGGLVQPGRSLTLSCAASTNIFSASPMGWYRQAPGKQRELVAAIHGSS TLYADSVKGRFTISRDNAKNSIYLQMNSLRPEDTALYYCNKVPWGDYHPRNVNWG QGTQVTVSS |
| 380. | 01D10 | EVQLVESGGGLVQPGRSLTLSCAASTNIFSASPMGWYRQAPGKQRELVAAIHGSS TLYADSVKGRFTISRDNAKNSIYLQMNSLRPEDTALYYCNKVPWGRYHPRNVYWG QGTQVTVSS |
| 381. | 01A07 | EVQLVESGGGLVQPGRSLTLSCAASTNISSISPMGWYRQAPGKQRELVAAIHGTS TLYADSVKGRFTISRDNAKNSIYLQMNSLRPEDTALYYCNKVPWGDYHPGNVYWG QGTQVTVSS |
| 382. | 02F12 | EVQLVESGGGLVQPGRSLTLSCAASTNIESISPMGWYRQAPGKQRELVAAIHGTS TLYADSVKGRFTISRDNAKNSIYLQMNSLRPEDTALYYCNKVPWGDYHPGNVYWG QGTQVTVSS |
| 383. | 02B05 | EVQLVESGGGLVQPGRSLTLSCAASTNIFSISPYGWYRQAPGKQRELVAAIHGTS TLYADSVKGRFTISRDNAKNSIYLQMNSLRPEDTALYYCNKVPWGDYHPGNVYWG QGTQVTVSS |
| 384. | 01E04 | EVQLVESGGGLVQPGRSLTLSCAASTNIASISPMGWYRQAPGKQRELVAAIHGTS TLYADSVKGRFTISRDNAKNSIYLQMNSLRPEDTALYYCNKVPWGDYHPGNVYWG QGTQVTVSS |
| 385. | 02A05 | EVQLVESGGGLVQPGRSLTLSCAASTNIASISPMGWYRQAPGKQRELVAAIHGKS TLYADSVKGRFTISRDNAKNSIYLQMNSLRPEDTALYYCNKVPWGDYHPGNVYWG QGTQVTVSS |
| 386. | 02C03 | EVQLVESGGGLVQPGRSLTLSCAASTNIASISPMGWYRQAPGKQRELVAAIHGSS TLYADSVKGRFTISRDNAKNSIYLQMNSLRPEDTALYYCNKVPWGDYHPGNVYWG QGTQVTVSS |
| 387. | 01E03 | EVQLVESGGGLVQPGRSLTLSCAASTNITSISPMGWYRQAPGKQRELVAAIHGDS TLYADSVKGRFTISRDNAKNSIYLQMNSLRPEDTALYYCNKVPWGDYHPGNVYWG QGTQVTVSS |
| 388. | 01H09 | EVQLVESGGGLVQPGRSLTLSCAASTNIMSISPMGWYRQAPGKQRELVAAIHGNS TLYADSVKGRFTISRDNAKNSIYLQMNSLRPEDTALYYCNKVPWGDYHPGNVYWG QGTQVTVSS |
| 389. | 02G05 | EVQLVESGGGLVQPGRSLTLSCAASTNITSISPMGWYRQAPGKQRELVAAIHGNS TLYADSVKGRFTISRDNAKNSIYLQMNSLRPEDTALYYCNKVPWGDYHPGNVYWG QGTQVTVSS |
| 390. | 01C01 | EVQLVESGGGLVQPGRSLTLSCAASTNIVSISPMGWYRQAPGKQRELVAAIHGHS TLYADSVKGRFTISRDNAKNSIYLQMNSLRPEDTALYYCNKVPWGDYHPGNVYWG QGTQVTVSS |
| 391. | 01D02 | EVQLVESGGGLVQPGRSLTLSCAASTNIVSISPMGWYRQAPGKQRELVAAIHGKS TLYADSVKGRFTISRDNAKNSIYLQMNSLRPEDTALYYCNKVPWGDYHPGNVYWG QGTQVTVSS |
| 392. | 02D09 | EVQLVESGGGLVQPGRSLTLSCAASTNVVSISPMGWYRQAPGKQRELVAAIHGKS TLYADSVKGRFTISRDNAKNSIYLQMNSLRPEDTALYYCNKVPWGDYHPNNVYWG QGTQVTVSS |
| 393. | 02C01 | EVQLVESGGGLVQPGRSLTLSCAASTNIISISPMGWYRQAPGKQRELVAAIHGAS TLYADSVKGRFTISRDNAKNSIYLQMNSLRPEDTALYYCNKVPWGDYHPGNVYWG QGTQVTVSS |
| 394. | 02G02 | EVQLVESGGGLVQPGRSLTLSCAASTNIFSITPMGWYRQAPGKQRELVAAIHGAS TLYADSVKGRFTISRDNAKNSIYLQMNSLRPEDTALYYCNKVPWGDYHPGNVYWG QGTQVTVSS |
| 395. | 01B05 | EVQLVESGGGLVQPGRSLTLSCAASTNITSISPMGWYRQAPGKQRELVAAIHGFE TLYADSVKGRFTISRDNAKNSIYLQMNSLRPEDTALYYCNKVPWGDYHPGNVYWG QGTQVTVSS |
| 396. | 01G08 | EVQLVESGGGLVQPGRSLTLSCAASTNIQSISPMGWYRQAPGKQRELVAAIHGFE TLYADSVKGRFTISRDNAKNSIYLQMNSLRPEDTALYYCNKVPWGDYHPGNVYWG QGTQVTVSS |
| 397. | 01H06 | EVQLVESGGGLVQPGRSLTLSCAASTSDFSISPMGWYRQAPGKQRELVAAIHGFE TLYADSVKGRFTISRDNAKNSIYLQMNSLRPEDTALYYCNKVPWGDYHPGNVYWG QGTQVTVSS |
| 398. | 01F04 | EVQLVESGGGLVQPGRSLTLSCAASTNIDSISPMGWYRQAPGKQRELVAAIHGFQ TLYADSVKGRFTISRDNAKNSIYLQMNSLRPEDTALYYCNKVPWGDYHPGNVYWG QGTQVTVSS |
| 399. | 01H08 | EVQLVESGGGLVQPGRSLTLSCAASTNIMSISPMGWYRQAPGKQRELVAAIHGFS TVYADSVKGRFTISRDNAKNSIYLQMNSLRPEDTALYYCNKVPWGDYHPGNVYWG QGTQVTVSS |
| 400. | 02F07 | EVQLVESGGGLVQPGRSLTLSCAASTNIESISPMGWYRQAPGKQRELVAAIHGFS TLYADSVKGRFTISRDNAKNSIYLQMNSLRPEDTALYYCNKVPWGDYHPGNVYWG QGTQVTVSS |
| 401. | 01C05 | EVQLVESGGGLVQPGRSLTLSCAASTNIFSSSPMGWYRQAPGKQRELVAAIHGFK TLYADSVKGRFTISRDNAKNSIYLQMNSLRPEDTARYYCNKVPWGDYHPGNVYWG QGTQVTVSS |
| 402. | 02F04 | EVQLVESGGGLVQPGRSLTLSCAASTNIFSSSPMGWYRQAPGKQRELVAAIHGFS TLYADSVKGRFTISRDNAKNSIYLQMNSLRPEDTALYYCNKVPWGDYHPGNVYWG QGTQVTVSS |
| 403. | 02B06 | EVQLVESGGGLVQPGRSLTLSCAASTNIFSNSPMGWYRQAPGKQRELVAAIHGFS TLYADSVKGRFTISRDNAKNSIYLQMNSLRPEDTALYYCNKVPWGDYHPGNVYWG QGTQVTVSS |
| 404. | 01F07 | EVQLVESGGGLVQPGRSLTLSCAASTNIFSTSPMGWYRQAPGKQRELVAAIHGFS TIYADSVKGRFTISRDNAKNSIYLQMNSLRPEDTALYYCNKVPWGDYHPGNVYWG QGTQVTVSS |
| 405. | 02B04 | EVQLVESGGGLVQPGRSLTLSCAASTNIFSTSPMGWYRQAPGKQRELVAAIHGFS TIYADSVKGRFTISRDNAKNSIYLQMNSLRPEDTALYYCNKVPWGDYHPLNVYWG QGTQVTVSS |
| 406. | 01H11 | EVQLVESGGGLVQPGRSLTLSCVASTNIFSTSPMGWYRQAPGKQRELVAAIHGFS TLYADSVKGRFTISRDNAKNSIYLQMNSLRPEDTALYYCNKVPWGDYHPGNVYWG QGTQVTVSS |
| 407. | 02E06 | EVQLVESGGGLVQPGRSLTLSCAASTNIFSDSPMGWYRQAPGKQRELVAAIHGFS TFYADSVKGRFTISRDNAKNSIYLQMNSLRPEDTALYYCNKVPWGDYHPGNVYWG QGTQVTVSS |
| 408. | 01E08 | EVQLVESGGGLVQPGRSLTLSCAASTNIFSQSPMGWYRQAPGKQRELVAAIHGDS TLYADSVKGRFTISRDNAKNSIYLQMNSLRPEDTALYYCNKVPWGDYHPGNVCWG QGTQVTVSS |
| 409. | 02A04 | EVQLVESGGGLVQPGRSLTLSCAASTNIFSQSPMGWYRQAPGKQRELVAAIHGKS TLYADSVKGRFTISRDNAKNSIYLQMNSLRPEDTALYYCNKVPWGDYHPSNVYWG KGTQVTVSS |
| 410. | 02A08 | EVQLVESGGGLVQPGRSLTLSCAASTNIFSRSPMGWYRQAPGKQRELVAAIHGES TLYADSVKGRFTISRDNAKNSIYLQMNSLRPEDTALYYCNKVPWGRYHPGNVYWG QGTQVTVSS |
| 411. | 02E05 | EVQLVESGGGLVQPGRSLTLSCAASTNIFSRSPMGWYRQAPGKQRELVAAIHGIS TLYADSVKGRFTISRDNAKNSIYLQMNSLRPEDTALYYCNKVPWGDYHPGNVYWG QGTQVTVSS |
| 412. | 02H09 | EVQLVESGGGLVQPGRSLTLSCAASTNIFSRSPMGWYRQAPGKQRELVAAIHGSS TLYADSVKGRFTISRDNAKNSIYLQMNSLRPEDTALYYCNKVPWGDYHPGNVYWG QGTQVTVSS |
| 413. | 02G06 | EVQLVESGGGLVQPGRSLTLSCAASTNIFSGSPMGWYRQAPGKQRELVAAIHGNS TLYADSVKGRFTISRDNAKNSIYLQMNSLRPEDTALYYCNKVPWGDYHPGNVYWG QGTQVTVSS |
| 414. | 01B09 | EVQLVESGGGLVQPGRSLTLSCAASSNIFSISPMGWYRQAPGKQRELVAAIHGSS TLYADSVKGRFTISRDNAKNSIYLQMNSLRPEDTALYYCNKVPWGDYHPGNVYWG QGTQVTVSS |
| 415. | 02F03 | EVQLVESGGGLVQPGRSLTLSCAASTNIFSIYPMGWYRQAPGKQRELVAAIHGSS TLYADSVKGRFTISRDNAKNSIYLQMNSLRPEDTALYYCNKVPWGDYHPKNVYWG QGTQVTVSS |
| 416. | 02F02 | EVQLVESGGGLVQPGRSLTLSCAASTNIFSKSPMGWYRQAPGKQRELVAAIHGSS TLYADSVKGRFTISRDNAKNSIYLQMNSLRPEDTALYYCNKVPWGDYHPGNVYWG QGTQVTVSS |
| 417. | 02H01 | EVQLVESGGGLVQPGRSLTLSCAASTNIFSKSPMGWYRQAPGKQRELVAAIHGSS TLYADSVKGRFTISRDNAKNSIYLQMNSLRPEDTALYYCNKVPWGDYHPRNVYWG QGTQVTVSS |
| 418. | 01G10 | EVQLVESGGGLVQPGRSLTLSCAASTNEFSISPMGWYRQAPGKQRELVAAIHGLS TLYADSVKGRFTISRDNAKNSIYLQMNSLRPEDTALYYCNKVPWGAYHPRNVYWG QGTQVTVSS |
| 419. | 02D11 | EVQLVESGGGLVQPGRSLTLSCAASTNEFSISPMGWYRQAPGKQRELVAAIHGES TLYADSVKGRFTISRDNAKNSIYLQMNSLRPEDTALYYCNKVPWGDYHPGNVYWG QGTQVTVSS |
| 420. | 01B01 | EVQLVESGGGLVQPGRSLTLSCAASTNIPSISPMGWYRQAPGKQRELVAAIHGES TLYADSVKGRFTISRDNAKNSIYLQMNSLRPEDTALYYCNKVPWGDYHPRNVAWG QGTQVTVSS |
| 421. | 01G11 | EVQLVESGGGLVQPGRSLTLSCAASTNIPSISPMGWYRQAPGKQRELVAAIHGAS TLYADSVKGRFTISRDNAKNSIYLQMNSLRPEDTALYYCNKVPWGDYHPRNVAWG QGTQVTVSS |
| 422. | 01H10 | EVQLVESGGGLVQPGRSLTLSCAASTNIPSISPMGWYRQAPGKQRELVAAIHGES TLYADSVKGRFTISRDNAKNSIYLQMNSLRPEDTALYYCNKVPWGDYHPRNVYWG QGTQVTVSS |
| 423. | 01C04 | EVQLVESGGGLVQPGRSLTLSCAASTNIPSISPMGWYRQAPGKQRELVAAIHGDS TLYADSVKGRFTISRDNAKNSIYLQMNSLRPEDTALYYCNKVPWGDYHPRNVYWG QGTQVTVSS |
| 424. | 01D04 | EVQLVESGGGLVQPGRSLTLSCAASTNITSISPMGWYRQAPGKQRELVAAIHGVS TLYADSVKGRFTISRDNAKNSIYLQMNSLRPEDTALYYCNKVPWGDYHPRNVQWG QGTQVTVSS |
| 425. | 01E07 | EVQLVESGGGLVQPGRSLTLSCAASTNIPSISPMGWYRQAPGKQRELVAAIHGQS TLYADSVKGRFTISRDNAKNSIYLQMNSLRPEDTALYYCNKVPWGDYHPRNVQWG QGTQVTVSS |
| 426. | 02B11 | EVQLVESGGGLVQPGRSLTLSCAASTNIVSISPMGWYRQAPGKQRELVAAIHGDS TLYADSVKGRFTISRDNAKNSIYLQMNSLRPEDTALYYCNKVPWGDYHPRNVSWG QGTQVTVSS |
| 427. | 01F10 | EVQLVESGGGLVQPGRSLTLSCAASSNIFSISPMGWYRQAPGKQRELVAAIHGES TLYADSVKGRFTISRDNAKNSIYLQMNSLRPEDTALYYCNKVPWGDYHPRNVTWG QGTQVTVSS |
| 428. | 02G08 | EVQLVESGGGLVQPGRSLTLSCAASTNIDSISPMGWYRQAPGKQRELVAAIHGES TLYADSVKGRFTISRDNAKNSIYLQMNSLRPEDTALYYCNKVPWGDYHPRNVTWG QGTQVTVSS |
| 429. | 02G11 | EVQLVESGGGLVQPGRSLTLSCAASTNIDSISPMGWYRQAPGKQRELVAAIHGSS TLYADSVKGRFTISRDNAKNSIYLQMNSLRPEDTALYYCNKVPWGDYHPRNVTWG QGTQVTVSS |
| 430. | 02H06 | EVQLVESGGGLVQPGRSLTLSCAASTNIRSISPMGWYRQAPGKQRELVAAIHGSS TLYADSVKGRFTISRDNAKNSIYLQMNSLRPEDTALYYCNKVPWGDYHPRNVVWG QGTQVTVSS |
| 431. | 01B02 | EVQLVESGGGLVQPGRSLTLSCAASTNITSISPMGWYRQAPGKQRELVAAISGFS TLYADSVKGRFTISRDNAKNSIYLQMNSLRPEDTALYYCNEVPWGDYHPRNVYWG QGTQVTVSS |
| 432. | 02H11 | EVQLVESGGGLVQPGRSLTLSCAASTNITSISPMGWYRQAPGKQRELVAAIHGES TLYADSVKGRFTISRDNAKNSIYLQMNSLRPEDTALYYCNKVPWGDYHPRNVYWG QGTQVTVSS |
| 433. | 01F08 | EVQLVESGGGLVQPGRSLTLSCAASTNITSVSPMGWYRQAPGKQRELVAAIHGPS TLYADSVKGRFTISRDNAKNSIYLQMNSLRPEDTALYYCNKVPWGDYHPTNVYWG QGTQVTVSS |
| 434. | 01H01 | EVQLVESGGGLVQPGRSLTLSCAASTNIGSISPMGWYRQAPGKQRELVAAIHGQS TLYADSVKGRFTISRDNAKNSIYLQMNSLRPEDTALYYCNKVPWGDYHPQNVYWG QGTQVTVSS |
| 435. | 01E10 | EVQLVESGGGLVQPGRSLTLSCAASTNIESISPMGWYRQAPGKQRELVAAIHGKS TLYADSVKGRFTISRDNAKNSIYLQMNSLRPEDTALYYCNKVPWGDYHPRRVYWG QGTQVTVSS |
| 436. | 01G01 | EVQLVESGGGLVQPGRSLTLSCAASTNIVSISPMGWYRQAPGKQRELVAAIHGDS TLYADSVKGRFTISRDNAKNSIYLQMNSLRPEDTALYYCNKVPWGDYHPRRVYWG QGTQVTVSS |
| 437. | 01G04 | EVQLVESGGGLVQPGRSLTLSCAASTNIDSISPMGWYRQAPGKQRELVAAIHGNS TLYADSVKGRFTISRDNAKNSIYLQMNSLRPEDTALYYCNKVPWGDYHPRMVYWG QGTQVTVSS |
| 438. | 01A04 | EVQLVESGGGLVQPGRSLTLSCAASTNIFMISPMGWYRQAPGKQRELVAAIHGDS TLYADSVKGRFTISRDNAKNSIYLQMNSLRPEDTALYYCNKVPWGRYHPRNVYWG QGTQVTVSS |
| 439. | 01F12 | EVQLVESGGGLVQPGRSLTLSCAASTNIFRISPMGWYRQAPGKQRELVAAIHGDS TLYADSVKGRFTISRDNAKNSIYLQMNSLRPEDTALYYCNKVPWGRYHPRNVYWG QGTQVTVSS |
| 440. | 01B06 | EVQLVESGGGLVQPGRSLTLSCAASTNIFSISPMGWYRQAPGKQRELVAAIHGDS TLYADSVKGRFTISRDNAKNSIYLQMNSLRPEDTALYYCNKVPWGEYHPRNVYWG QGTQVTVSS |
| 441. | 01C06 | EVQLVESGGGLVQPGRSLTLSCAASTNIFSISPMGWYRQAPGKQRELVAAIHGDS TLYADSVKGRFTISRDNAKNSIYLQMNSLRPEDTALYYCNKVPWGKYHPRNVYWG QGTQVTVSS |
| 442. | 01B08 | EVQLVESGGGLVQPGRSLTLSCAASTNIESISPMGWYRQAPGKQRELVAAIHGSS TLYADSVKGRFTISRDNAKNSIYLQMNSLRPEDTALYYCNKVPWGRYHPRNVYWG QGTQVTVSS |
| 443. | 01C02 | EVQLVESGGGLVQPGRSLTLSCAASTNIESISPMGWYRQAPGKQRELVAAIHGNS TLYADSVKGRFTISRDNAKNSIYLQMNSLRPEDTALYYCNKVPWGRYHPRNVYWG QGTQVTVSS |
| 444. | 01C10 | EVQLVESGGGLVQPGRSLTLSCAASTNISSISPMGWYRQAPGKQRELVAAIHGFS TLYADSVKGRFTISRDNAKNSIYLQMNSLRPEDTALYYCNKVPWGYYHPRNVYWG QGTQVTVSS |
| 445. | 01F09 | EVQLVESGGGLVQPGRSLTLSCAASTNISSISPMGWYRQAPGKQRELVAAIHGHS TLYADSVKGRFTISRDNAKNSIYLQMNSLRPEDTALYYCNKVPWGRYHPRNVYWG QGTQVTVSS |
| 446. | 02D06 | EVQLVESGGGLVQPGRSLTLSCAASTNISSISPMGWYRQAPGKQRELVAAIHGFS TVYADSVKGRFTISRDNAKNSIYLQMNSLRPEDTALYYCNKVPWGRYHPRNVYWG QGTQVTVSS |
| 447. | 01A06 | EVQLVESGGGLVQPGRSLTLSCAASTNIFSIRPMGWYRQAPGKQRELVAAIHGFS TVYADSVKGRFTISRDNAKNSIYLQMNSLRPEDTALYYCNKVPWGDYHPRNVYWG QGTQVTVSS |
| 448. | 01C07 | EVQLVESGGGLVQPGRSLTLSCAASTNIFSIYPMGWYRQAPGKQRELVAAIHGFS TYYADSVKGRFTISRDNAKNSIYLQMNSLRPEDTALYYCNKVPWGSYHPRNVYWG QGTQVTVSS |
| 449. | 01G09 | EVQLVESGGGLVQPGRSLTLSCAASTNIFNISPMGWYRQAPGKQRELVAAIHGFS TYYADSVKGRFTISRDNAKNSIYLQMNSLRPEDTALYYCNKVPWGRYHPRNVYWG QGTQVTVSS |
| 450. | 01F05 | EVQLVESGGGLVQPGRSLTLSCAASTNIFSSSPMGWYRQAPGKQRELVAAIHGFS TWYADSVKGRFTISRDNAKNSIYLQMNSLRPEDTALYYCNKVPWGRYHPRNVYWG QGTQVTVSS |
| 451. | 02B12 | EVQLVESGGGLVQPGRSLTLSCAASTNISSISPMGWYRQAPGKQRELVAAIHGFD TLYADSVKGRFTISRDNAKNSIYLQMNSLRPEDTALYYCNKVPWGDYHPRNVYWG QGTQVTVSS |
| 452. | 02G01 | EVQLVESGGGLVQPGRSLTLSCAASTNIFSINPMGWYRQAPGKQRELVAAIHGFD TLYADSVKGRFTISRDNAKNSIYLQMNSLRPEDTALYYCNKVPWGDYHPRNVSWG QGTQVTVSS |
| 453. | 01A09 | EVQLVESGGGLVQPGRSLTLSCAASTNIFSITPMGWYRQAPGKQRELVAAIHGRS TLYADSVKGRFTISRDNAKNSIYLQMNSLRPEDTALYYCNKVPWGSYHPRNVYWG QGTQVTVSS |
| 454. | 01H05 | EVQLVESGGGLVQPGRSLTLSCAASTNIFSITPMGWYRQAPGKQRELVAAIHGTS TLYADSVKGRFTISRDNAKNSIYLQMNSLRPEDTALYYCNKVPWGRYHPRNVYWG QGTQVTVSS |
| 455. | 02F06 | EVQLVESGGGLVQPGRSLTLSCAASTNIFSITPMGWYRQAPGKQRELVAAIHGES TLYADSVKGRFTISRDNAKNSIYLQMNSLRPEDTALYYCNKVPWGRYHPRNVYWG QGTQVTVSS |
| 456. | 02G07 | EVQLVESGGGLVQPGRSLTLSCAASTNIFSITPMGWYRQAPGKQRELVAAIHGES TLYADSVKGRFTISRDNAKNSIYLQMNSLRPEDTALYYCNKVPWGDYHPRDVYWG QGTQVTVSS |
| 457. | 01F07-M34Y | EVQLVESGGGLVQPGRSLTLSCAASTNIFSTSPYGWYRQAPGKQRELVAAIHGFS TIYADSVKGRFTISRDNAKNSIYLQMNSLRPEDTALYYCNKVPWGDYHPGNVYWG QGTQVTVSS |
| 458. | 01F01-M34G | EVQLVESGGGLVQPGRSLTLSCAASTNIFSTSPGGWYRQAPGKQRELVAAIHGFS TIYADSVKGRFTISRDNAKNSIYLQMNSLRPEDTALYYCNKVPWGDYHPGNVYWG QGTQVTVSS |
| 459. | 02G02-M34Y | EVQLVESGGGLVQPGRSLTLSCAASTNIFSITPYGWYRQAPGKQRELVAAIHGAS TLYADSVKGRFTISRDNAKNSIYLQMNSLRPEDTALYYCNKVPWGDYHPGNVYWG QGTQVTVSS |
| 460. | 02G02-M34G | EVQLVESGGGLVQPGRSLTLSCAASTNIFSITPGGWYRQAPGKQRELVAAIHGAS TLYADSVKGRFTISRDNAKNSIYLQMNSLRPEDTALYYCNKVPWGDYHPGNVYWG QGTQVTVSS |
| 461. | F1 | EVQLVESGGGLVQPGRSLTLSCAAS |
| 462. | F1 | EVQLVESGGGLVQPGRSLTLSCVAS |
| 463. | F2 | WYRQAPGKQRELVA |
| 464. | F3 | GRFTISRDNAKNSIYLQMNSLRPEDTALYYCNK |
| 465. | F3 | GRFTISRDNAKNSIYLQMNSLRPEDTALYYCNE |
| 466. | F4 | WGQGTQVTVSS |
| 467. | F4 | WGKGTQVTVSS |
| 468. | Human BCMA | MLQMAGQCSQNEYFDSLLHACIPCQLRCSSNTPPLTCQRYCNASVTNSVKGTNAI LWTCLGLSLIISLAVFVLMFLLRKINSEPLKDEFKNTGSGLLGMANIDLEKSRTG DEIILPRGLEYTVEECTCEDCIKSKPKVDSDHCFPLPAMEEGATILVTTKTNDYC KSLPAALSATEIEKSISAR |
| 469. | Murine BCMA | MAQQCFHSEYFDSLLHACKPCHLRCSNPPATCQPYCDPSVTSSVKGTYTVLWIFL GLTLVLSLALFTISFLLRKMNPEALKDEPQSPGQLDGSAQLDKADTELTRIRAGD DRIFPRSLEYTVEECTCEDCVKSKPKGDSDHFFPLPAMEEGATILVTTKTGDYGK SSVPTALQSVMGMEKPTHTR |
| 470. | Cynomolgu s BCMA | MLQMARQCSQNEYFDSLLHDCKPCQLRCSSTPPLTCQRYCNASMTNSVKGMNAIL WTCLGLSLIISLAVFVLTFLLRKMSSEPLKDEFKNTGSGLLGMANIDLEKGRTGD EIVLPRGLEYTVEECTCEDCIKNKPKVDSDHCFPLPAMEEGATILVTTKTNDYCN SLSAALSVTEIEKSISAR |
| 471. | 6x His tag | His-His-His-His-His-His |
| 472. | 253BH10 (llama anti-BCMA antibody) | QVQLVESGGGLVQPGESLRLSCAASTNIFSISPMGWYRQAPGKQRELVAAIHGFS TLYADSVKGRFTISRDNAKNTIYLQMNSLKPEDTAVYYCNKVPWGDYHPRNVYWG QGTQVTVSS |
| 473. | 253BH10 CDR1 | TNIFSISPMG |
| 474. | 253BH10 CDR2 | AIHGFSTLYADSVK |
| 475. | 253BH10 CDR3 | VPWGDYHPRNVY |

## Claims

1. A single domain B cell maturation agent (BCMA) binding protein, wherein the single domain BCMA binding protein comprises an amino acid sequence of SEQ ID NO: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419, or 421.

2. The single domain BCMA binding protein of claim 1, comprising the amino acid sequence of SEQ ID NO: 383.

3. The single domai4n BCMA binding protein of claim 1 or 2, wherein the single domain BCMA binding protein further comprises an Fc domain.

4. The single domain BCMA binding protein of any one of claims 1-3, wherein the single domain BCMA binding protein further comprises an anti-cancer agent.

5. A multispecific binding protein comprising a single domain BCMA binding protein, a human serum albumin binding domain and a CD3 binding domain; wherein the single domain BCMA binding protein comprises an amino acid sequence of SEQ ID NO: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419, or 421.

6. The multispecific binding protein of claim 5, wherein the single domain BCMA binding protein comprises the amino acid sequence of SEQ ID NO: 383.

7. The multispecific binding protein of claim 5 or 6, comprising a domain order of H2N-(C)-(A)-(B)-COOH, wherein A is the human serum albumin binding domain, B is the single domain BCMA binding protein, and C is the CD3 binding domain.

8. The multispecific binding protein according to claim 7, for use in a method of treating a B cell lineage cancer.

9. The multispecific binding protein for the use according to claim 8, wherein the B cell lineage cancer is multiple myeloma, leukemia or lymphoma.

## Patentansprüche

1. Ein Einzeldomänen-B-Zell-Reifungsantigen(BCMA)-bindendes Protein, wobei das Einzeldomänen-BCMA-bindende Protein eine Aminosäuresequenz von SEQ ID NO: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419 oder 421 umfasst.

2. Das Einzeldomänen-BCMA-bindende Protein nach Anspruch 1, das die Aminosäuresequenz von SEQ ID NO: 383 umfasst.

3. Das Einzeldomänen-BCMA-bindende Protein nach Anspruch 1 oder 2, wobei das Einzeldomänen-BCMA-bindende Protein ferner eine Fc-Domäne umfasst.

4. Das Einzeldomänen-BCMA-bindende Protein nach einem der Ansprüche 1 - 3, wobei das Einzeldomänen-BCMA-bindende Protein ferner ein Mittel gegen Krebs umfasst.

5. Ein multispezifisch bindendes Protein, das ein Einzeldomänen-BCMA-bindendes Protein, eine Humanserumalbumin-bindende Domäne und eine CD3-bindende Domäne umfasst, wobei das Einzeldomänen-BCMA-bindende Protein eine Aminosäuresequenz von SEQ ID NO: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419 oder 421 umfasst.

6. Das multispezifisch bindende Protein nach Anspruch 5, wobei das Einzeldomänen-BCMA-bindende Protein die Aminosäuresequenz von SEQ ID NO: 383 umfasst.

7. Das multispezifisch bindende Protein nach Anspruch 5 oder 6, das eine Domänenreihenfolge H2N-(C)-(A)-(B)-COOH umfasst, wobei A die Humanserumalbumin-bindende Domäne ist, B das Einzeldomänen-BCMA-bindende Protein ist und C die CD3-bindende Domäne ist.

8. Das multispezifisch bindende Protein gemäß Anspruch 7 zur Verwendung bei einem Verfahren zur Behandlung eines B-Zelllinienkrebses.

9. Das multispezifisch bindende Protein zur Verwendung gemäß Anspruch 8, wobei der B-Zelllinienkrebs multiples Myelom, Leukämie oder Lymphom ist.

## Revendications

1. Protéine de liaison d'agent de maturation de cellules B (BCMA) à domaine unique, dans laquelle la protéine de liaison de BCMA à domaine unique comprend une séquence d'acides aminés de SEQ ID NO: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419, ou 421.

2. Protéine de liaison de BCMA à domaine unique selon la revendication 1, comprenant la séquence d'acides aminés de SEQ ID NO: 383.

3. Protéine de liaison de BCMA à domai4n unique selon la revendication 1 ou 2, dans laquelle la protéine de liaison de BCMA à domaine unique comprend en outre un domaine Fc.

4. Protéine de liaison de BCMA à domaine unique selon l'une quelconque des revendications 1 à 3, dans laquelle la protéine de liaison de BCMA à domaine unique comprend en outre un agent anticancéreux.

5. Protéine de liaison multispécifique comprenant une protéine de liaison de BCMA à domaine unique, un domaine de liaison de sérum albumine humaine et un domaine de liaison de CD3 ; dans laquelle la protéine de liaison de BCMA à domaine unique comprend une séquence d'acides aminés de SEQ ID NO: 365, 368, 374, 376, 381, 382, 383, 384, 385, 386, 387, 388, 389, 390, 391, 393, 394, 395, 396, 397, 398, 399, 400, 401, 402, 403, 404, 406, 407, 408, 411, 412, 413, 414, 416, 419, ou 421.

6. Protéine de liaison multispécifique selon la revendication 5, dans laquelle la protéine de liaison de BCMA à domaine unique comprend la séquence d'acides aminés de SEQ ID NO: 383.

7. Protéine de liaison multispécifique selon la revendication 5 ou 6, comprenant un ordre de domaines de H2N-(C)-(A)-(B)-COOH, dans laquelle A est le domaine de liaison de sérum albumine humaine, B est la protéine de liaison de BCMA à domaine unique, et C est le domaine de liaison de CD3.

8. Protéine de liaison multispécifique selon la revendication 7, destinée à être utilisée dans un procédé de traitement d'un cancer de lignée de cellules B.

9. Protéine de liaison multispécifique pour l'utilisation selon la revendication 8, dans laquelle le cancer de lignée de cellules B est un myélome multiple, une leucémie ou un lymphome.
